# EUROPÄISCHE PATENTANMELDUNG

(11) **EP 0 502 307 A2**
(43) Veröffentlichungstag der Anmeldung: **09.09.1992**
(21) Anmeldenummer: 92101234.0
(22) Anmeldetag: 27.01.1992
(51) Int. Cl.: C07D 249/14, C07D 403/12, C07D 405/12, A01N 43/653, C07C 265/08, C07C 331/24

(54) **Substituierte 4,5-Diamino-1,2,4-triazol-3-(thi)one**

(30) Priorität: 07.02.1991 DE 4103700
(71) Anmelder: BAYER AG, 51368 Leverkusen (DE)
(72) Erfinder: Kuhnt, Dietmar, Dr., W-5090 Leverkusen (DE); Findeisen, Kurt, Prof. Dr., W-5090 Leverkusen (DE); Haug, Michael, Dr., W-5060 Bergisch Gladbach 2 (DE); Kluth, Joachim, Dr., W-4019 Langenfeld (DE); Müller, Klaus-Helmut, Dr., W-4000 Düsseldorf 13 (DE); König, Klaus, Dr., W-5068 Odenthal (DE); Himmler, Thomas, Dr., W-5068 Odenthal-Glöbusch (DE); Beck, Gunther, Dr., W-5090 Leverkusen (DE); Santel, Hans-Joachim, Dr., W-5090 Leverkusen (DE); Lürssen, Klaus, Dr., W-5090 Bergisch Gladbach 2 (DE); Schmidt, Robert R., Dr., W-5090 Bergisch Gladbach 2 (DE); Krauskopf, Birgit, Dr., W-5090 Bergisch Gladbach 1 (DE)

(57) **Zusammenfassung**

Die Erfindung betrifft neue, substituierte 4,5-Diamino-1,2,4-triazol-3-(thi)one der allgemeinen Formel (I) gefunden, 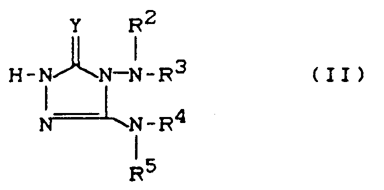
in welcher
- R¹: für Alkyl, Alkenyl, Alkinyl, Halogenalkyl, Halogenalkenyl, Halogenalkinyl, Cyanalkyl, Hydroxyalkyl, Alkoxyalkyl, Aryloxyalkyl, Arylthioalkyl, Arylsulfinylalkyl, Arylsulfonylalkyl, Arylaminoalkyl, N-Alkyl-arylaminoalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Arylalkenyl, Arylalkinyl, Aroyl, Aryl, Aralkyloxy oder Aryloxy, für Alkoxy, Alkenyloxy oder Alkinyloxy steht,
- R², R³, R⁴ und R⁵: gleich oder verschieden sind und unabhängig voneinander für Wasserstoff, Alkyl, Alkenyl, Alkinyl, Halogenalkyl, Halogenalkenyl, Halogenalkinyl, Cyanalkyl, Alkoxyalkyl, Alkylthioalkyl, für jeweils gegebenenfalls substituiertes Cycloalkyl, Cycloalkylalkyl, Aryl oder Aralkyl stehen, oder jeweils zwei dieser Reste (R² und R³ oder R⁴ und R⁵) zusammen für Alkandiyl stehen, und R⁵ auch für Alkoxy stehen kann,
- X: für Sauerstoff oder Schwefel steht und
- Y: für Sauerstoff oder Schwefel steht,
mehrere Verfahren und neue Zwischenprodukte zu ihrer Herstellung, sowie ihre Verwendung als Herbizide und Pflanzenwuchsregulatoren.

## Beschreibung

Die Erfindung betrifft neue, substituierte 4,5-Diamino-1,2,4-triazol-3-(thi)one, mehrere Verfahren und neue Zwischenprodukte zu ihrer Herstellung, sowie ihre Verwendung als Herbizide und Pflanzenwuchsregulatoren.

Es ist bekannt, daß bestimmte substituierte Triazolone, wie z.B. 4-Amino-5-methyl-2-phenylaminocarbonyl-2,4-dihydro-3H-1,2,4-triazol-3-on, herbizide Eigenschaften aufweisen (vgl. EP-A 294666). Die Herbizidwirkung dieser bekannten Verbindung ist jedoch nicht in allen Belangen zufriedenstellend.

Es wurden nun neue, substituierte 4,5-Diamino-1,2,4-triazol-3-(thi)one der allgemeinen Formel (I) gefunden, 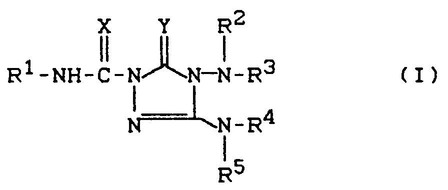
in welcher
- R¹: für Alkyl, Alkenyl, Alkinyl, Halogenalkyl, Halogenalkenyl, Halogenalkinyl, Cyanalkyl, Hydroxyalkyl, Alkoxyalkyl, Aryloxyalkyl, Arylthioalkyl, Arylsulfinylalkyl, Arylsulfonylalkyl, Arylaminoalkyl, N-Alkyl-arylaminoalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Arylalkenyl, Arylalkinyl, Aroyl, Aryl, Aralkyloxy oder Aryloxy, für Alkoxy, Alkenyloxy oder Alkinyloxy steht,
- R², R³, R⁴ und R⁵: gleich oder verschieden sind und unabhängig voneinander für Wasserstoff, Alkyl, Alkenyl, Alkinyl, Halogenalkyl, Halogenalkenyl, Halogenalkinyl, Cyanalkyl, Alkoxyalkyl, Alkylthioalkyl, für jeweils gegebenenfalls substituiertes Cycloalkyl, Cycloalkylalkyl, Aryl oder Aralkyl stehen, oder jeweils zwei dieser Reste (R² und R³ oder R⁴ und R⁵) zusammen für Alkandiyl stehen, und R⁵ auch für Alkoxy stehen kann,
- X: für Sauerstoff oder Schwefel steht und
- Y: für Sauerstoff oder Schwefel steht.

Die Verbindungen der Formel (I) können gegebenenfalls in Abhängigkeit von der Art der Substituenten R¹, R², R³, R⁴ und R⁵ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiter wurde gefunden, daß man die neuen substituierten 4,5-Diamino-1,2,4-triazol-3-(thi)one der allgemeinen Formel (I) 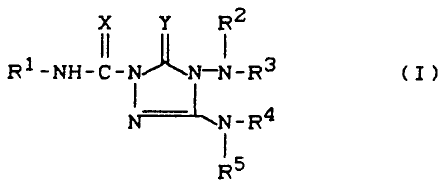
in welcher
- R¹: für Alkyl, Alkenyl, Alkinyl, Halogenalkyl, Halogenalkenyl, Halogenalkinyl, Cyanalkyl, Hydroxyalkyl, Alkoxyalkyl, Aryloxyalkyl, Arylthioalkyl, Arylsulfinylalkyl, Arylsulfonylalkyl, Arylaminoalkyl, N-Alkyl-arylaminoalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Arylalkenyl, Arylalkinyl, Aroyl, Aryl, Aralkyloxy oder Aryloxy, für Alkoxy, Alkenyloxy oder Alkinyloxy steht,
- R², R³, R⁴ und R⁵: gleich oder verschieden sind und unabhängig voneinander für Wasserstoff, Alkyl, Alkenyl, Alkinyl, Halogenalkyl, Halogenalkenyl, Halogenalkinyl, Cyanalkyl, Alkoxyalkyl, Alkylthioalkyl, für jeweils gegebenenfalls substituiertes Cycloalkyl, Cycloalkylalkyl, Aryl oder Aralkyl stehen, oder jeweils zwei dieser Reste (R² und R³ oder R⁴ und R⁵) zusammen für Alkandiyl stehen, und R⁵ auch für Alkoxy stehen kann,
- X: für Sauerstoff oder Schwefel steht und
- Y: für Sauerstoff oder Schwefel steht,
erhält, wenn man
(a) 4,5-Diamino-1,2,4-triazol-3-(thi)one der allgemeinen Formel (II) 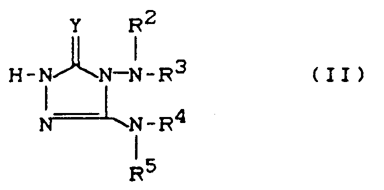 in welcher
   - R², R³, R⁴, R⁵ und Y: die oben angegebenen Bedeutungen haben,
   mit Iso(thio)cyanaten der allgemeinen Formel (III)

   R¹-N=C=X (III)

   in welcher
   - R¹ und X: die oben angegebenen Bedeutungen haben,
   gegebenenfalls in Gegenwart eines Verdünnungsmittels und gegebenenfalls in Gegenwart eines Reaktionshilfsmittels umsetzt,
   oder wenn man
(b) 4-Alkylidenamino-5-amino-1,2,4-triazol-3-(thi)one der allgemeinen Formel (IV) 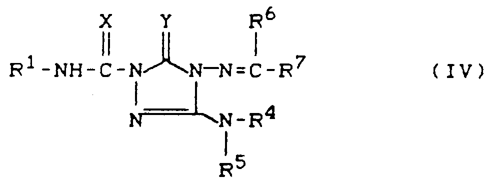 in welcher
   - R¹, R⁴, R⁵, X und Y: die oben angegebenen Bedeutungen haben und
   - R⁶ und R⁷: gleich oder verschieden sind und unabhängig voneinander für Wasserstoff, Alkyl, Aralkyl oder Aryl stehen,
   mit wäßrigen Säuren, gegebenenfalls in Gegenwart organischer Lösungsmittel, umsetzt,
   oder wenn man
c) substituierte 1,2,4-Triazol-3-(thi)one der allgemeinen Formel (V) 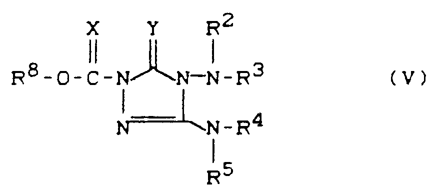 in welcher
   - R², R³, R⁴, R⁵, X und Y: die oben angegebenen Bedeutungen haben und
   - R⁸: für Alkyl, Aralkyl oder Aryl steht,
   mit Aminoverbindungen der allgemeinen Formel (VI)

   R¹-NH₂ (VI)

   in welcher
   - R¹: die oben angegebene Bedeutung hat,
   gegebenenfalls in Gegenwart eines Verdünnungsmittels und gegebenenfalls in Gegenwart eines Reaktionshilfsmittels umsetzt,
   oder wenn man
(d) 4,5-Diamino-1,2,4-triazol-3-(thi)one der allgemeinen Formel (II) 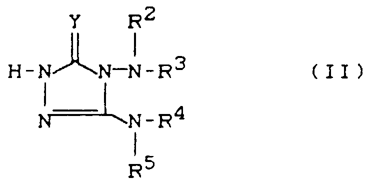 in welcher
   - R², R³, R⁴, R⁵ und Y: die oben angegebenen Bedeutungen haben,
   mit (Thio)Urethanen der allgemeinen Formel (VII) 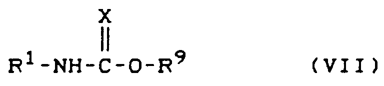 in welcher
   - R¹ und X: die oben angegebenen Bedeutungen haben und
   - R⁹: für Alkyl, Aralkyl oder Aryl steht,
   gegebenenfalls in Gegenwart eines Verdünnungsmittels und gegebenenfalls in Gegenwart eines Reaktionshilfsmittels umsetzt,
   oder wenn man
(e) 4-Oxyalkylidenamino-5-amino-1,2,4-triazol-3-(thi)one der allgemeinen Formel (VIII) 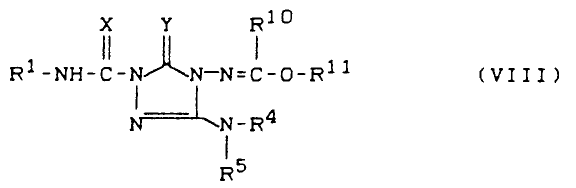 in welcher
   - R¹, R⁴, R⁵, X und Y: die oben angegebenen Bedeutungen haben und
   - R¹⁰ und R¹¹: gleich oder verschieden sind und unabhängig voneinander für Alkyl, Alkenyl, Alkinyl, Cycloalkyl, Aralkyl oder Aryl stehen, sowie R¹⁰ auch für Wasserstoff steht,
   mit Hydridkomplexen der allgemeinen Formel (IX)

   M¹M²H₄ (IX)

   in welcher
   - M¹: für Lithium, Natrium oder Kalium steht und
   - M²: für Bor oder Aluminium steht,
   gegebenenfalls in Gegenwart eines Verdünnungsmittels umsetzt,
   oder wenn man
(f) 4-Alkylidenamino-5-amino-1,2,4-triazol-3-(thi)one der allgemeinen Formel (IV) 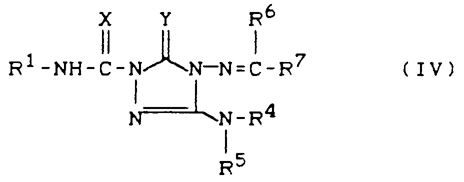 in welcher
   - R¹, R⁴, R⁵, X und Y: die oben angegebenen Bedeutungen haben und
   - R⁶ und R⁷: gleich oder verschieden sind und unabhängig voneinander für Wasserstoff, Alkyl, Aralkyl oder Aryl stehen,
   mit Reduktionsmitteln, gegebenenfalls in Gegenwart von Katalysatoren und gegebenenfalls in Gegenwart von Verdünnungsmitteln umsetzt.

Schließlich wurde gefunden, daß die neuen substituierten 4,5-Diamino-1,2,4-triazol-3-one der allgemeinen Formel (I) interessante herbizide Eigenschaften aufweisen.

Überraschenderweise zeigen die erfindungsgemäßen substituierten 4,5-Diamino-1,2,4-triazol-3-one der allgemeinen Formel (I) erheblich stärkere Herbizidwirkung gegenüber Problemunkräutern, als die nach Struktur und Wirkprofil naheliegende Verbindung 4-Amino-5-methyl-2-phenylaminocarbonyl-2,4-dihydro-3H-1,2,4-triazol-3-on.

In den Definitionen stehen die aromatischen Reste wie beispielsweise Aryl, Aryloxy, Aralkyl vorzugsweise für Phenyl oder Naphthyl, insbesondere für Phenyl. Die aliphatischen Kohlenstoffketten sind, auch wenn es nicht ausdrücklich angegeben ist, jeweils geradkettig oder verzweigt.

Der Substituent Heterocyclylalkyl in R¹ bedeutet vorzugsweise Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen -insbesondere Stickstoff, Sauerstoff und/oder Schwefel - im Heterocyclylteil, wobei der Heterocyclylteil einfach oder mehrfach, insbesondere einfach, zweifach oder dreifach, gleich oder verschieden substituiert sein kann mit Halogen, Cyano, Nitro sowie C₁-C₅-Alkyl, C₁-C₅-Alkoxy, C₁-C₅-Alkylthio, Halogen-C₁-C₅-alkyl, Halogen-C₁-C₅-alkoxy, Halogen-C₁-C₅-alkylthio oder C₁-C₅-Alkoxycarbonyl. Der Heterocyclylteil kann insbesondere substituiert sein mit Fluor, Chlor, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio. Besonders bevorzugt steht der Substituent Heterocyclylalkyl in R¹ für im Heterocyclylteil gegebenenfalls ein bis dreifach, gleich oder verschieden substituiertes Heterocyclylmethyl, Heterocyclylpropyl oder Heterocyclylethyl, wobei als Heterocyclen jeweils infrage kommen: 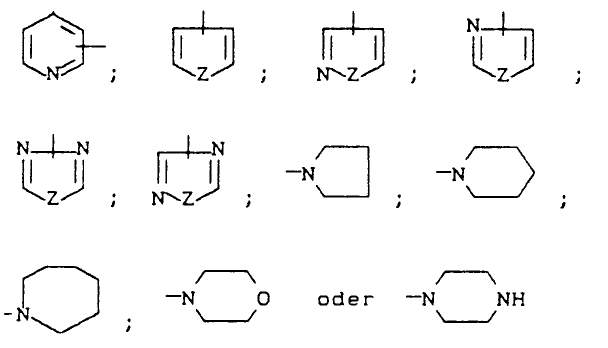
wobei Z jeweils für Sauerstoff oder Schwefel steht und wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio. Die erfindungsgemäßen substituierten 4,5-Diamino-1,2,4-triazol-3-(thi)one sind durch die Formel (I) allgemein definiert. Bevorzugt sind die Verbindungen der Formel (I), bei welchen
- R¹: für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 1 bis 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl, Phenylthioalkyl, Phenylsulfinylalkyl, Phenylsulfonylalkyl, Phenylaminoalkyl oder N-(C₁-C₄-Alkyl)-phenylaminoalkyl mit jeweils 1 bis 4 Kohlenstoffatomen im Alkylteil, Alkoxyalkyl, Alkoxycarbonylalkyl oder Alkoxycarbonylalkenyl mit jeweils 1 bis 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach bis dreifach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl-bzw. Cycloalkenylteil und gegebenenfalls 1 bis 6 Kohlenstoffatomen im Alkylteil steht, wobei als Substituenten jeweils infrage kommen: Halogen, Cyano, Phenyl, Cyclohexyl, Phenylethyl, Phenylisopropyl sowie jeweils geradkettiges oder verzweigtes Alkyl oder Halogenalkyl mit jeweils 1 bis 4 Kohlenstoffatomen und gegebenenfalls 1 bis 9 gleichen oder verschiedenenen Halogenatomen und geradkettiges oder verzweigtes Halogenalkenyl mit 1 bis 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; R¹ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; R¹ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach bis dreifach, gleich oder verschieden substituiertes Aralkyl, Arylalkenyl, Arylalkinyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil bzw. 2 bis 8 Kohlenstoffatomen im geradkettigen oder verzweigten Alkenylteil oder Alkinylteil steht, wobei die Wasserstoffatome des α-Kohlenstoffatoms durch Ethan-1,2-diyl, Propan-1,3-diyl, Butan-1,4-diyl oder Pentan-1,5-diyl ersetzt sein können und wobei als Alkylsubstituenten gegebenenfalls Halogen und Cyano infrage kommen und wobei als Arylsubstituenten jeweils infrage kommen: Halogen, Cyano, Nitro, Amino, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder R¹ für Benzyl mit im Phenylteil ankondensierter -O-CH₂-O- Gruppe steht,
- R², R³, R⁴ und R⁵: gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 1 bis 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R² bis R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter auch jeweils zwei dieser Reste - R² und R³ oder R⁴ und R⁵ - zusammen für geradkettiges oder verzweigtes Alkandiyl mit 2 bis 6 Kohlenstoffatomen stehen können, und R⁵ auch für geradkettiges oder verzweigtes Alkoxy mit 1 bis 8 Kohlenstoffatomen stehen kann,
- X: für Sauerstoff oder Schwefel steht und
- Y: für Sauerstoff oder Schwefel steht.

Besonders bevorzugt sind die Verbindungen der Formel (I), in welcher
- R¹: für Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;
- R¹: weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen: 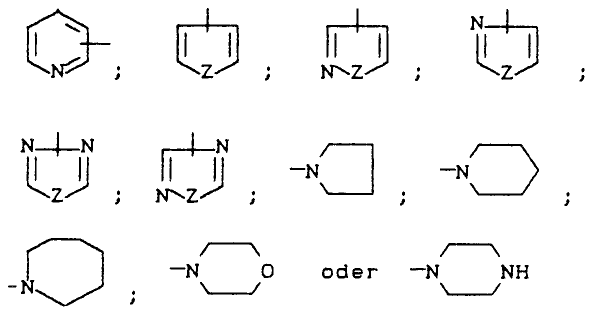 wobei Z jeweils für Sauerstoff oder Schwefel steht und wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
- R¹: außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylpropenyl, Phenylpropinyl, Phenylbutyl, Phenylbutenyl, Phenylbutinyl, Phenylpentyl, Phenylpentenyl, Phenylpentinyl, Phenylhexyl, Phenylhexenyl, Phenylhexinyl, Phenylheptyl, Phenylheptenyl, Phenylheptinyl, Phenyloctyl, Phenyloctenyl, Phenyloctinyl, Phenylcyanmethyl, Phenylcyanethyl, Phenylcyanpropyl, Benzyloxy, Phenylethyloxy, Phenoxy, Phenoxyethyl, Phenoxypropyl, Phenoxy-i-propyl, Phenoxyisobutyl, Phenoxy-tert-butyl, Phenylthioisobutyl, Phenylthio-tert-butyl, Phenylthioethyl, Phenylthiopropyl, Phenylthio-i-propyl, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Difluormethyl, Trifluormethyl, Difluormethoxy, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, n- oder i-Propoxycarbonyl, Cyclohexyl oder Phenoxy,
- R²: für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
- R³: für Wasserstoff, Methyl, Ethyl, Propyl, Butyl, Allyl, Propargyl, Cyanoethyl, Methoxyethyl oder gegebenenfalls durch Chlor substituiertes Benzyl steht, oder zusammen mit R² für Butan-1,4-diyl oder Pentan-1,5-diyl steht,
- R⁴: für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
- R⁵: für Wasserstoff, Methyl, Ethyl, Propyl, Butyl, Allyl, Propargyl, Cyanoethyl, Methoxyethyl, Methoxy oder Benzyl steht, oder zusammen mit R⁴ für Butan-1,4-diyl oder Pentan-1,5-diyl steht,
- X: für Sauerstoff oder Schwefel steht und
- Y: für Sauerstoff oder Schwefel steht,
Ganz besonders bevorzugt sind die Verbindungen der Formel (I), in welcher
- R¹: für jeweils gegebenenfalls einfach bis dreifach, gleich oder verschieden substituiertes 1-Methyl-3-phenyl-propyl, 1-Ethyl-3-phenyl-propyl, 1-Propyl-3-phenyl-propyl, 1-Isopropyl-3-phenyl-propyl, 2-Methyl-3-phenyl-propyl, 1,1-Dimethyl-3-phenyl-propyl, 1-Methyl-1-ethyl-3-phenyl-propyl, 1,1-Diethyl-3-phenyl-propyl, 1-Methyl-1-propyl-3-phenyl-propyl, 1-Methyl-3-phenyl-2-propenyl, 1-Ethyl-3-phenyl-2-propenyl, 1-Propyl-3-phenyl-2-propenyl, 1-Isopropyl-3-phenyl-2-propenyl, 1,1-Dimethyl-3-phenyl-2-propenyl, 1-Methyl-1-ethyl-3-phenyl-2-propenyl, 1,1-Diethyl-3-phenyl-2-propenyl, 1-Methyl-1-propyl-3-phenyl-2-propenyl, 1-Methyl-3-phenyl-2-propinyl, 1-Ethyl-3-phenyl-2-propinyl, 1-Propyl-3-phenyl-2-propinyl, 1-Isopropyl-3-phenyl-2-propinyl, 1,1-Dimethyl-3-phenyl-2-propinyl, 1-Methyl-1-ethyl-3-phenyl-2-propinyl, 1,1-Diethyl-3-phenyl-2-propinyl, 1-Methyl-1-propyl-3-phenyl-2-propinyl, 1,2-Dimethyl-3-phenyl-propyl, 2-Ethyl-1-methyl-3-phenylpropyl, 1,2,2-Trimethyl-3-phenyl-propyl, 1,3,3-Trimethyl-3-phenyl-propyl oder 1,1,2,2-Tetramethyl-3-phenyl-propyl steht, wobei als Phenylsubstituenten jeweils infrage kommen: Fluor, Chlor, Brom, Methyl, Ethyl, n- oder i-Propyl, n-, i-, s- oder t-Butyl, Trifluormethyl, Phenyl, Methoxy, Ethoxy, Propoxy, Isopropoxy, Difluormethoxy, Trifluormethoxy, Difluormethyl, Methoxycarbonyl, Ethoxycarbonyl, Propoxycarbonyl, Cyano, Methylthio, Ethylthio, Propylthio, Isopropylthio, Difluormethylthio, Trifluormethylthio, Methylsulfinyl, Methylsulfonyl und Trifluormethylsulfonyl, weiterhin
- R²: für Wasserstoff steht,
- R³: für Wasserstoff oder Methyl steht,
- R⁴: für Wasserstoff, Methyl oder Ethyl steht,
- R⁵: für Wasserstoff, Methyl, Ethyl, n- oder iso-Propyl steht, oder R⁴ und R⁵ zusammen für Butan-1,4-diyl stehen,
- X: für Sauerstoff oder Schwefel steht und
- Y: für Sauerstoff steht,

Verwendet man beispielsweise 4-Methylamino-5-dimethylamino-2,4-dihydro-3H-1,2,4-triazol-3-on und 1-Methyl-3-phenyl-propyl-isocyanat als Ausgangsstoffe, so läßt sich der Reaktionsablauf beim erfindungsgemäßen Verfahren (a) durch das folgende Formelschema darstellen:

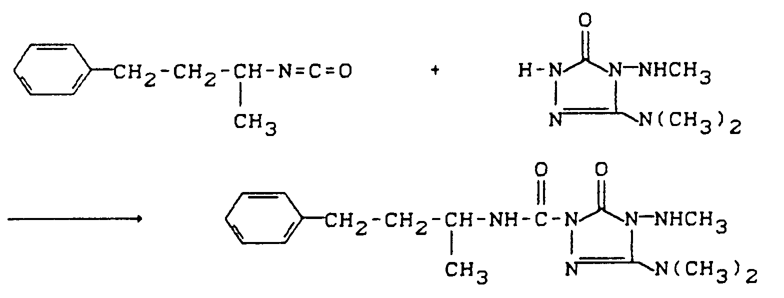

Verwendet man beispielsweise 2-(1-Methyl-3-phenyl-2-propinyl-aminocarbonyl)-4-isopropylidenamino-5-dipropylamino-2,4-dihydro-3H-1, 2,4-triazol-3-on als Ausgangsstoff, so läßt sich der Reaktionsablauf beim erfindungsgemäßen Verfahren (b) durch das folgende Formelschema darstellen:

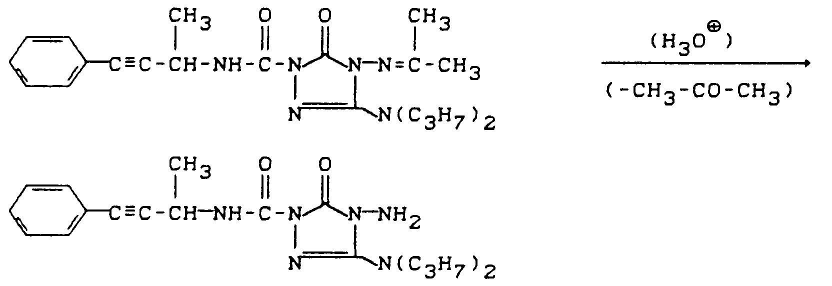

Verwendet man beispielsweise 2-Phenoxycarbonyl-4-methylamino-5-(N-methyl-propylamino)-2,4-dihydro-3H-1,2,4-triazol-3-on und 1,1-Dimethyl-3-(4-methyl-phenyl)-propylamin als Ausgangsstoffe, so läßt sich der Reaktionsablauf beim erfindungsgemäßen Verfahren (c) durch das folgende Formelschema darstellen:

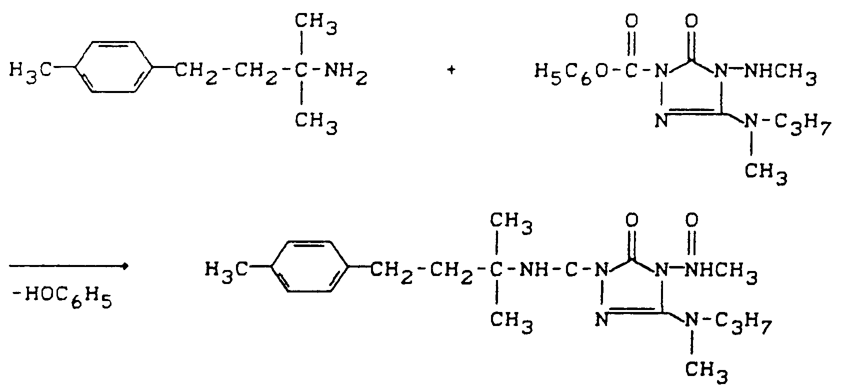

Verwendet man beispielsweise 4-Dimethylamino-5-diethylamino-2,4-dihydro-3H-1,2,4-triazol-3-on und N-(1-Methyl-3-phenyl-2-propenyl)-O-phenyl-urethan als Ausgangsstoffe, so läßt sich der Reaktionsablauf beim erfindungsgemäßen Verfahren (d) durch das folgende Formelschema darstellen:

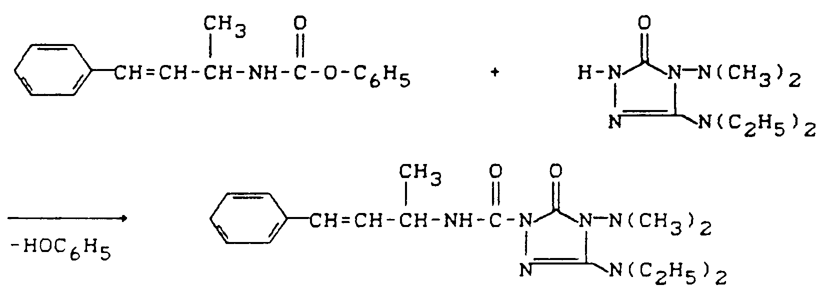

Verwendet man beispielsweise 2-(1,1-Dimethyl-3-phenylpropylamino-carbonyl)-4-ethoxymethylenamino-5-(pyrrolidin-1-yl)-2,4-dihydro-3H-1,2,4-triazol-3-on und Natriumboranat als Ausgangsstoffe, so läßt sich der Reaktionsablauf beim erfindungsgemäßen Verfahren (e) durch das folgende Formelschema darstellen:

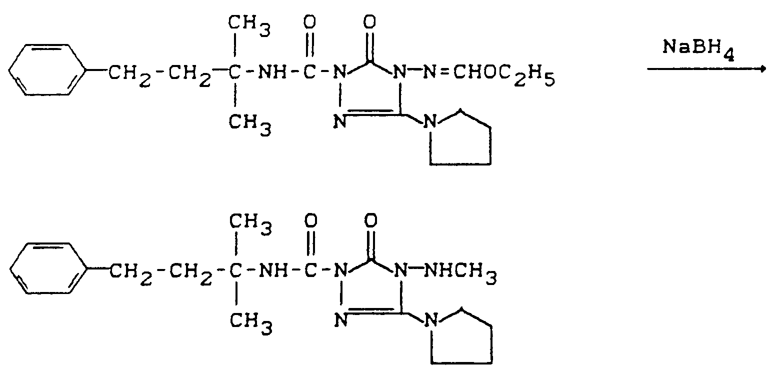

Verwendet man beispielsweise 2-(1-Methyl-3-phenyl-propyl-amino-carbonyl)-4-isobutylidenamino-5-(N-methyl-propylamino)-2,4-dihydro-3H-1,2,4-triazol-3-on und Natriumcyanoborhydrid als Ausgangsstoffe, so läßt sich der Reaktionsablauf beim erfindungsgemäßen Verfahren (f) durch das folgende Formelschema darstellen:

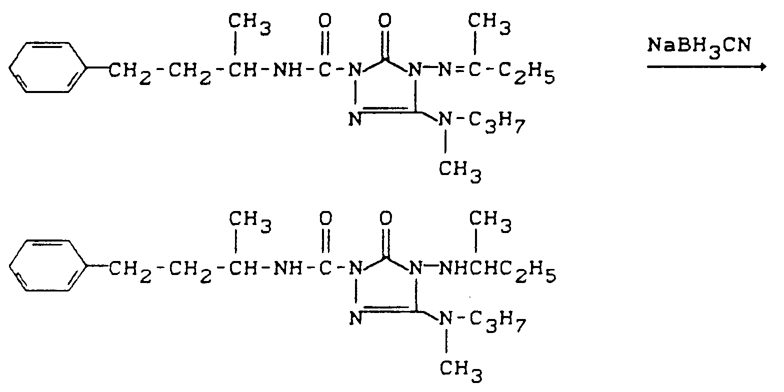

Die bei den erfindungsgemäßen Verfahren (a) und (d) zur Herstellung von Verbindungen der Formel (I) als Ausgangsstoffe zu verwendenden 4,5-Diamino-1,2,4-triazol-3-one sind durch die Formel (II) allgemein definiert.

In Formel (II) haben R², R³, R⁴, R⁵ und Y vorzugsweise bzw, insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R², R³, R⁴, R⁵ und Y angegeben wurden.

Die 4,5-Diamino-1,2,4-triazol-3-(thi)one der Formel (II) sind bekannt und/oder können nach an sich bekannten Verfahren hergestellt werden (vgl. Advan. Heterocycl. Chem. 5 (1965), 119-204; Chem. Ber. 99 (1966), 81-84; J. Chem. Soc. 1952, 4817; J. Heterocycl. Chem. 2 (1965), 302-304; Eur. J. Med. Chem. - Chim. Ther. 21 (1986), 235-244; J. Chem. Soc. C 1968, 2099-2107; J. Chem. Soc. C 1970, 26-34; Liebigs Ann. Chem. 702 (1967), 101-111; Liebigs Ann. Chem. 703 (1967), 116-130; Herstellungsbeispiele).

Man erhält die Verbindungen der Formel (II) beispielsweise, wenn man
(α) Amino- bzw. Iminoverbindungen der allgemeinen Formeln (X), (XI) oder (XII) 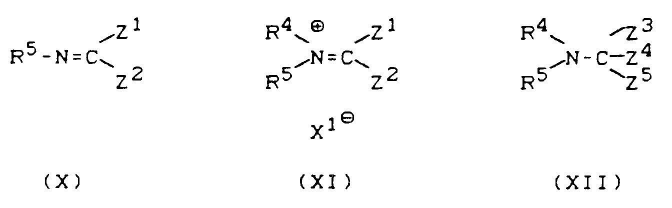 in welchen
   - R⁴ und R⁵: die oben angegebenen Bedeutungen haben,
   - X¹: für Halogen steht und
   - Z¹, Z², Z³, Z⁴ und Z⁵: für in der Kohlensäurechemie übliche Abgangsgruppen stehen,
   mit Carbodihydrazid-Derivaten der allgemeinen Formeln (XIIIa) oder (XIIIb) 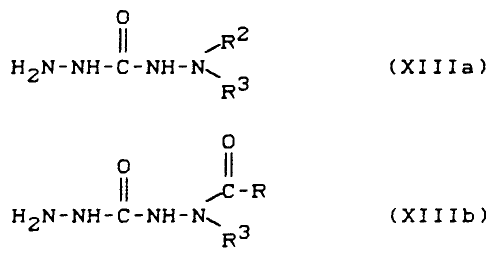 in welchen
   - R² und R³: die oben angegebenen Bedeutungen haben und
   - R: für Alkyl, Alkoxy oder Aryl steht,
   gegebenenfalls in Gegenart eines Verdünnungsmittels, wie z.B. Phenol und/oder Chlorbenzol, gegebenenfalls in Gegenwart eines Katalysators, wie z.B. Dibutylzinnoxid, und gegebenenfalls in Gegenwart eines Säureakzeptors, wie z.B. Natriumcarbonat, bei Temperaturen zwischen 20°C und 200°C umsetzt, gegebenenfalls die Gruppierung -CO-R durch Umsetzung mit einer wäßrigen Lauge, wie z.B. Natronlauge, anschließend bei Temperaturen zwischen 20°C und 120°C abspaltet und nach üblichen Methoden aufarbeitet (vgl. die Herstellungsbeispiele), oder wenn man
(β) Diaminoguanidin-Derivate der allgemeinen Formel (XIV) 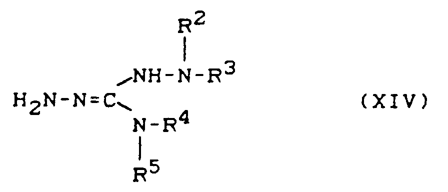 in welcher
   - R², R³, R⁴ und R⁵: die oben angegebenen Bedeutungen haben,
   oder Säureaddukte von Verbindungen der Formel (XIV) oder Tautomere von Verbindungen der Formel (XIV)
   mit Kohlensäurederivaten der allgemeinen Formel (XV) 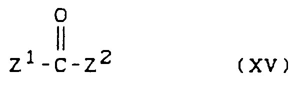 in welcher
   - Z¹ und Z²: die oben angegebenen Bedeutungen haben,
   gegebenenfalls in Gegenwart eines Verdünnungsmittels, wie z.B. Phenol, und gegebenenfalls in Gegenwart eines Säureakzeptors, wie z.B. Natriumcarbonat, bei Temperaturen zwischen 20°C und 200°C umsetzt und nach üblichen Methoden aufarbeitet (vgl. die Herstellungsbeispiele).

In den Formeln (X), (XI) und (XII) haben R⁴ und R⁵ vorzugsweise bzw. insbesondere diejenigen Bedeutungen, die bereits oben bei der Beschreibung der Verbindungen der Formel (IIa) als bevorzugt bzw. als besonders bevorzugt für R⁴ und R⁵ angegeben wurden und
- Z¹, Z², Z³, Z⁴ und Z⁵: sind gleich oder verschieden und stehen vorzugsweise für Halogen, C₁-C₄-Alkoxy, C₁-C₄-Alkylthio, C₁-C₄-Alkylamino, Di-(C₁-C₂-alkyl)-amino, Phenoxy oder Phenylthio, insbesondere für Chlor, Methoxy, Ethoxy, Methylthio, Ethylthio, Methylamino, Ethylamino, Dimethylamino oder Phenoxy.

Die Abgangsgruppen aus der Reihe Z¹ bis Z⁵ können gegebenenfalls auch verknüpft sein. Vorzugsweise stehen dann Z¹ und Z² oder Z³ und Z⁴ zusammen für C₂-C₄-Alkandioxy, insbesondere für Ethan-1,2-dioxy (-OCH₂CH₂O-).

Die Verbindungen der Formeln (X), (XI) und (XII) sind bekannt und/oder können nach an sich bekannten Verfahren hergestellt werden (vgl. Synthesis 1977, 73-90; loc. cit. 1988, 460-466; J. Chem. Soc. 1951, 2492-2494; Chem. Ber. 120 (1987), 339-344; Tetrahedron Lett. 1982, 3539-3542; Houben-Weyl, Methoden der Organischen Chemie, 4. Auflage, Band E4 (1983), 522-624 und 652-722).

In den Formeln (XIIIa) und (XIIIb) haben R² und R³ vorzugsweise bzw. insbesondere diejenigen Bedeutungen, die bereits oben bei der Beschreibung der Verbindungen der Formel (IIa) als bevorzugt bzw. als besonders bevorzugt für R² und R³ angegeben wurden und R steht vorzugsweise für C₁-C₄-Alkyl, C₁-C₄-Alkoxy oder Phenyl, insbesondere für Methyl, Ethyl, Methoxy oder Ethoxy.

Die Carbodihydrazid-Derivate der Formel (XIIIa) sind mit Ausnahme von Carbodihydrazid (R²=R³=H) noch nicht aus der Literatur bekannt.

Man erhält die neuen Verbindungen der Formel (XIIIa), wenn man Kohlensäurederivate der Formel (XV) 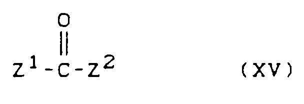
in welcher
- Z¹ und Z²: die oben angegebenen Bedeutungen haben,
nacheinander mit etwa einem Moläquivalent eines Hydrazinderivates der Formel (XVI) 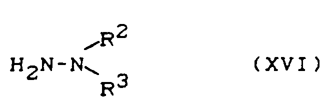
in welcher
- R² und R³: die oben angegebenen Bedeutungen haben
und etwa einem Moläquivalent Hydrazin oder Hydrazinhydrat bei Temperaturen zwischen 0°C und 100°C umsetzt. Die Aufarbeitung kann anschließend nach üblichen Methoden erfolgen. Vorzugsweise werden jedoch die Verbindungen der Formel (XIIIa) nicht in reiner Form isoliert, sondern direkt weiter umgesetzt.

Man erhält die neuen Verbindungen der Formel (XIIIb), wenn man Hydrazinderivate der Formel (XVIa) 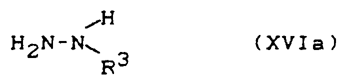
in welcher
- R³: die oben angegebene Bedeutung hat,
mit Acylierungsmitteln der Formel (XVII) 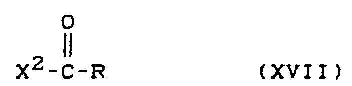
in welcher
- R: die oben angegebene Bedeutung hat und
- X²: für Halogen oder die Gruppierung -O-CO-R steht,
gegebenenfalls in Gegenwart eines Verdünnungsmittels, wie z.B. Methanol, Methylenchlorid oder Toluol, und gegebenenfalls in Gegenwart eines Säureakzeptors, wie z.B. Kaliumcarbonat oder Pyridin, bei Temperaturen zwischen -80°C und +80°C umsetzt, die hierbei erhaltenen acylierten Hydrazine der allgemeinen Formel (XVIII) 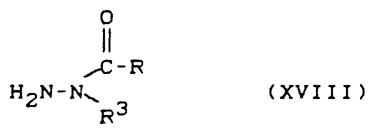
in welcher
- R³ und R: die oben angegebenen Bedeutungen haben,
mit Kohlensäurederivaten der allgemeinen Formel (XV) 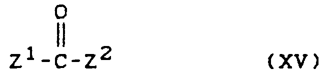
in welcher
- Z¹ und Z²: die oben angegebenen Bedeutungen haben,
gegebenenfalls in Gegenwart eines Verdünnungsmittels, wie z.B. Methylenchlorid oder Ethylenchlorid, und gegebenenfalls in Gegenwart eines Säureakzeptors, wie z.B. Natriumcarbonat, bei Temperaturen zwischen -20°C und +80°C umsetzt und die hierbei erhaltenen zweifach acylierten Hydrazine der Formel (XIX) 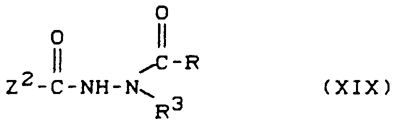
in welcher
- R³, R und Z²: die oben angegebenen Bedeutungen haben,
mit Hydrazin oder Hydrazinhydrat gegebenenfalls in Gegenwart eines Verdünnungsmittels, wie z.B. Chlorbenzol, bei Temperaturen zwischen 0°C und 120°C umsetzt (vgl. die Herstellungsbeispiele).

Die als Vorprodukte benötigten Verbindungen der Formeln (XV), (XVI), (XVIa) und (XVII) sind bekannte organische Synthesechemikalien.

In der Formel (XIV) haben R², R³, R⁴ und R⁵ vorzugsweise bzw. insbesondere diejenigen Bedeutungen, die bereits oben bei der Beschreibung der Verbindungen der Formel (IIa) als bevorzugt bzw. als besonders bevorzugt für R², R³, R⁴ und R⁵ angegeben wurden.

Die Verbindungen der Formel (XIV) sind bekannt und/oder können nach an sich bekannten Verfahren hergestellt werden (vgl. EP-A 150677).

In der Formel (XV) sind Z¹ und Z² gleich oder verschieden und stehen jeweils vorzugsweise für Halogen, C₁-C₄-Alkoxy, C₁-C₄-Alkylthio, C₁-C₄-Alkylamino, Di-(C₁-C₂-alkyl)-amino, Phenoxy oder Phenylthio, insbesondere für Chlor, Methoxy, Ethoxy, Phenoxy, Methylthio oder Dimethylamino. Z¹ und Z² können auch cyclisch verknüpft sein. Vorzugsweise stehen dann Z¹ und Z² zusammen für C₂-C₄-Alkandioxy, insbesondere für Ethan-1,2-dioxy (-OCH₂CH₂O-). Die Verbindungen der Formel (XV) sind bekannte organische Synthesechemikalien.

Die beim erfindungsgemäßen Verfahren (a) weiter als Ausgangsstoffe zu verwendenden Iso(thio)cyanate sind durch die Formel (III) allgemein definiert.

In Formel (III) haben R¹ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und X angegeben wurden.

Die Iso(thio)cyanate der Formel (III) sind weitgehend bekannte organische Synthesechemikalien (vgl. z.B. Nouv. J. Chim. 1 (1977), 243-254 - zitiert in Chem. Abstracts 87: 151614a; Liebigs Ann. Chem. 562 (1949), 75-136).

Neu und Gegenstand der vorliegenden Anmeldung sind die Iso(thio)cyanate der Formel (III), in welcher
- R¹: für jeweils gegebenenfalls einfach bis dreifach, gleich oder verschieden substituiertes 1-Ethyl-3-phenyl-propyl, 1-Propyl-3-phenyl-propyl, 1-Isopropyl-3-phenyl-propyl, 2-Methyl-3-phenyl-propyl, 1,1-Dimethyl-3-phenyl-propyl, 1,2-Dimethyl-3-phenylpropyl, 1,1-Diethyl-3-phenyl-propyl, 1-Methyl-1-propyl-3-phenyl-propyl, 1,2,2-Trimethyl-3-phenylpropyl, 1-Methyl-3-phenyl-2-propenyl, 1-Ethyl-3-phenyl-2-propenyl, 1-Propyl-3-phenyl-2-propenyl, 1-Isopropyl-3-phenyl-2-propenyl, 1,1-Dimethyl-3-phenyl-2-propenyl, 1-Methyl-1-ethyl-3-phenyl-2-propenyl, 1,1-Diethyl-3-phenyl-2-propenyl, 1-Methyl-1-propyl-3-phenyl-2-propenyl, 1-Methyl-3-phenyl-2-propinyl, 1-Ethyl-3-phenyl-2-propinyl, 1-Propyl-3-phenyl-2-propinyl, 1-Isopropyl-3-phenyl-2-propinyl, 1,1-Dimethyl-3-phenyl-2-propinyl, 1-Methyl-1-ethyl-3-phenyl-2-propinyl, 1,1-Diethyl-3-phenyl-2-propinyl, 1-Methyl-1-propyl-3-phenyl-2-propinyl, 1,2-Dimethyl-3-phenyl-propyl, 2-Ethyl-1-methyl-3-phenylpropyl, 1,2,2-Trimethyl-3-phenyl-propyl, 1,3,3-Trimethyl-3-phenyl-propyl oder 1,1,2,2-Tetramethyl-3-phenyl-propyl steht, wobei als Phenylsubstituenten jeweils infrage kommen: Fluor, Chlor, Brom, Methyl, Ethyl, n- oder i-Propyl, n-, i-, s- oder t-Butyl, Trifluormethyl, Phenyl, Methoxy, Ethoxy, Propoxy, Isopropoxy, Difluormethoxy, Trifluormethoxy, Difluormethyl, Methoxycarbonyl, Ethoxycarbonyl, Propoxycarbonyl, Cyano, Methylthio, Ethylthio, Propylthio, Isopropylthio, Difluormethylthio, Trifluormethylthio, Methylsulfinyl, Methylsulfonyl und Trifluormethylsulfonyl, und
- X: für Sauerstoff oder Schwefel, vorzugsweise für Sauerstoff, steht.

Man erhält die neuen Iso(thio)cyanate der Formel (III), wenn man Aminoverbindungen der allgemeinen Formel (VI)

R¹-NH₂ (VI)

in welcher
- R¹: für jeweils gegebenenfalls einfach bis dreifach, gleich oder verschieden substituiertes 1-Ethyl-3-phenyl-propyl, 1-Propyl-3-phenyl-propyl, 1-Isopropyl-3-phenyl-propyl, 2-Methyl-3-phenyl-propyl, 1,1-Dimethyl-3-phenyl-propyl, 1,2-Dimethyl-3-phenylpropyl, 1,1-Diethyl-3-phenyl-propyl, 1-Methyl-1-propyl-3-phenyl-propyl, 1,2,2-Trimethyl-3-phenylpropyl, 1-Methyl-3-phenyl-2-propenyl, 1-Ethyl-3-phenyl-2-propenyl, 1-Propyl-3-phenyl-2-propenyl, 1-Isopropyl-3-phenyl-2-propenyl, 1,1-Dimethyl-3-phenyl-2-propenyl, 1-Methyl-1-ethyl-3-phenyl-2-propenyl, 1,1-Diethyl-3-phenyl-2-propenyl, 1-Methyl-1-propyl-3-phenyl-2-propenyl, 1-Methyl-3-phenyl-2-propinyl, 1-Ethyl-3-phenyl-2-propinyl, 1-Propyl-3-phenyl-2-propinyl, 1-Isopropyl-3-phenyl-2-propinyl, 1,1-Dimethyl-3-phenyl-2-propinyl, 1-Methyl-1-ethyl-3-phenyl-2-propinyl, 1,1-Diethyl-3-phenyl-2-propinyl oder 1-Methyl-1-propyl-3-phenyl-2-propinyl, 1,2-Dimethyl-3-phenyl-propyl, 2-Ethyl-1-methyl-3-phenyl-propyl, 1,2,2-Trimethyl-3-phenyl-propyl, 1,3,3-Trimethyl-3-phenyl-propyl, 1,1,2,2-Tetramethyl-3-phenyl-propyl steht, wobei als Phenylsubstituenten jeweils infrage kommen: Fluor, Chlor, Brom, Methyl, Ethyl, n- oder i-Propyl, n-, i-, s- oder t-Butyl, Trifluormethyl, Phenyl, Methoxy, Ethoxy, Propoxy, Isopropoxy, Difluormethoxy, Trifluormethoxy, Difluormethyl, Methoxycarbonyl, Ethoxycarbonyl, Propoxycarbonyl, Cyano, Methylthio, Ethylthio, Propylthio, Isopropylthio, Difluormethylthio, Trifluormethylthio, Methylsulfinyl, Methylsulfonyl und Trifluormethylsulfonyl,
mit Phosgen, gegebenenfalls in Gegenwart eines Verdünnungsmittels, wie z.B. Toluol oder Chlorbenzol, bei Temperaturen zwischen 0°C und 150°C umsetzt (vgl. die Herstellungsbeispiele), oder wenn man die als Ausgangsstoffe angegebenen Aminoverbindungen der Formel (VI) mit Thiophosgen, gegebenenfalls in Gegenwart von Verdünnungsmitteln, wie z.B. Toluol oder Chloroform und Wasser, bei Temperaturen zwischen -10°C und +50°C umsetzt.

Auf die hierbei benötigten Ausgangsstoffe der Formel (VI) wird weiter unten im Zusammenhang mit der Beschreibung des erfindungsgemäßen Verfahrens (c) weiter eingegangen.

Die bei den erfindungsgemäßen Verfahren (b) und (f) zur Herstellung von Verbindungen der Formel (I) als Ausgangsstoffe zu verwendenden 4-Alkylidenamino-5-amino-1,2,4-triazol-3-(thi)one sind durch die Formel (IV) allgemein definiert.

In Formel (IV) haben R¹, R⁴, R⁵, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R⁴, R⁵, X und Y angegeben wurden und R⁶ und R⁷ stehen vorzugsweise jeweils unabhängig voneinander für Wasserstoff, geradkettiges oder verzweigtes Alkyl mit 1 bis 4 Kohlenstoffatomen, Phenyl oder Benzyl.

Man erhält die Verbindungen der Formel (IV) beispielsweise, wenn man die oben beschriebenen 4,5-Diamino-1,2,4-triazol-3-(thi)one der allgemeinen Formel (II) 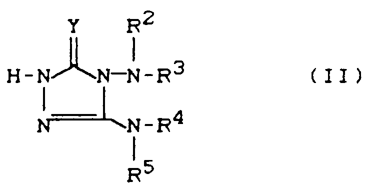
in welcher
- R², R³, R⁴, R⁵ und Y: die oben angegebenen Bedeutungen haben mit der Maßgabe, daß R² und R³ für Wasserstoff stehen,
mit Aldehyden oder Ketonen der Formel (XX) 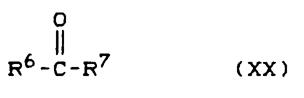
in welcher
- R⁶ und R⁷: die oben angegebenen Bedeutungen haben,
gegebenenfalls in Gegenwart eines Verdünnungsmittels, wie z.B. Methylenchlorid oder Toluol, und gegebenenfalls in Gegenwart eines Katalysators, wie z.B. p-Toluolsulfonsäure, bei Temperaturen zwischen 20°C und 120°C umsetzt und die so erhaltenen 4-Alkylidenamino-5-amino-1, 2,4-triazol-3-(thi)one der Formel (XXI) 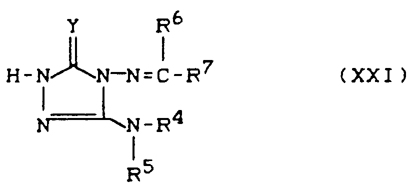
in welcher
- R⁴, R⁵, R⁶, R⁷ und Y: die oben angegebenen Bedeutungen haben,
entweder in einer anschließenden zweiten Stufe mit Iso(thio)cyanaten der Formel (III)

R¹-N=C=X (III)

in welcher
- R¹ und X: die oben angegebenen Bedeutungen haben,
gegebenenfalls in Gegenwart eines Verdünnungsmittels, wie z.B. Methylenchlorid oder Dioxan, und gegebenenfalls in Gegenwart eines Reaktionshilfsmittels, wie z.B. Triethylamin, bei Temperaturen zwischen 20°C und 150°C umsetzt,
oder wenn man alternativ in einer zweiten Stufe die Verbindungen der Formel (XXI) mit Chlor(thio)ameisensäureestern der Formel (XXII) 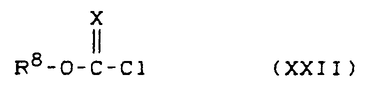
in welcher
- X: die oben angegebene Bedeutung hat und
- R⁸: für Alkyl, Aryl oder Aralkyl steht,
gegebenenfalls in Gegenwart eines Verdünnungsmittels, wie z.B. Tetrahydrofuran, und gegebenenfalls in Gegenwart eines Reaktionshilfsmittels, wie z.B. Natriumhydrid oder Kalium-tert-butylat, bei Temperaturen zwischen - 20°C und +40°C umsetzt und die so erhaltenen 2-Oxy-(thio)-carbonyl-4-alkylidenamino-5-amino-1,2,4-triazol- 3-(thi)one der Formel (XXIII) 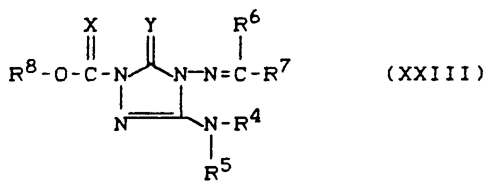
in welcher
- R⁴, R⁵, R⁶, R⁷, R⁸, X und Y: die oben angegebenen Bedeutungen haben,
in einer anschließenden dritten Stufe mit Aminen der Formel (VI)

R¹-NH₂ (VI)

in welcher
- R¹: die oben angegebene Bedeutung hat,
gegebenenfalls in Gegenwart eines Verdünnungsmittels, wie z.B. Tetrahydrofuran oder Dioxan, und gegebenenfalls in Gegenwart einer Base, wie z.B. Natriumhydroxid oder Kaliumhydroxid, bei Temperaturen zwischen 20°C und 100°C umsetzt.

Dabei ist es möglich und gegebenenfalls von Vorteil, die Umsetzung der Verbindungen der Formel (XXI) mit Chlor(thio)ameisensäureestern und die anschließende Umsetzung mit Aminen in sogenannten Eintopfverfahren durchzuführen.

Die Aldehyde bzw. Ketone der Formel (XX), die Iso(thio)cyanate der Formel (III), die Chlor(thio)ameisensäureester der Formel (XXII) und die Amine der Formel (VI) sind weitgehend bekannte organische Synthesechemikalien.

Die beim erfindungsgemäßen Verfahren (c) zur Herstellung von Verbindungen der Formel (I) als Ausgangsstoffe zu verwendenden substituierten 1,2,4-Triazol-3-(thi)one sind durch die Formel (V) allgemein definiert.

In Formel (V) haben R², R³, R⁴, R⁵, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R², R³, R⁴, R⁵, X und Y angegeben wurden, und
- R⁸: steht vorzugsweise für Methyl, Ethyl, Propyl, Isopropyl, Butyl, Isobutyl, sec-Butyl, tert-Butyl, Phenyl oder Benzyl.

Man erhält die Verbindungen der Formel (V) beispielsweise, wenn man 4,5-Diamino-1,2,4-triazol-3-(thi)one der Formel (II) 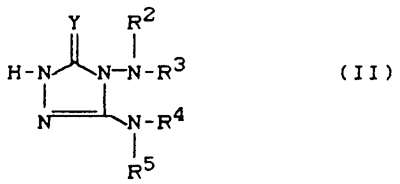
in welcher
- R², R³, R⁴, R⁵ und Y: die oben angegebenen Bedeutungen haben,
mit Chlor(thio)ameisensäureestern der Formel (XXII) 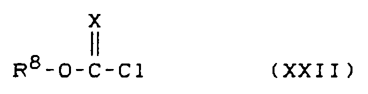
in welcher
- R⁸ und X: die oben angegebenen Bedeutungen haben,
gegebenenfalls in Gegenwart eines Verdünnungsmittels, wie z.B. Tetrahydrofuran, und gegebenenfalls in Gegenwart eines Säureakzeptors, wie z.B. Kalium-tert-butylat, bei Temperaturen zwischen -20°C und +100°C umsetzt.

Die beim erfindungsgemäßen Verfahren (c) weiter als Ausgangsstoffe zu verwendenden Aminoverbindungen sind durch die Formel (VI) allgemein definiert.

In Formel (VI) hat R¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R¹ angegeben wurde.

Die Ausgangsstoffe der Formel (VI) sind bekannt und/oder können nach an sich bekannten Verfahren hergestellt werden (vgl. J. Med. Chem. 25 (1982), 1363-1370; Tetrahedron Lett. 29 (1988), 223-224; Chem. Ber. 117 (1984), 856-858; DE-OS 3426919; EP-A 237305; J. Am. Chem. Soc. 71 (1949), 3482-3485; Tetrahedron Lett. 27 (1986), 3957-3960; Bull. Soc. Chim. France 1974 (3-4, Pt. 2), 615-622; Tetrahedron Lett. 31 (1990), 2661-2664; J. Med. Chem. 13 (1970), 1249-1250; Herstellungsbeispiele).

Die beim erfindungsgemäßen Verfahren (d) zur Herstellung von Verbindungen der Formel (I) als Ausgangsstoffe zu verwendenden (Thio)Urethane sind durch die Formel (VII) allgemein definiert.

In Formel (VII) haben R¹ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und X angegeben wurden und
- R⁹: steht vorzugsweise für Methyl, Ethyl, Propyl, Isopropyl, Butyl, Isobutyl, sec-Butyl, tert-Butyl, Phenyl oder Benzyl.

Die Ausgangsstoffe der Formel (VII) sind weitgehend bekannte organische Synthesechemikalien.

Die beim erfindungsgemäßen Verfahren (e) zur Herstellung von Verbindungen der Formel (I) als Ausgangsstoffe zu verwendenden 4-Oxyalkylidenamino-5-amino-1,2,4-triazol(thi)one sind durch die Formel (VIII) allgemein definiert.

In Formel (VIII) haben R¹, R⁴, R⁵, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R⁴, R⁵, X und Y angegeben wurden und
- R¹⁰ und R¹¹: stehen vorzugsweise für Wasserstoff, C₁-C₆-Alkyl, C₂-C₆-Alkenyl, C₂-C₆-Alkinyl, C₃-C₆-Cycloalkyl, Phenyl oder Benzyl.

Insbesondere stehen R¹⁰ für Wasserstoff, Methyl, Ethyl, Propyl, Phenyl oder Benzyl und R¹¹ für Methyl, Ethyl, Propyl oder Benzyl.

Man erhält die Verbindungen der Formel (VIII) beispielsweise, wenn man 4,5-Diamino-1,2,4-triazol-3-(thi)one der allgemeinen Formel (II) 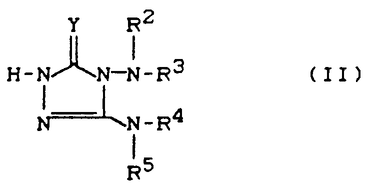
in welcher
- R² und R³: für Wasserstoff stehen und
- Y, R⁴ und R⁵: die oben angegebenen Bedeutungen haben,
mit Orthocarbonsäureestern der allgemeinen Formel (XXIV)

R¹⁰-C(OR¹¹)₃ (XXIV)

in welcher
- R¹⁰ und R¹¹: die oben angegebenen Bedeutungen haben,
gegebenenfalls in Gegenwart eines Verdünnungsmittels, wie z.B. Chloroform, Toluol oder Chlorbenzol, und gegebenenfalls in Gegenwart eines Katalysators, wie z.B. p-Toluolsulfonsäure, bei Temperaturen zwischen 50°C und 200°C umsetzt und die so erhaltenen Oxyalkylidenverbindungen der Formel (XXV) 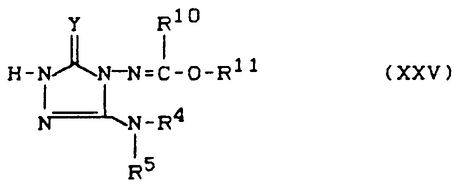
in welcher
- R⁴, R⁵, R¹⁰, R¹¹ und Y: die oben angegebenen Bedeutungen haben,
in einer anschließenden zweiten Stufe mit Iso(thio)cyanaten der Formel (III)

R¹-N=C=X (III)

in welcher
- R¹ und X: die oben angegebenen Bedeutungen haben,
gegebenenfalls in Gegenwart eines Verdünnungsmittels, wie z.B. Methylenchlorid oder Dioxan, und gegebenenfalls in Gegenwart eines Reaktionshilfsmittels, wie z.B. Triethylamin, bei Temperaturen zwischen 20°C und 150°C umsetzt.

Alternativ erhält man die Verbindungen der Formel (VIII) auch, wenn man Verbindungen der Formel (I) 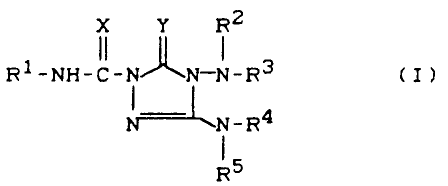
in welcher
- R² und R³: für Wasserstoff stehen und
- R¹, R⁴, R⁵, X und Y: die oben angegebenen Bedeutungen haben,
mit Orthocarbonsäureestern der Formel (XXIV)

R¹⁰-C(OR¹¹)₃ (XXIV)

in welcher
- R¹⁰ und R¹¹: die oben angegebenen Bedeutungen haben,
gegebenenfalls in Gegenwart eines Verdünnungsmittels, wie z.B. Chloroform, Toluol oder Chlorbenzol, und gegebenenfalls in Gegenwart eines Katalysators, wie z.B. p-Toluolsulfonsäure, bei Temperaturen zwischen 50°C und 200°C umsetzt.

Die Orthocarbonsäureester der Formel (XXIV) sind bekannte organische Synthesechemikalien.

In der Formel (XXV) haben R⁴, R⁵ und Y vorzugsweise bzw. insbesondere diejenigen Bedeutungen, die oben bei der Beschreibung der erfindungsgemäßen Verbindungen der Formel (I) als bevorzugt bzw. als besonders bevorzugt für R⁴, R⁵ und Y angegeben wurden und R¹⁰ und R¹¹ stehen vorzugsweise für C₁-C₆-Alkyl, C₂-C₆-Alkenyl, C₂-C₆-Alkinyl, C₃-C₆-Cycloalkyl, Phenyl oder Benzyl; R¹⁰ auch für Wasserstoff; insbesondere stehen R¹⁰ für Wasserstoff, Methyl, Ethyl, Propyl, Phenyl oder Benzyl und R¹¹ für Methyl, Ethyl, Propyl oder Benzyl.

Die bei Verfahren (e) weiter als Ausgangsstoffe benötigten Hydridkomplexe der Formel (IX) sind bekannte Synthesechemikalien.

Das erfindungsgemäße Verfahren (a) wird vorzugsweise in Gegenwart eines Verdünnungsmittels durchgeführt.

Als Verdünnungsmittel zur Durchführung des erfindungsgemäßen Verfahrens (a) kommen insbesondere inerte organische Lösungsmittel infrage. Hierzu gehören beispielsweise aliphatische, alicyclische oder aromatische, gegebenenfalls halogenierte Kohlenwasserstoffe, wie Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (a) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man auf 1 Mol 4,5-Diamino-1,2,4-triazol-3-(thi)on der Formel (II) im allgemeinen 1.0 bis 2.0 Mol, vorzugsweise 1.0 bis 1.5 Mol an Iso(thio)cyanat der Formel (III) und gegebenenfalls 0.001 bis 2.0 Mol, vorzugsweise 0.001 bis 1.0 Mol an Reaktionshilfsmittel ein. Die Reaktionsdurchführung, Aufarbeitung und Isolierung der Reaktionsprodukte erfolgt nach allgemein üblichen Methoden.

Als Säuren zur Durchführung des erfindungsgemäßen Verfahrens (b) kommen alle üblicherweise für Hydrazonspaltungen verwendbaren anorganischen und organischen Säuren infrage. Vorzugsweise verwendet man anorganische Mineralsäuren wie Chlorwasserstoffsäure, Schwefelsäure oder Phosphorsäure.

Als Verdünnungsmittel zur Durchführung des erfindungsgemäßen Verfahrens (b) kommen alle üblichen organischen oder anorganischen Lösungsmittel infrage. Vorzugsweise verwendet man polare mit Wasser mischbare organische Lösungsmittel, insbesondere Alkohole, wie Methanol, Ethanol, Propanol oder Butanol, deren Gemische mit Wasser oder reines Wasser als Verdünnungsmittel.

Die Reaktionstemperaturen können bei der Durchführung des erfindungsgemäßen Verfahrens (b) in einem größeren Bereich variiert werden. Im allgemeinen arbeitet man bei Temperaturen zwischen 20°C und 150°C, vorzugsweise bei Temperaturen zwischen 50°C und 120°C.

Das erfindungsgemäße Verfahren (b) wird üblicherweise bei Normaldruck oder unter vermindertem Druck durchgeführt. Arbeitet man unter vermindertem Druck, so kommen Druckbereiche zwischen 20 und 400 mbar, vorzugsweise zwischen 100 und 200 mbar infrage.

Zur Durchführung des erfindungsgemäßen Verfahrens (b) setzt man auf 1 Mol 4-Alkylidenamino-5-amino-1,2,4-triazol-3-(thi)on der Formel (IV) im allgemeinen 0.01 bis 50 Mol, vorzugsweise 0.1 bis 20 Mol einer Säure ein.

Im allgemeinen löst man die Verbindung der Formel (IV) in einem geeigneten Verdünnungsmittel, gibt dann die erforderliche Säuremenge dazu und engt die Mischung unter vermindertem Druck über mehrere Stunden langsam ein.

In einer besonderen Durchführungsform ist es auch möglich, das erfindungsgemäße Verfahren (b) und die Herstellung der dafür erforderlichen Vorprodukte der Formel (IV) in einem Reaktionsschritt in einem sogenannten Eintopfverfahren durchzuführen.

Dabei gibt es die Möglichkeit, als Ausgangsstoffe die Verbindungen der Formel (XXIII) zu wählen und diese nacheinander im Eintopfverfahren mit Aminen der Formel (VI) und anschließend mit Säure gemäß dem erfindungsgemäßen Verfahren (b) umzusetzen oder alternativ, als Ausgangsstoffe die Verbindungen der Formel (XXI) zu wählen und diese nacheinander im Eintopfverfahren entweder mit (Thio)Chlorameisensäureestern der Formel (XXII), dann mit Aminen der Formel (VI), oder mit Iso(thio)cyanaten der Formel (III) und anschließend mit Säure gemäß dem erfindungsgemäßen Verfahren (b) umzusetzen.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester, oder Sulfoxide, wie Dimethylsulfoxid.

Das erfindungsgemäße Verfahren (c) kann gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 120 °C, vorzugsweise bei Temperaturen zwischen 20 °C und 50 °C.

Zur Durchführung des erfindungsgemäßen Verfahrens (c) setzt man auf 1 Mol substituiertes 1,2,4-Triazol-3-(thi)on der Formel (V) im allgemeinen 1 bis 5 Mol, vorzugsweise 1.0 bis 2.5 Mol, Aminoverbindung der Formel (VI) und gegebenenfalls 0.1 bis 2 Mol, vorzugsweise 1.0 bis 1.2 Mol, Reaktionshilfsmittel ein.

Die Reaktionsdurchführung, Aufarbeitung und Isolierung der Reaktionsprodukte erfolgt nach allgemein üblichen Methoden.

In einer besonderen Durchführungsform ist es auch möglich, das erfindungsgemäße Verfahren (c) und die Herstellung der dafür erforderlichen Vorprodukte der Formel (V) in einem Reaktionsschritt in einem sogenannten Eintopfverfahren durchzuführen.

Man geht dabei von Verbindungen der Formel (II) aus und setzt diese nacheinander im Eintopfverfahren zunächst mit (Thio)Chlorameisensäureestern der Formel (XXII) und anschließend mit Aminen der Formel (VI) gemäß dem erfindungsgemäßen Verfahren (c) um.

Das erfindungsgemäße Verfahren (d) wird vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Verfahren (d) wird vorzugsweise in Gegenwart eines Reaktionshilfsmittels durchgeführt. Es können hierbei die gleichen Reaktionshilfsmittel eingesetzt werden, die oben für das erfindungsgemäße Verfahren (c) angegeben sind.

Die Reaktionstemperaturen können bei dem erfindungsgemäßen Verfahren (d) in einem größeren Bereich variiert werden. Im allgemeinen arbeitet man bei Temperaturen zwischen 0 °C und 150 °C, vorzugsweise bei Temperaturen zwischen 10 °C und 120 °C.

Zur Durchführung des erfindungsgemäßen Verfahrens (d) setzt man auf 1 Mol 4,5-Diamino-1,2,4-triazol-3-(thi)on der Formel (II) im allgemeinen 1 bis 2 Mol, vorzugsweise 1.0 bis 1.5 Mol, (Thio)Urethan der Formel (VII) ein.

Die Reaktionsdurchführung, Aufarbeitung und Isolierung der Produkte der Formel (I) erfolgt nach allgemein üblichen Methoden.

Das erfindungsgemäße Verfahren (e) wird vorzugsweise in Gegenwart eines polaren Lösungsmittels durchgeführt. Als solche kommen vorzugsweise Wasser, Alkohole wie Methanol, Ethanol, Propanol, Isopropanol, Butanol, Isobutanol, sec-Butanol und tert-Butanol, Etheralkohole wie Methoxyethanol und Ethoxyethanol, oder Ether wie Diethylether, Dipropylether, Diisopropylether, Tetrahydrofuran und Dioxan, in Betracht.

Die Reaktionstemperaturen können bei der zweiten Stufe von Verfahren (e) in einem größeren Bereich variiert werden. Im allgemeinen arbeitet man bei Temperaturen zwischen -20°C und +100°C, vorzugsweise zwischen 0°C und +30°C.

Zur Durchführung des erfindungsgemäßen Verfahrens (e) setzt man auf 1 Mol 4-Oxyalkylidenamino-5-amino-1,2,4-triazol-3-(thi)on der Formel (VIII) im allgemeinen 0.5 bis 5 Mol, vorzugsweise 1 bis 3 Mol, Hydridkomplex der Formel (IX) ein.

Die Reaktionsdurchführung, Aufarbeitung und Isolierung der Reaktionsprodukte erfolgt nach allgemein üblichen Methoden.

Das erfindungsgemäße Verfahren (f) wird unter Verwendung eines Reduktionsmittels und gegebenenfalls eines Katalysators durchgeführt. Geeignete Systeme aus Reduktionsmitteln und Katalysatoren sind beispielsweise Wasserstoff in Kombination mit üblichen Hydrierungskatalysatoren, wie z.B. Raney-Nickel, Palladium oder Platin, ferner auch gegebenenfalls komplexe Metallhydride, wie z.B. Lithiumalanat, Natriumboranat und Natriumcyanoborhydrid, gegebenenfalls in Kombination mit sauren Katalysatoren, wie z.B. Salzsäure oder Essigsäure.

Verfahren (f) wird vorzugsweise in Gegenwart eines Verdünnungsmittels durchgeführt. Es können hierbei die gleichen Lösungsmittel verwendet werden, die oben für das erfindungsgemäße Verfahren (e) angegeben sind.

Die Reaktionstemperaturen können bei dem erfindungsgemäßen Verfahren (f) in einem größeren Bereich variiert werden. Im allgemeinen arbeitet man bei Temperaturen zwischen -20 °C und +100 °C, vorzugsweise bei Temperaturen zwischen 0 °C und +30 °C.

Die Reaktionsdurchführung, Aufarbeitung und Isolierung der Reaktionsprodukte erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen, wie z.B in Mais, vor allem im Nachauflauf-Verfahren.

Die Verbindungen der Formel (I) versickern extrem langsam im Boden; somit ist eine Belastung des Grundwassers praktisch ausgeschlossen.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); 2-Chlor-4-ethylamino-6-(3-cyanopropylamino)-1,3,5-triazin (CYANAZIN); 4-Amino-6-t-butyl-3-ethylthio-1,2,4-triazin-5(4H)-on (ETHIOZIN); 3,5-Diiod-4-hydroxybenzonitril (IOXYNIL); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

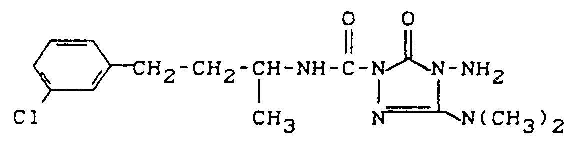

### (Verfahren (b) - mit Herstellung der Vorstufe im "EinTopf-Verfahren")

Eine Mischung aus 5,2 g (15 mmol) 2-Phenoxycarbonyl-4-(3-methyl-2-butyliden-amino)-5-dimethylamino-2,4-dihydro-3H-1,2,4-triazol-3-on, 3,36 g (18 mmol) 1-Methyl-3-(3-chlor-phenyl)-propylamin und 50 ml Tetrahydrofuran wird 24 Stunden bei 20°C gerührt. Anschließend wird im Wasserstrahlvakuum eingeengt, der Rückstand in 100 ml Ethanol aufgenommen und nach Zugabe von 20 ml Wasser und 5 ml konz. Salzsäure wird das Gemisch 3 Stunden bei 60°C/200 mbar gerührt. Nach Einengen im Wasserstrahlvakuum wird der Rückstand mit 150 ml Methylenchlorid/150 ml gesättigter Natriumhydrogencarbonat-Lösung in Wasser extrahiert, die organische Phase abgetrennt, mit Natriumsulfat getrocknet und filtriert. Das Filtrat wird eingeengt und der Rückstand durch Säulenchromatographie (Kieselgel und Cyclohexan/Essigsäureethylester 1:3) weiter gereinigt. Der Inhalt der Hauptfraktion wird durch Verreiben mit Ether/Petrolether zur Kristallisation gebracht und durch Absaugen isoliert.

Man erhält 3,3 g (73% der Theorie) 2-(1-Methyl-3-(3-chlor-phenyl)-propyl-amino-carbonyl)-4-amino-5-dimethylamino-2,4-dihydro-3H-1,2,4-triazol-3-on vom Schmelzpunkt 74°C.

### Beispiel 2

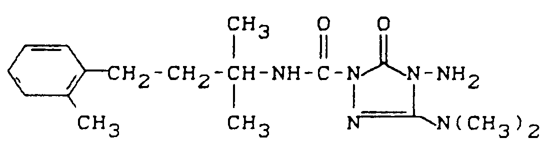

### (Verfahren (b) - mit Herstellung der Vorstufe im "Eintopf-Verfahren")

Eine Mischung aus 4,5 g (20 mmol) 4-(3-Methyl-2-butyliden-amino)-5-dimethylamino-2,4-dihydro-3H-1,2,4-triazol-3-on, 4,1 g (20 mmol) 1,1-Dimethyl-3-(2-methyl-phenyl)-propylisocyanat, 0,1 g 1,8-Diazabiyclo-[5.4.0]-undec-7-en und 100 ml Methylenchlorid wird 18 Stunden bei 20°C gerührt. Anschließend wird im Wasserstrahlvakuum eingeengt, der Rückstand in 100 ml Ethanol aufgenommen und die Mischung nach Zugabe von 20 ml Wasser und 5 ml konz. Salzsäure 3 Stunden bei 60°C/200 mbar gerührt. Nach Einengen im Wasserstrahlvakuum wird der Rückstand mit 200 ml Methylenchlorid/200 ml gesättigter wäßriger Natriumhydrogencarbonat-Lösung extrahiert, die oganische Phase abgetrennt, mit Magnesiumsulfat getrocknet und filtriert. Nach Einengen des Filtrats wird der Rückstand durch Verreiben mit Ether/Petrolether zur Kristallisation gebracht und das kristalline Produkt durch Absaugen isoliert.

Man erhält 4,5 g (65% der Theorie) 2-(1,1-Dimethyl-3-(2-methyl-phenyl)-propyl-amino-carbonyl)-4-amino-5-dimethylamino-2,4-dihydro-3H-1,2,4-triazol-3-on vom Schmelzpunkt 117°C.

Analog zu den Beispielen 1 und 2 und entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

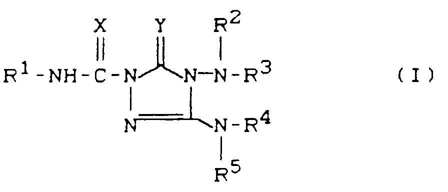

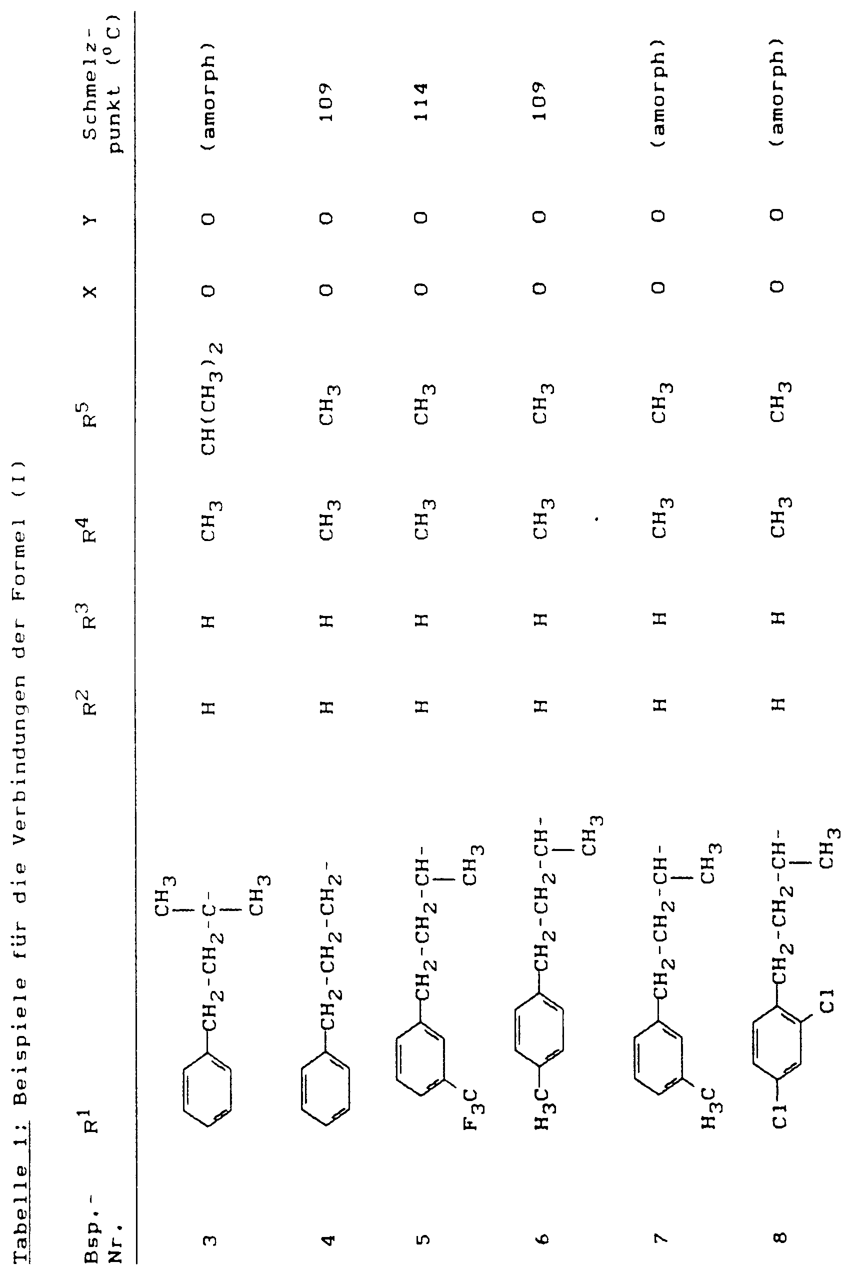

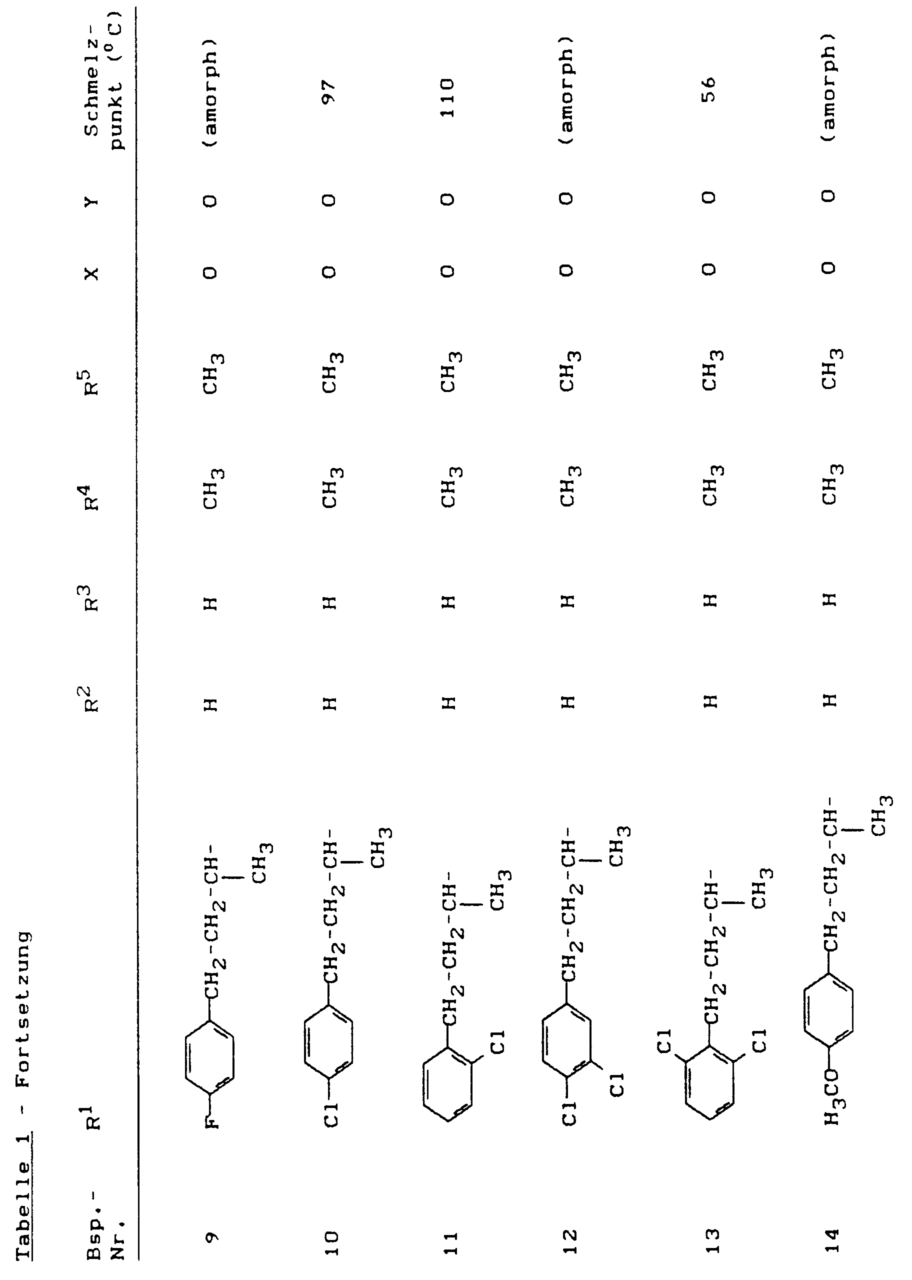

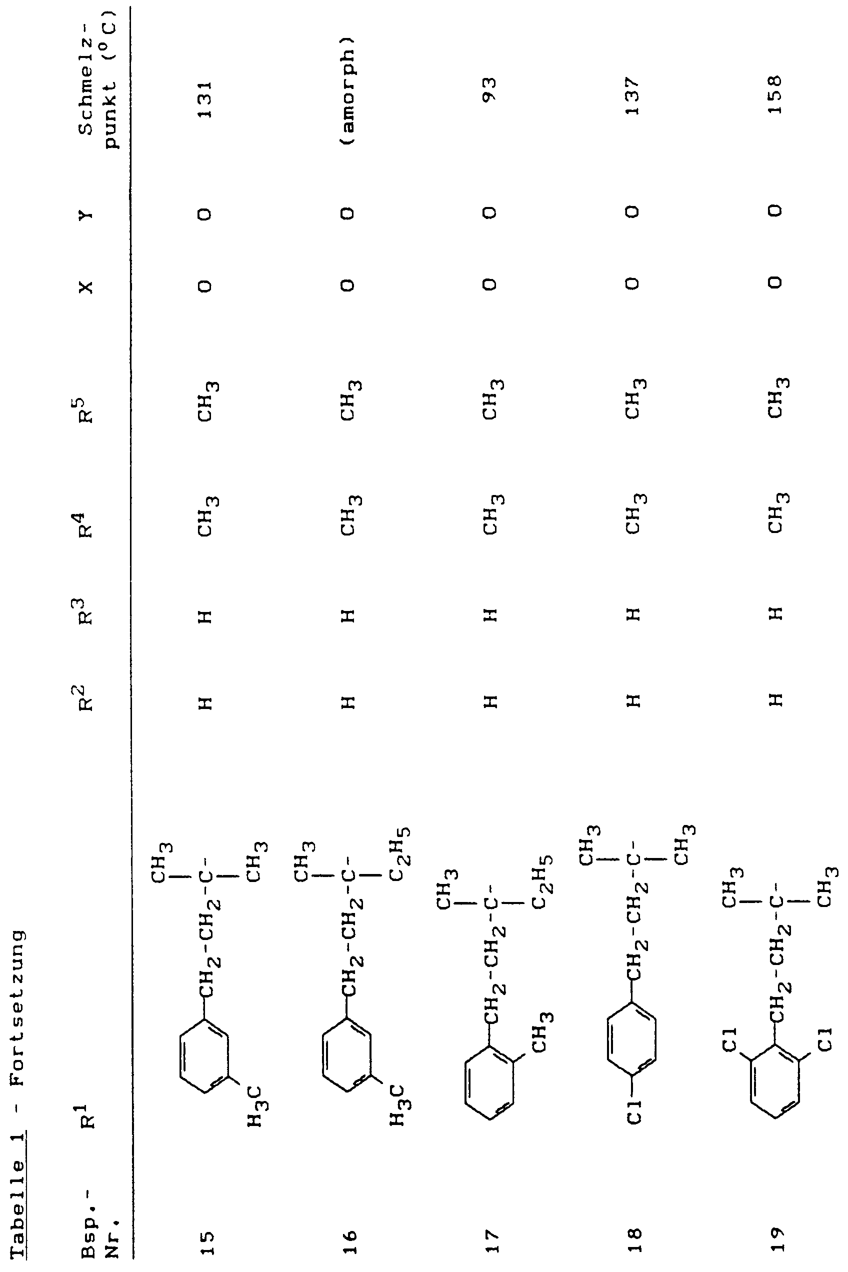

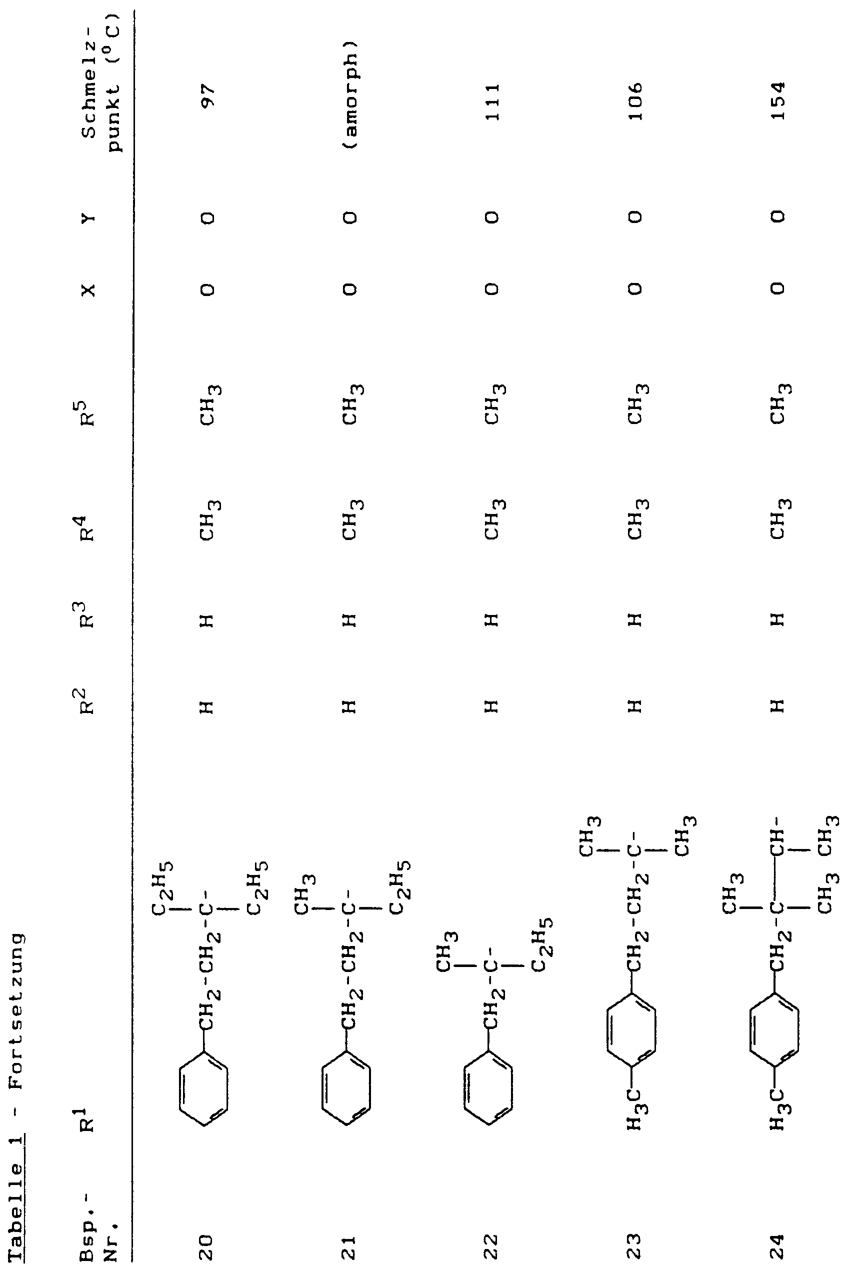

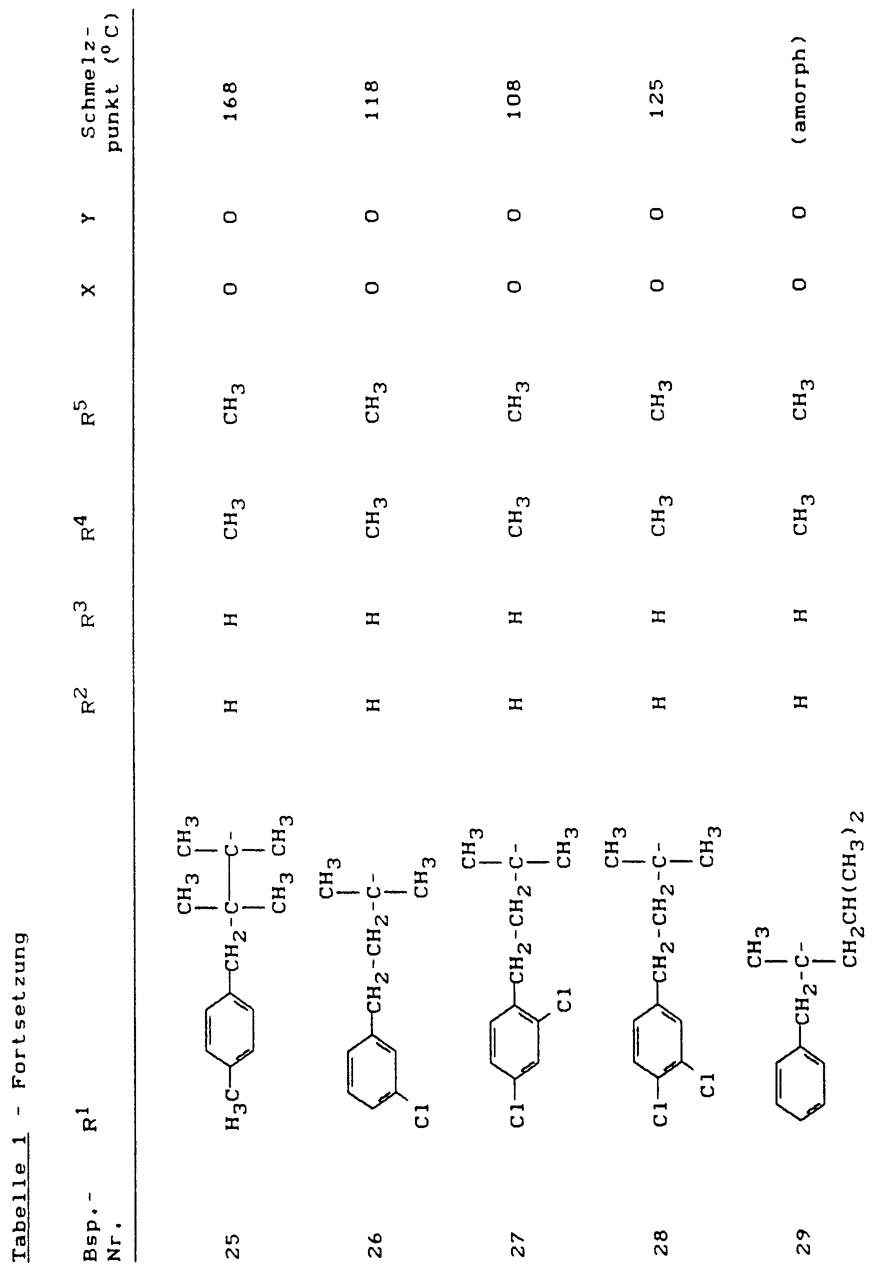

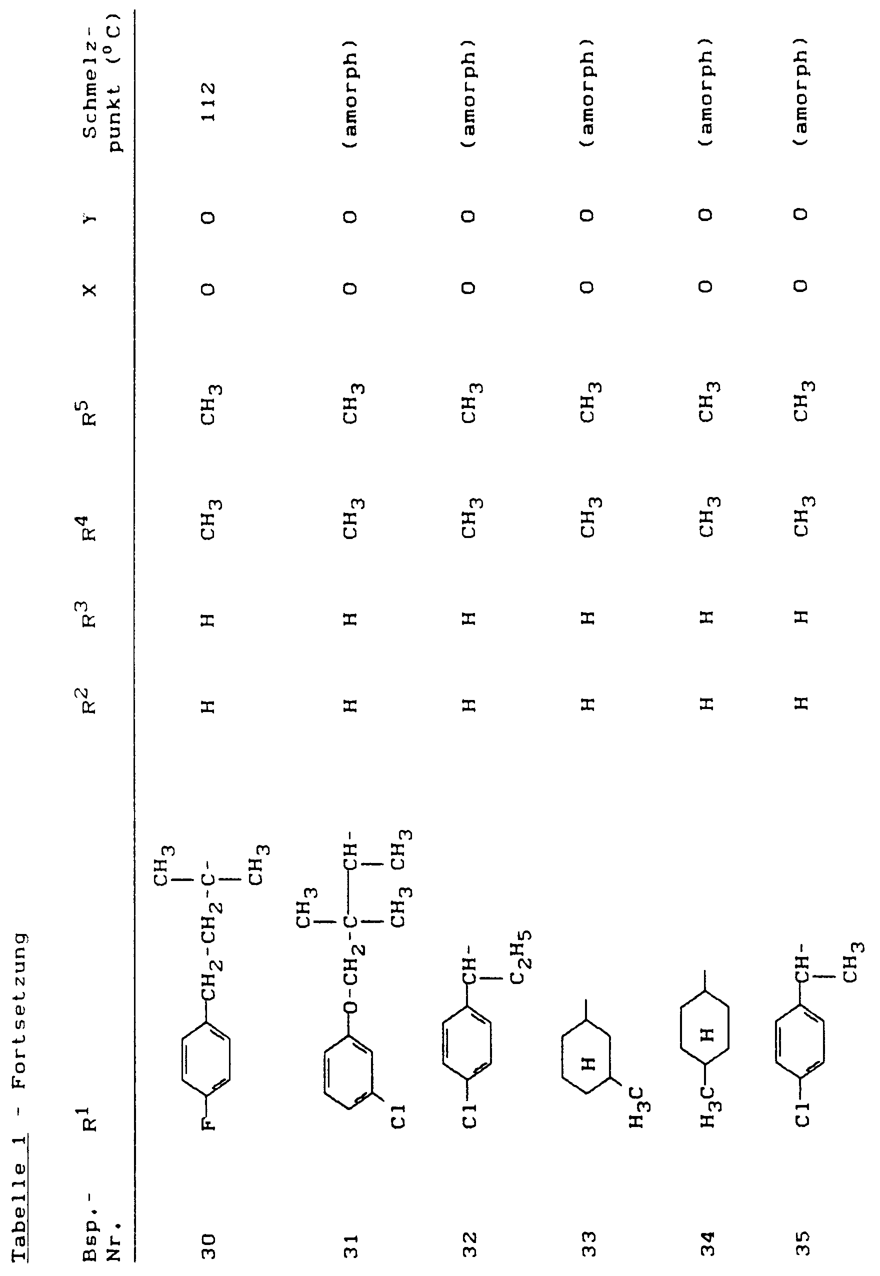

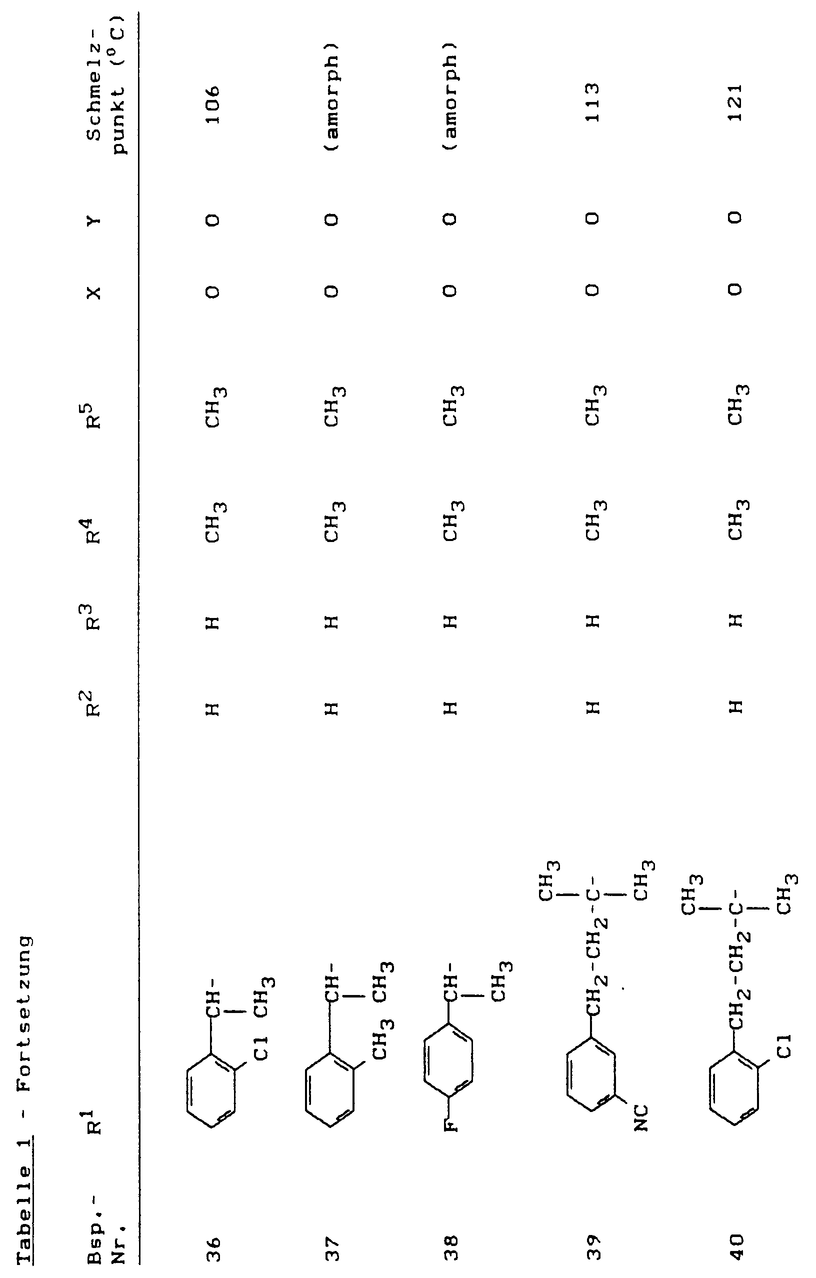

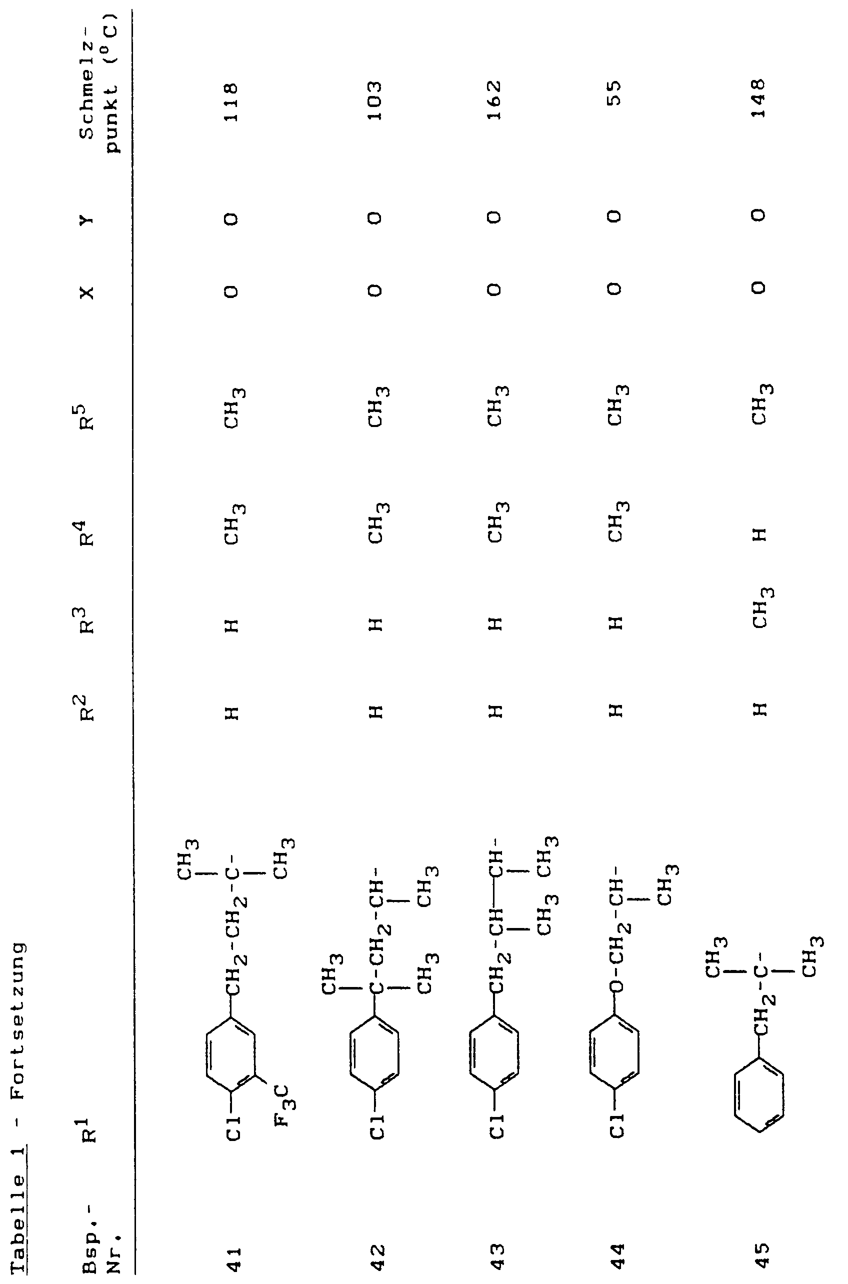

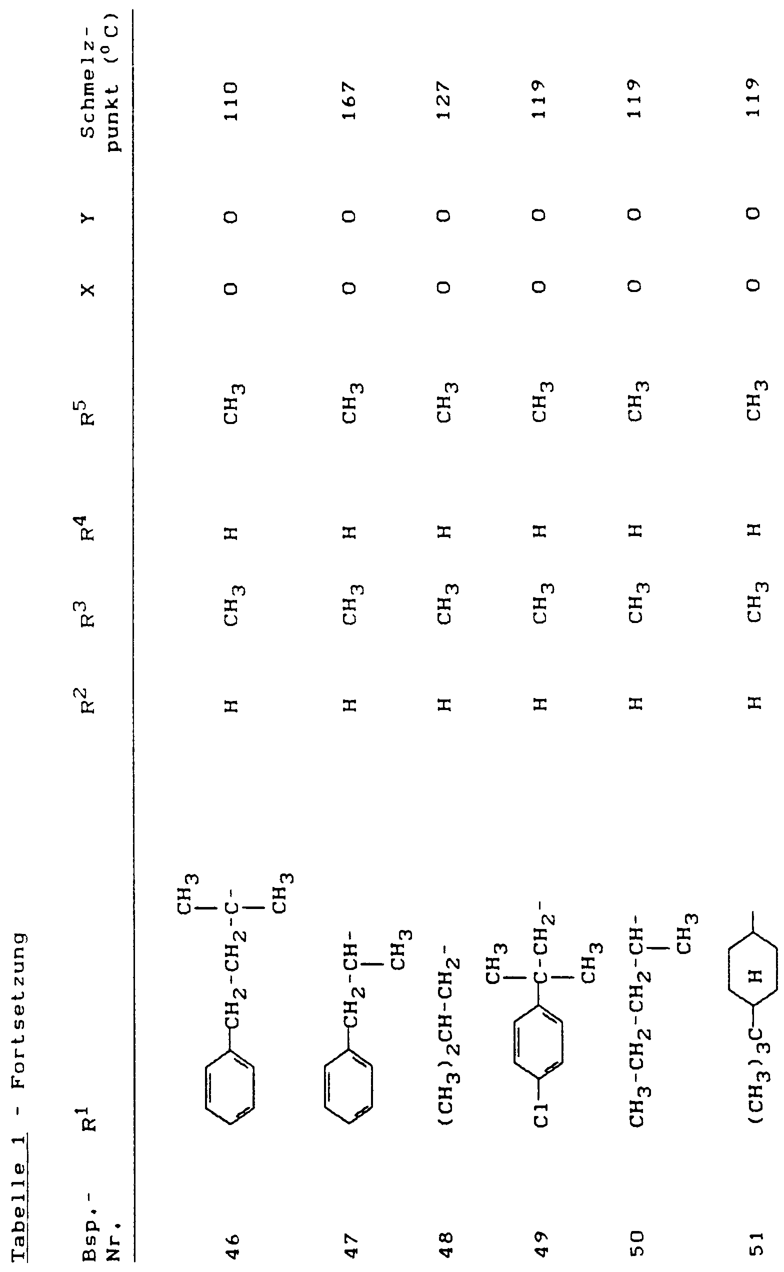

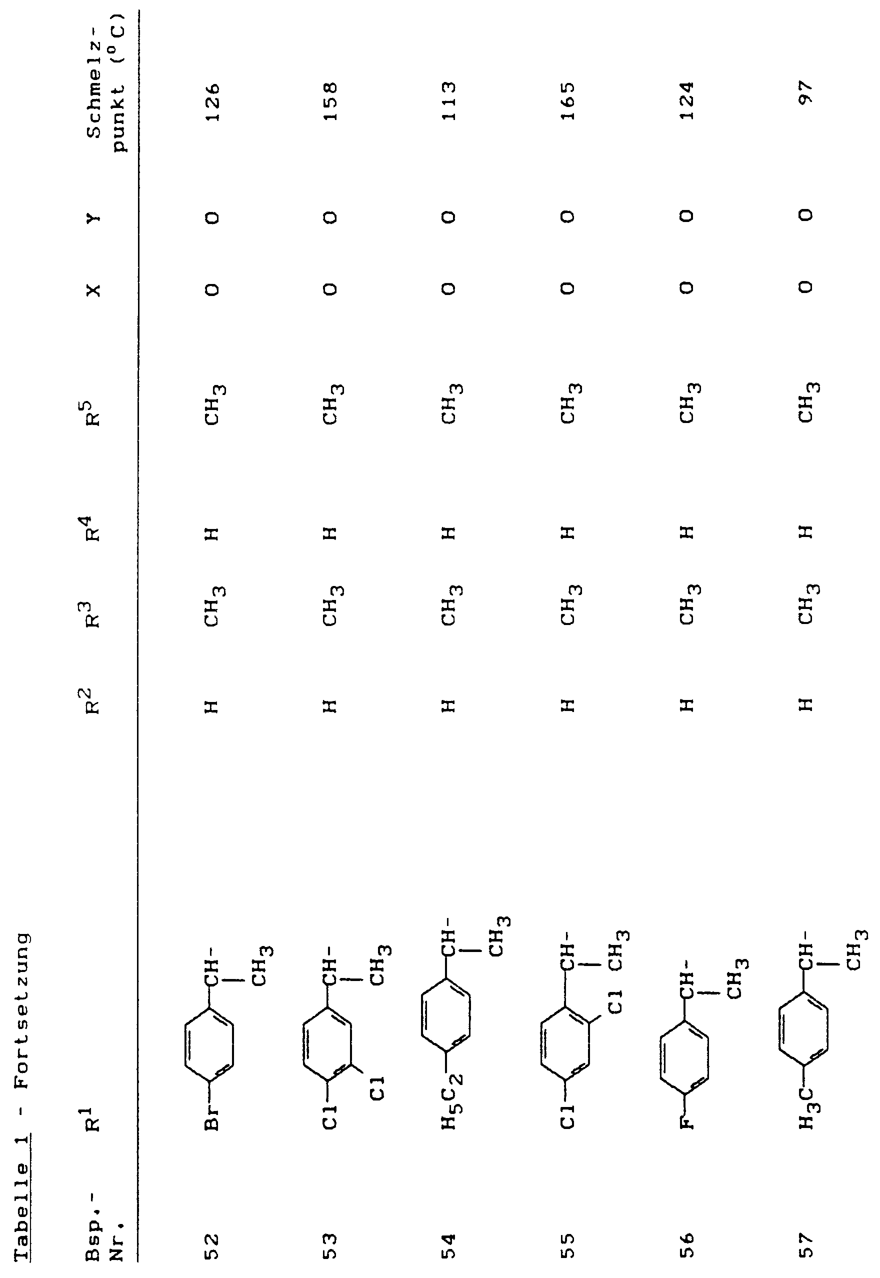

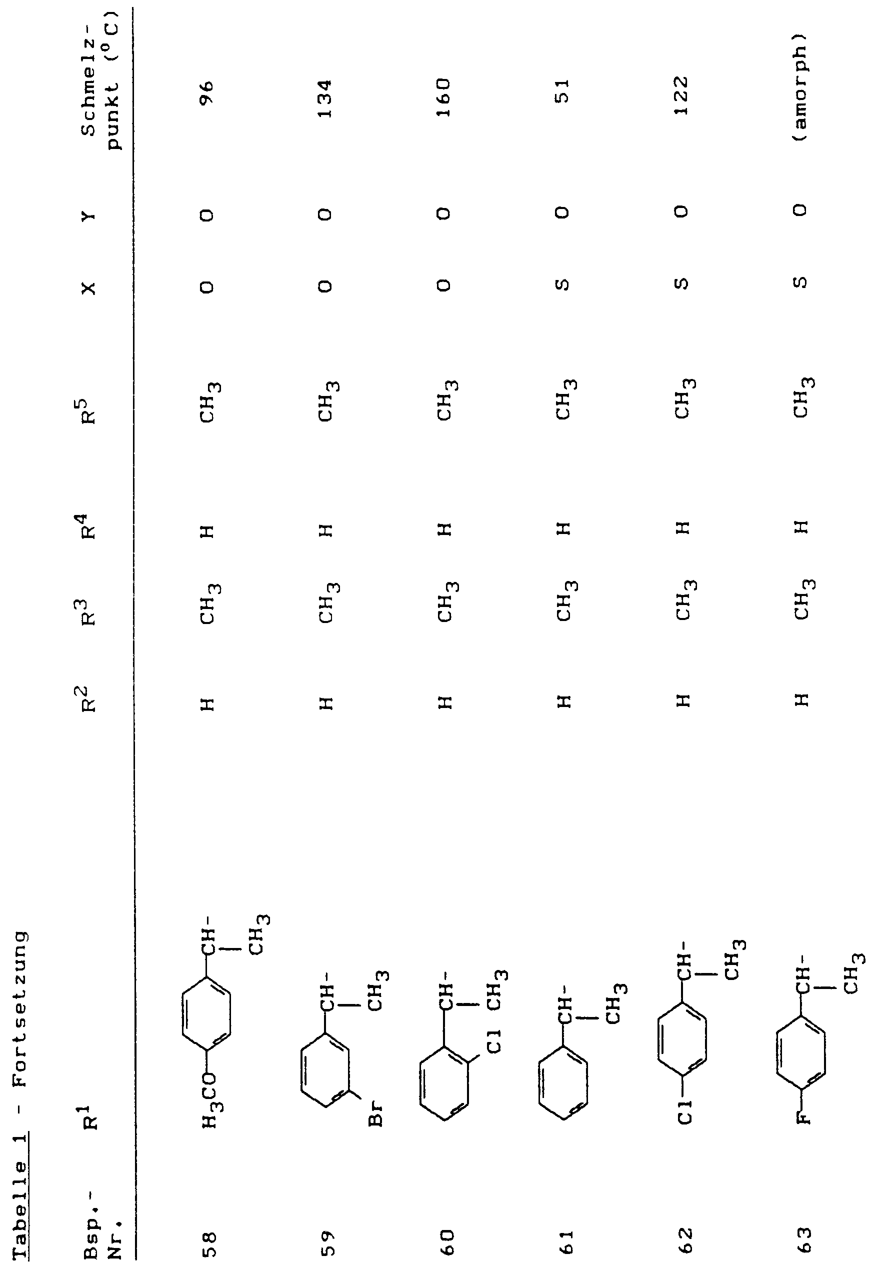

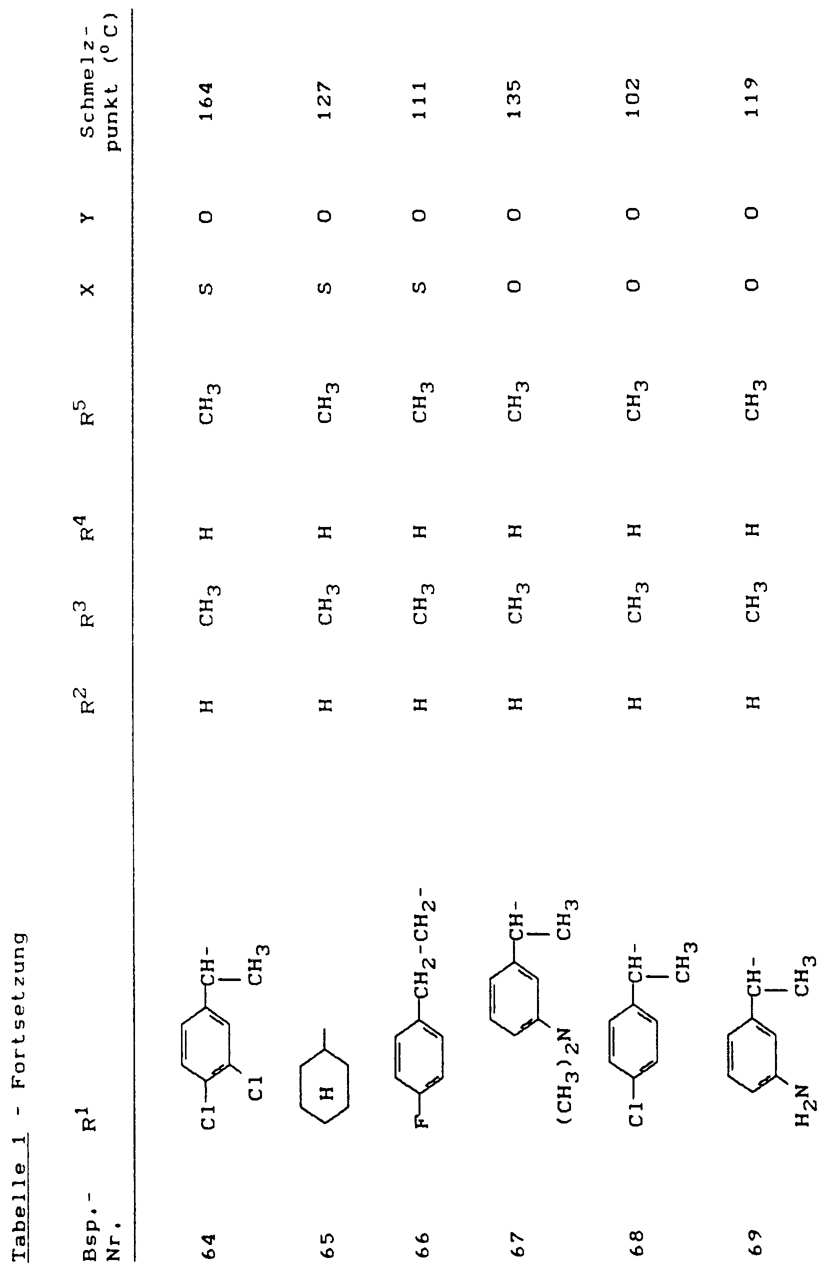

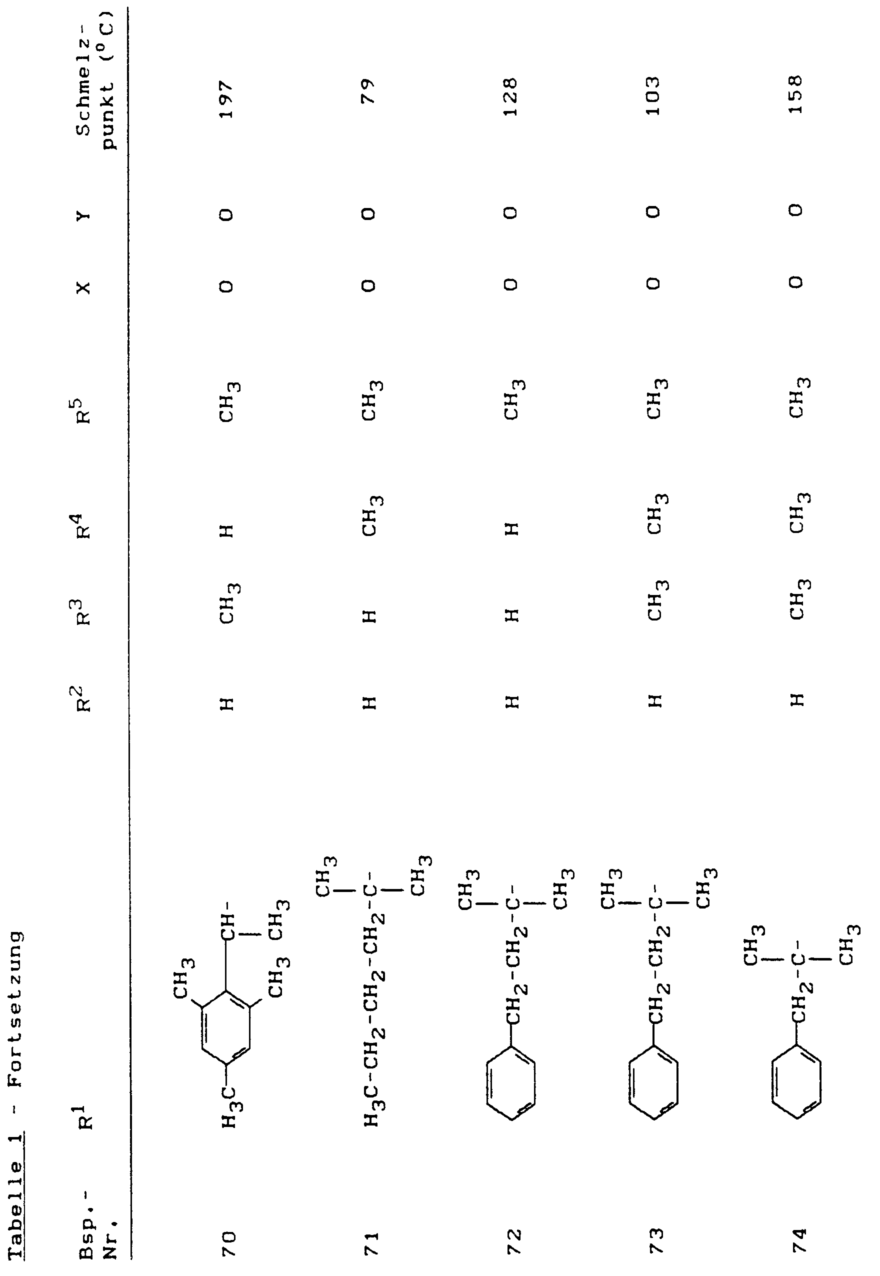

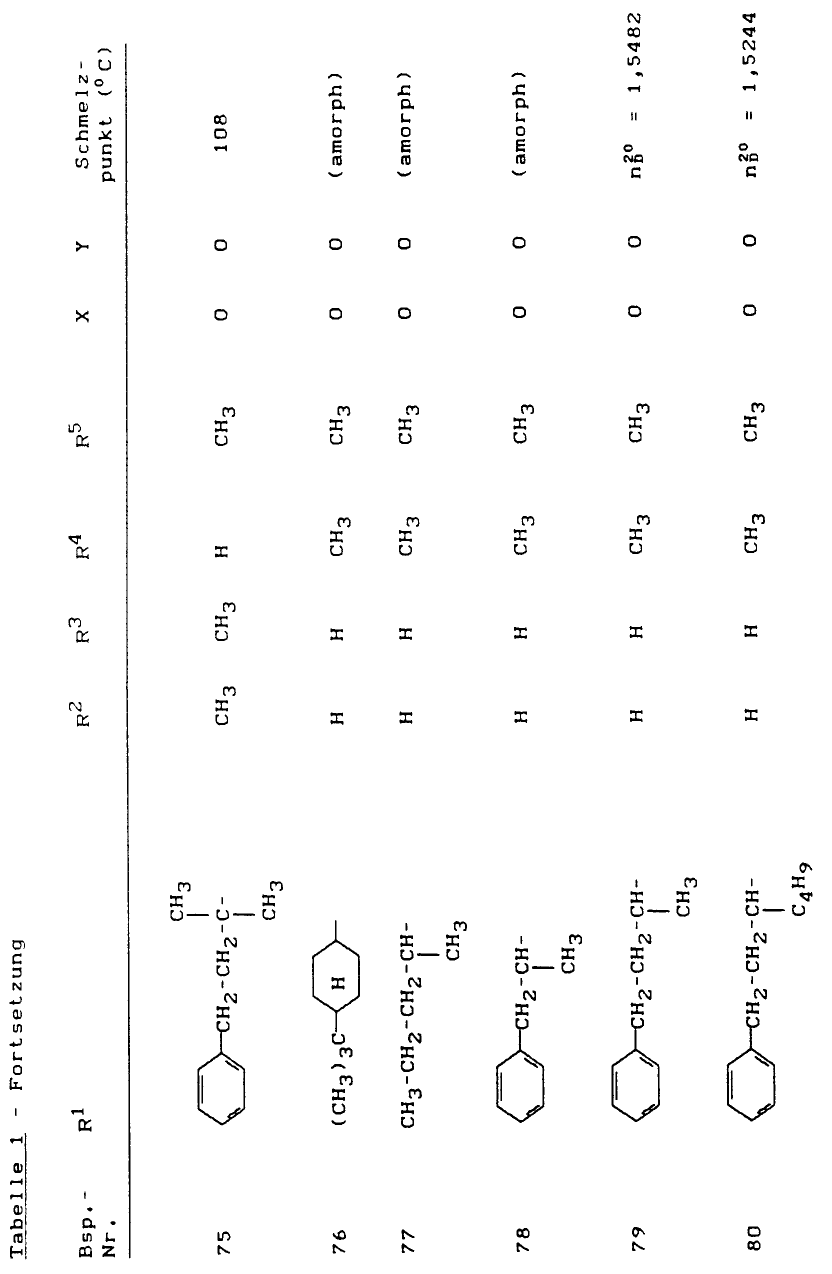

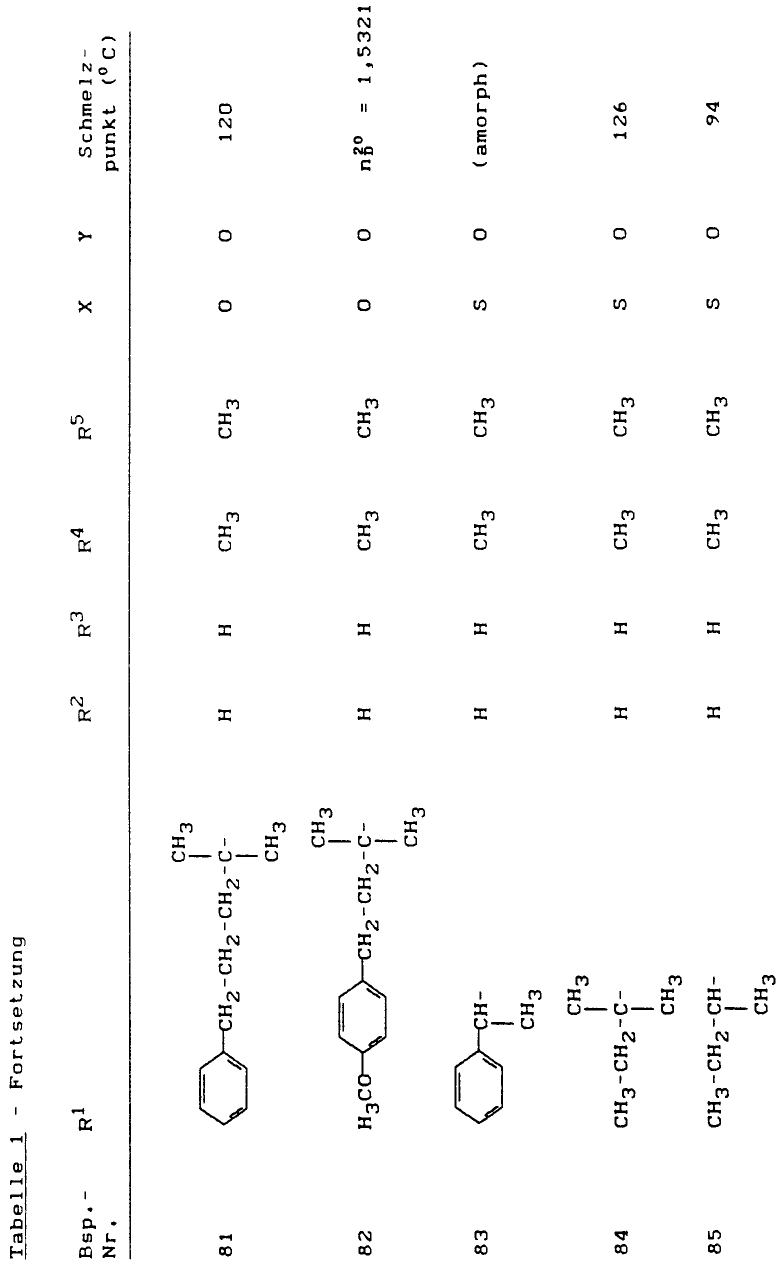

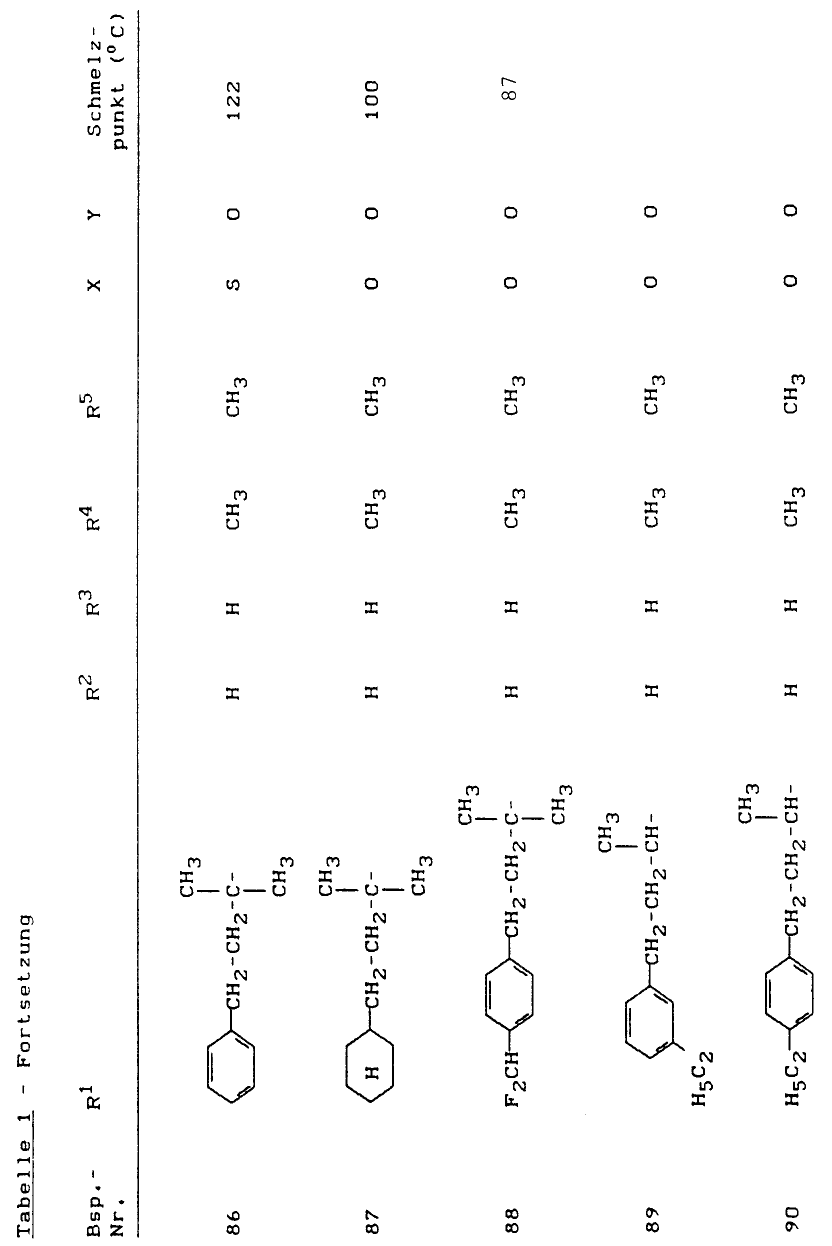

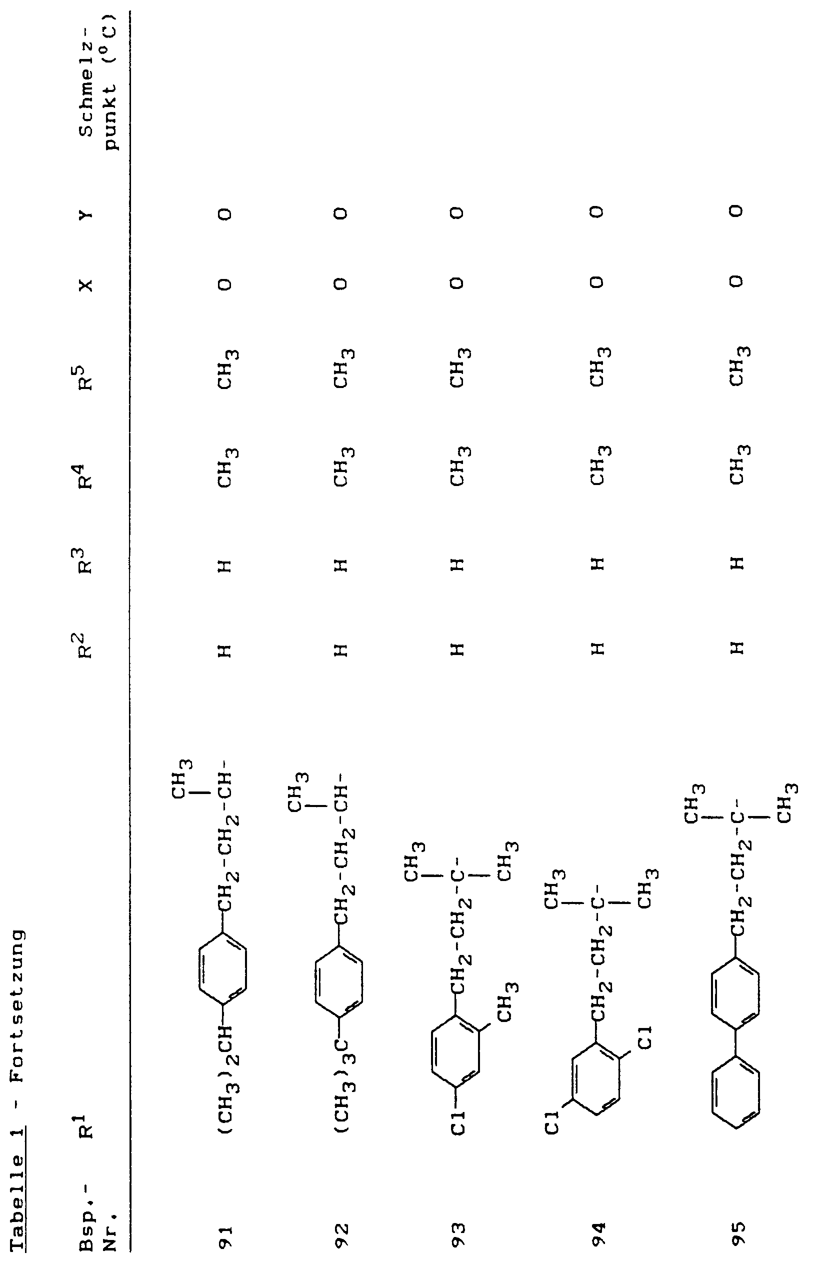

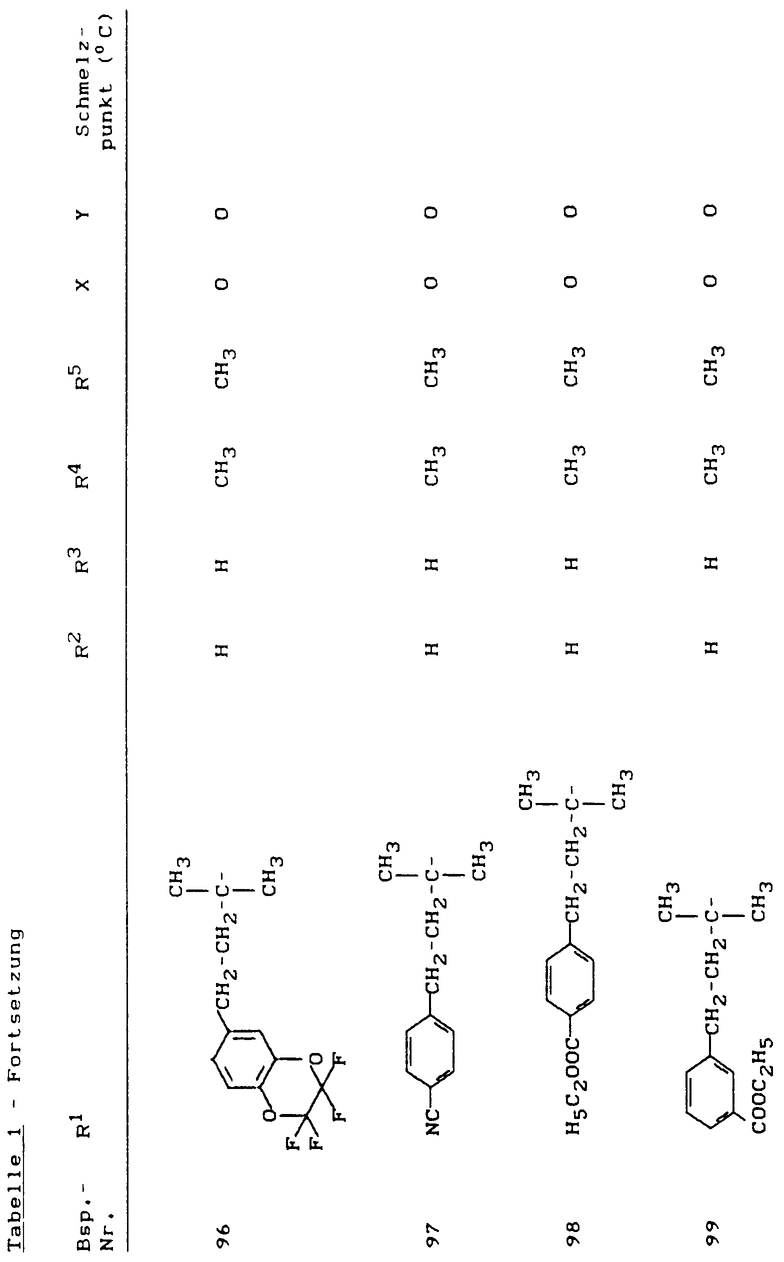

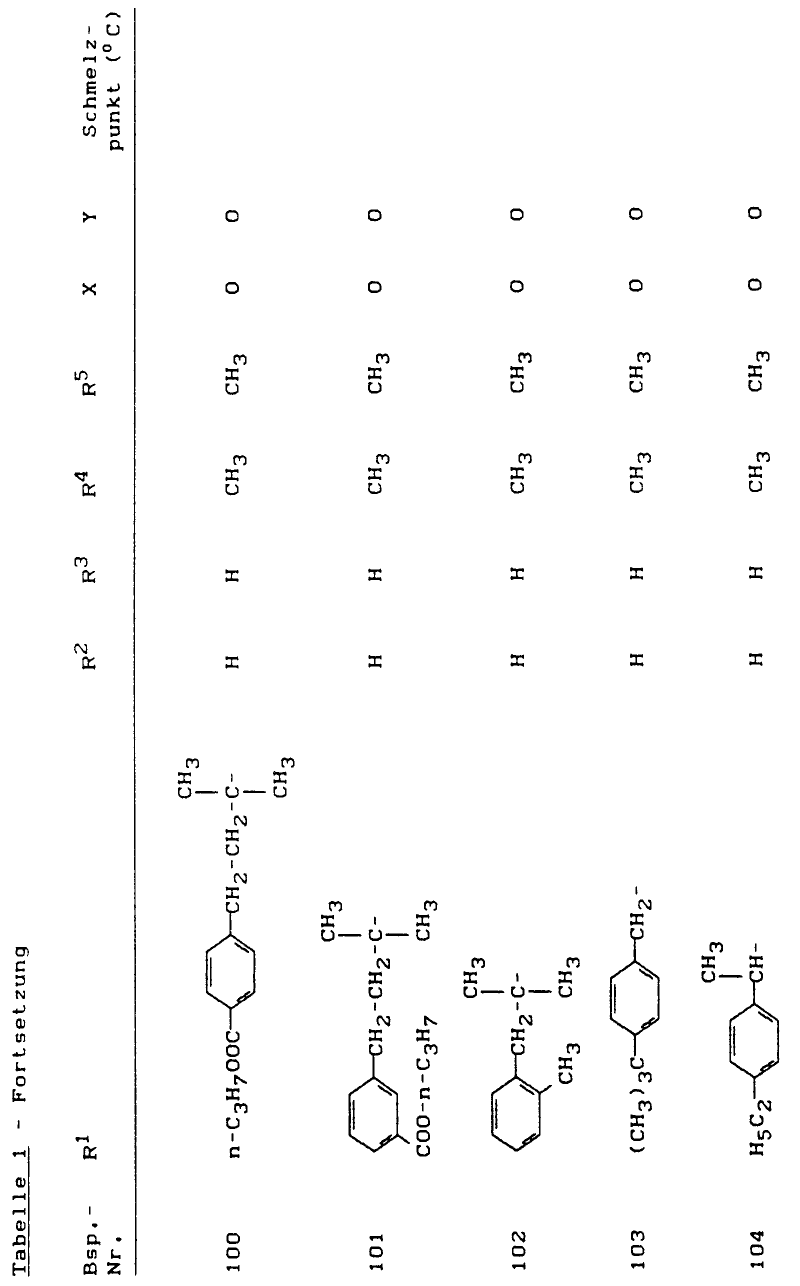

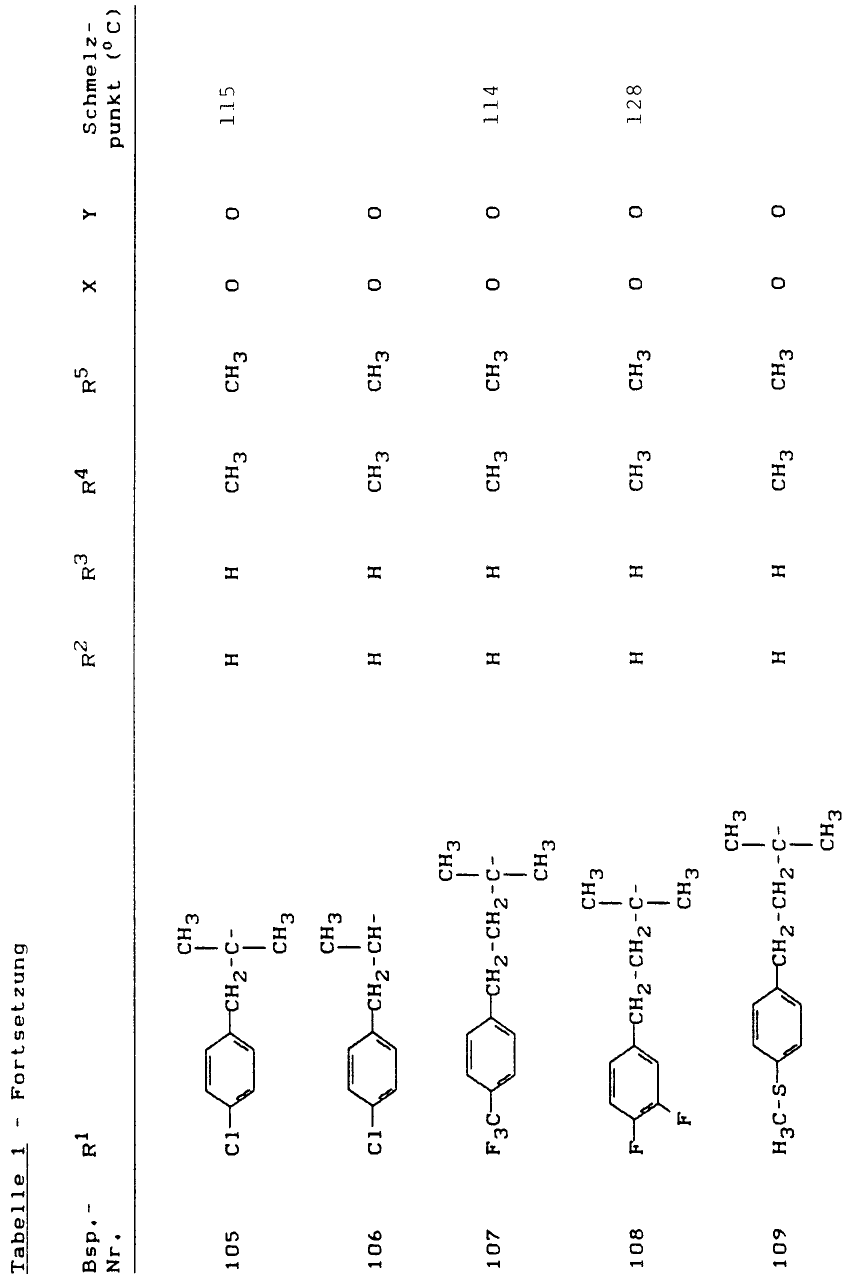

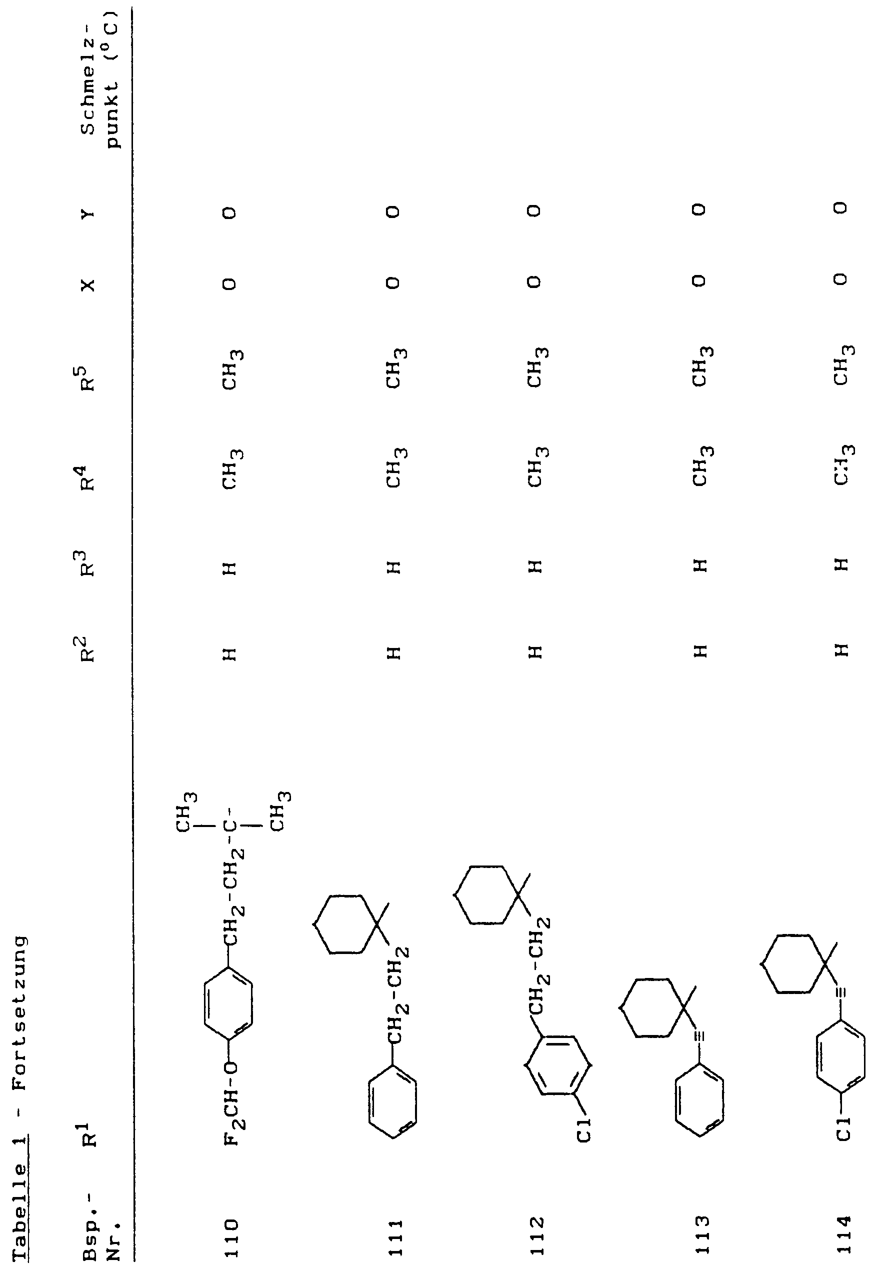

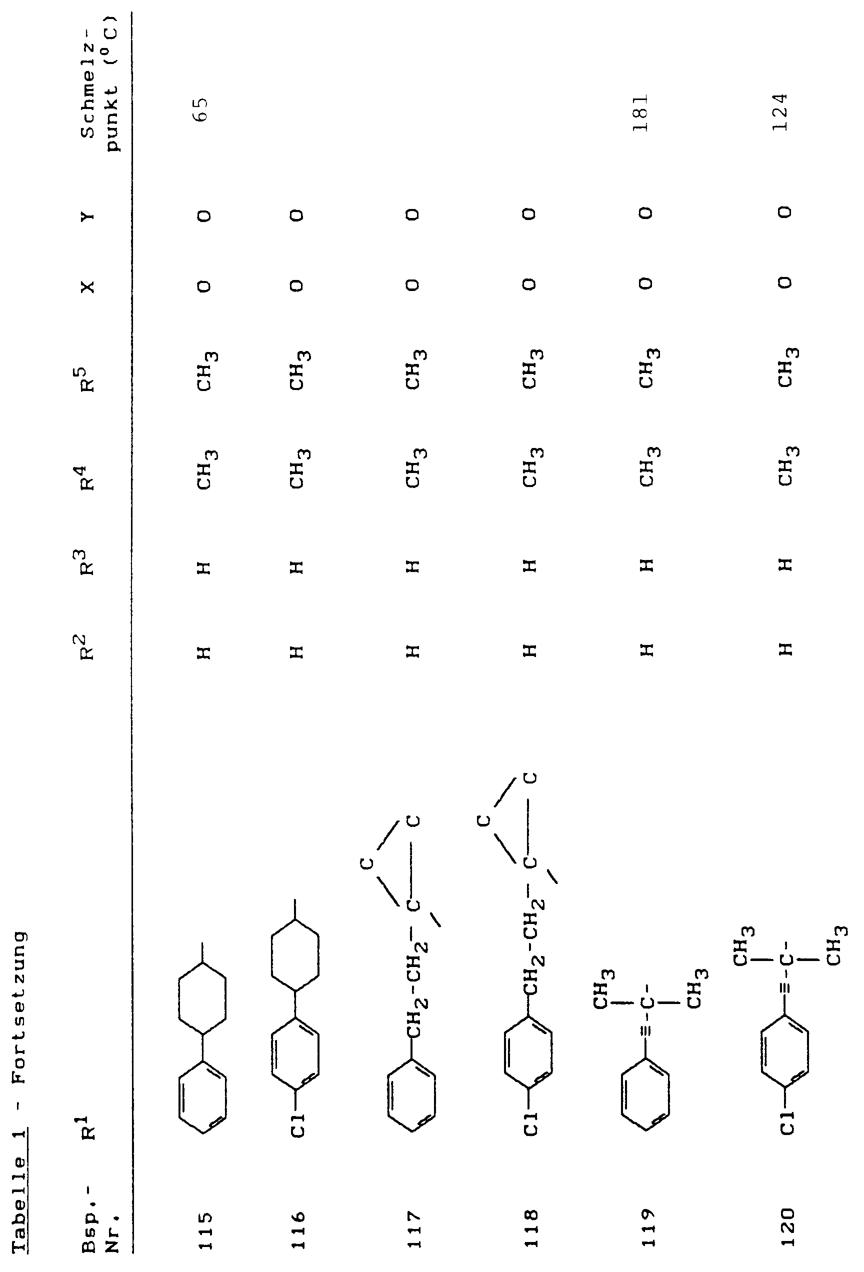

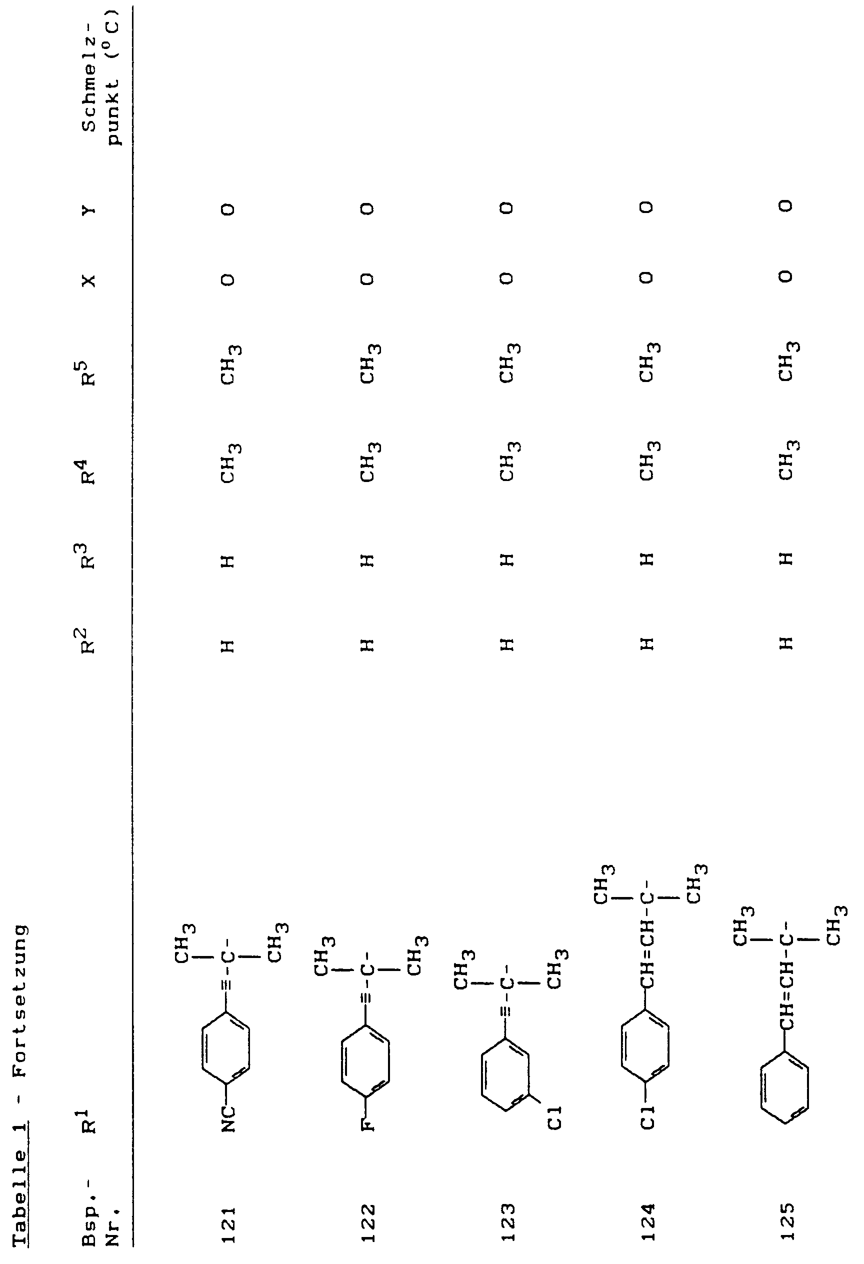

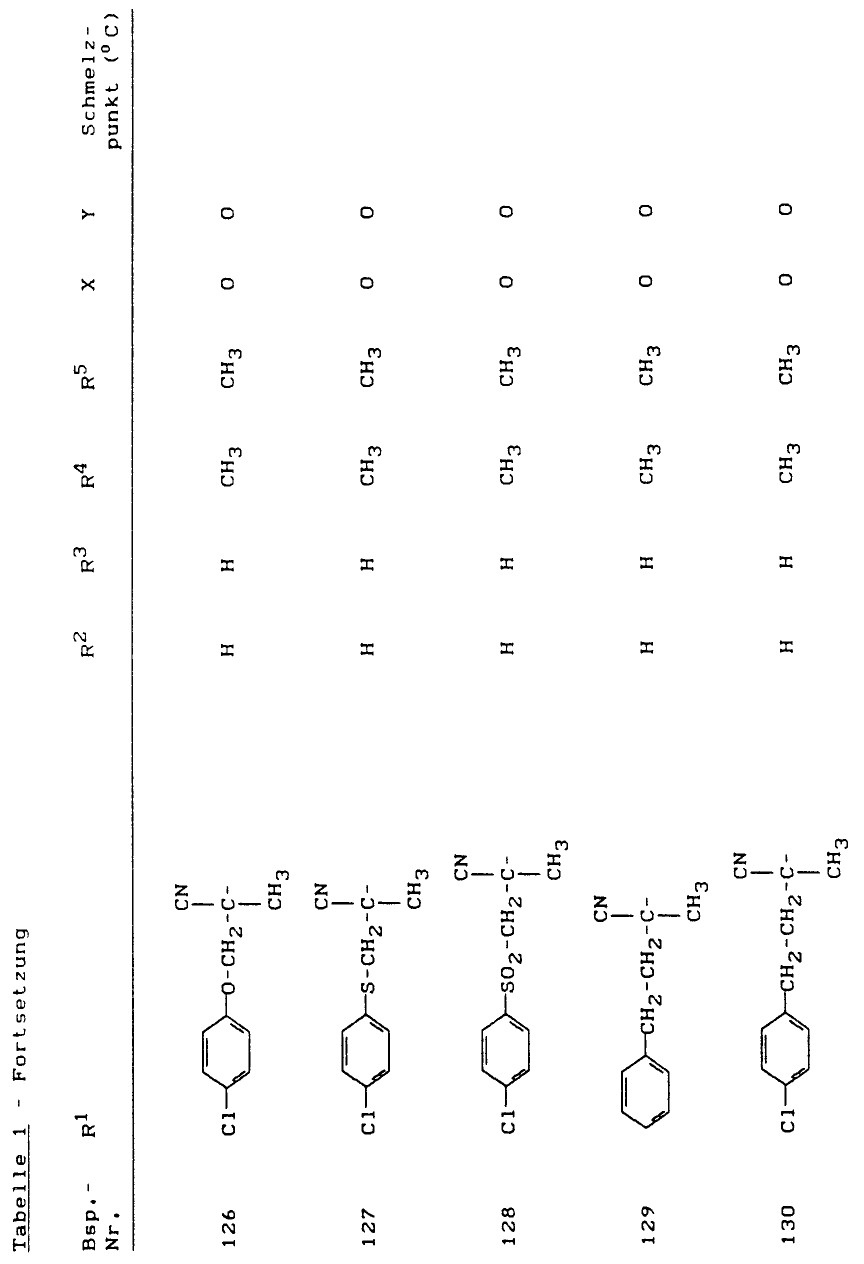

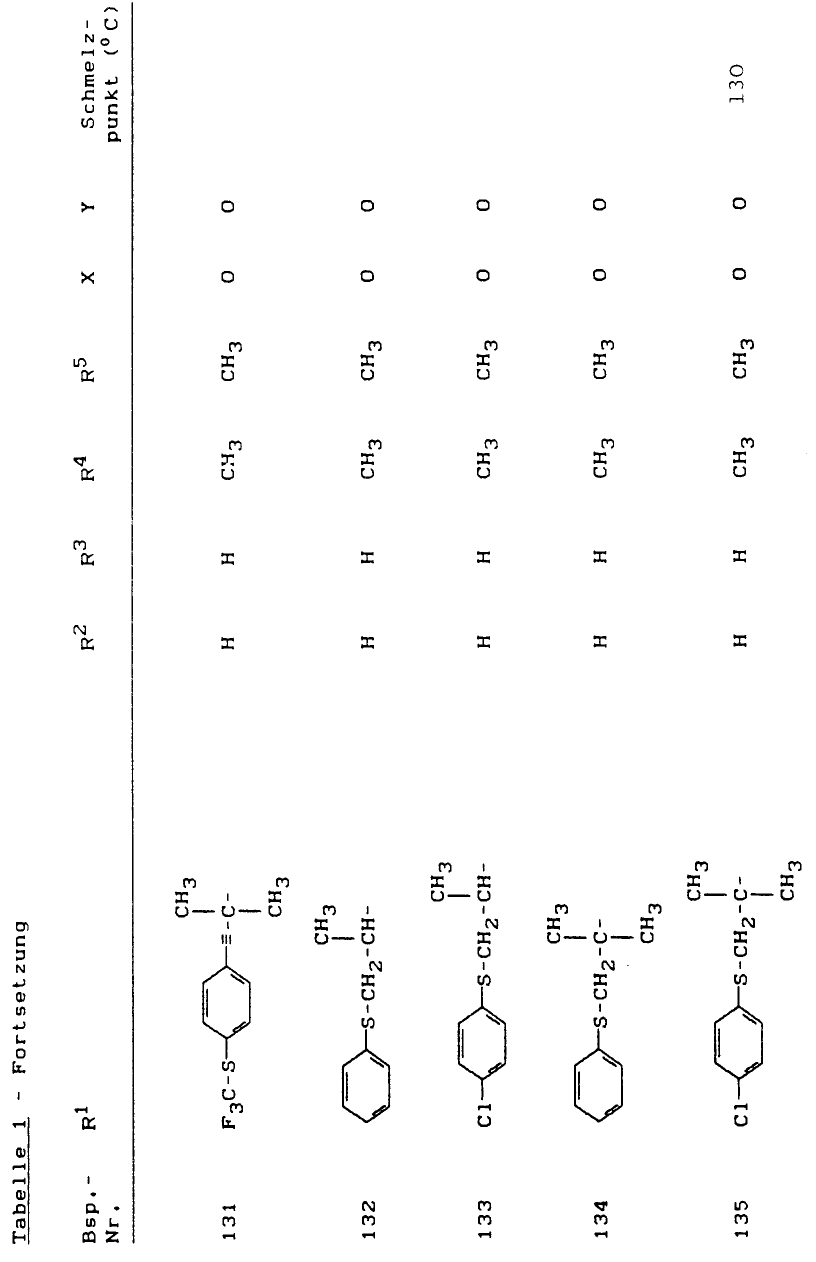

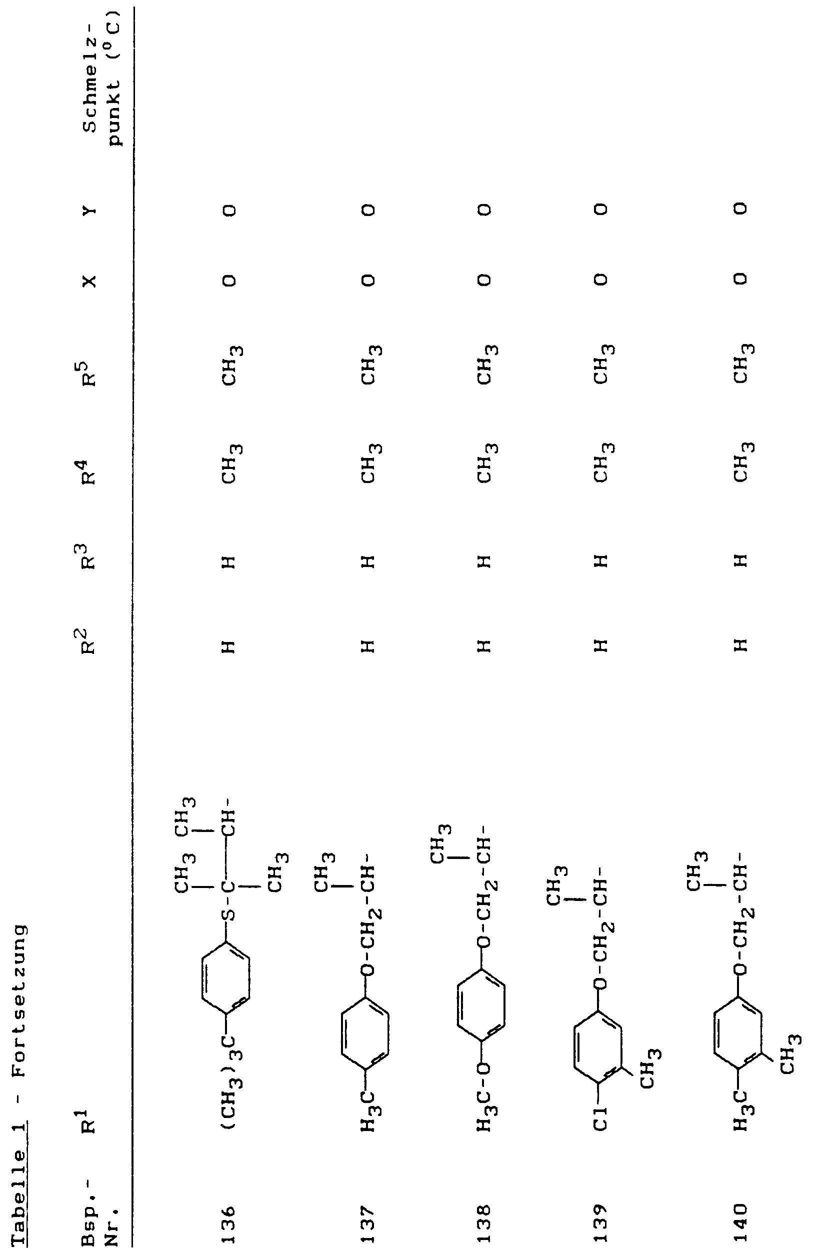

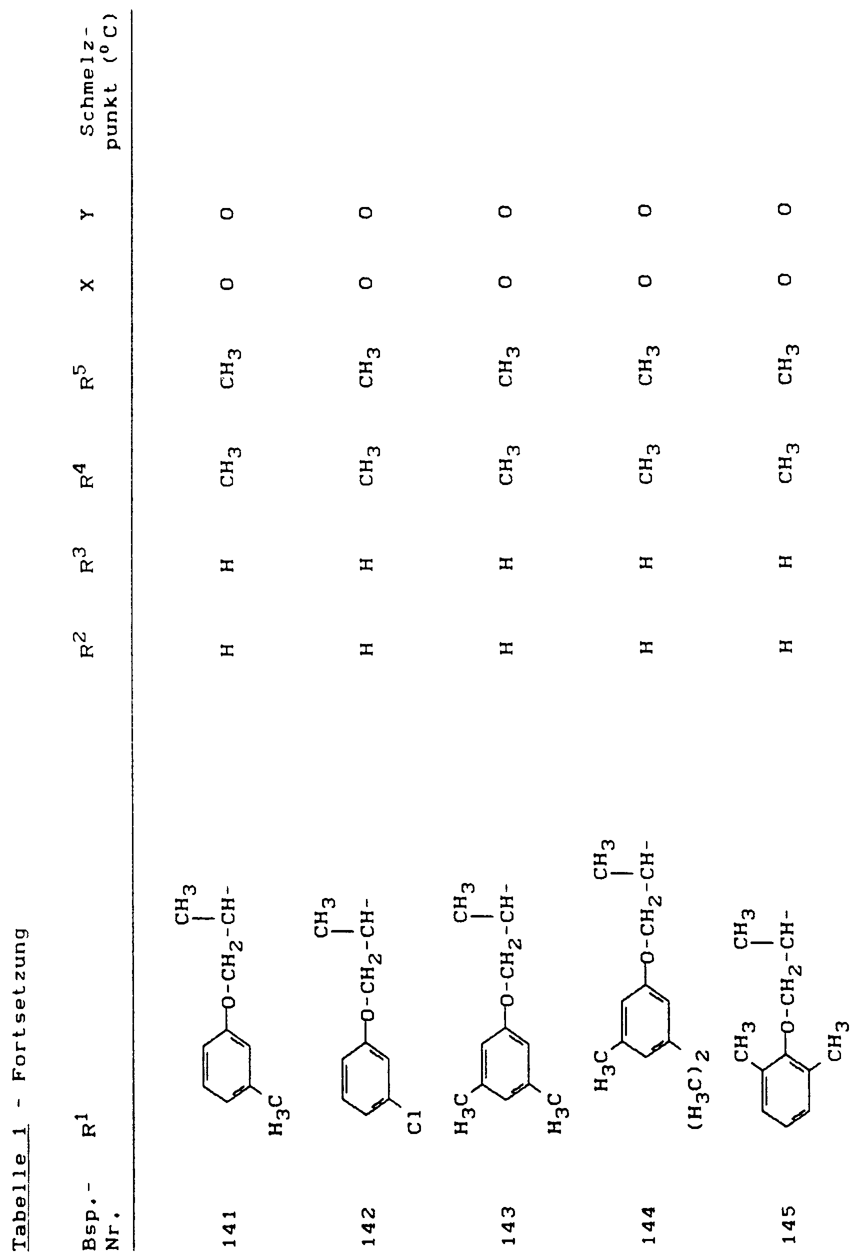

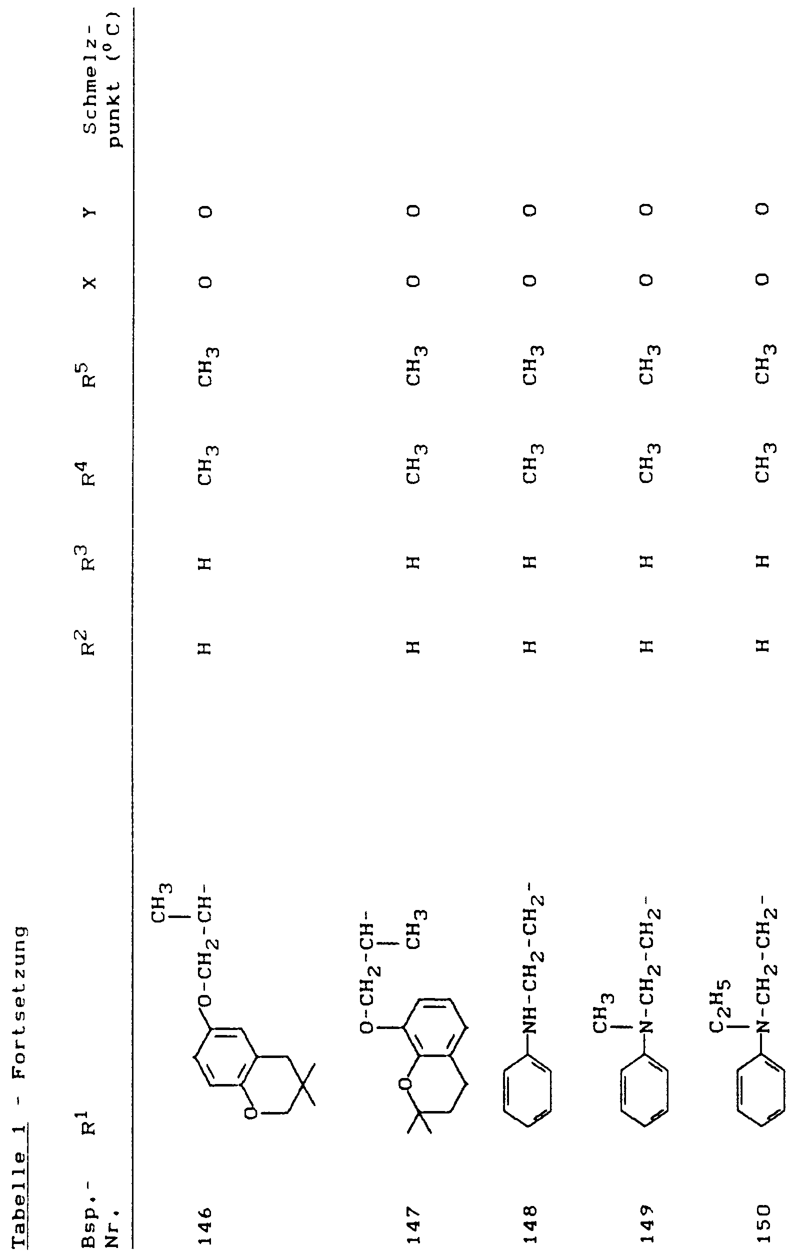

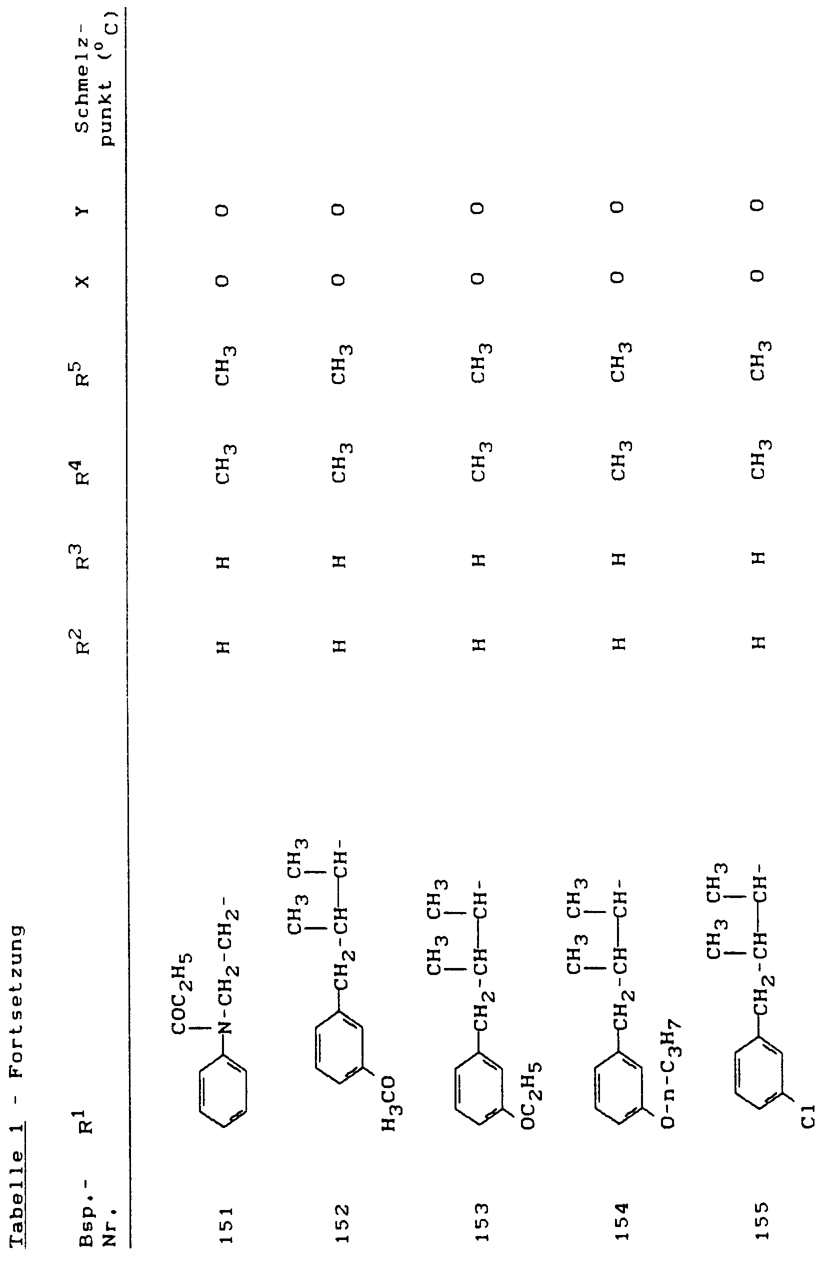

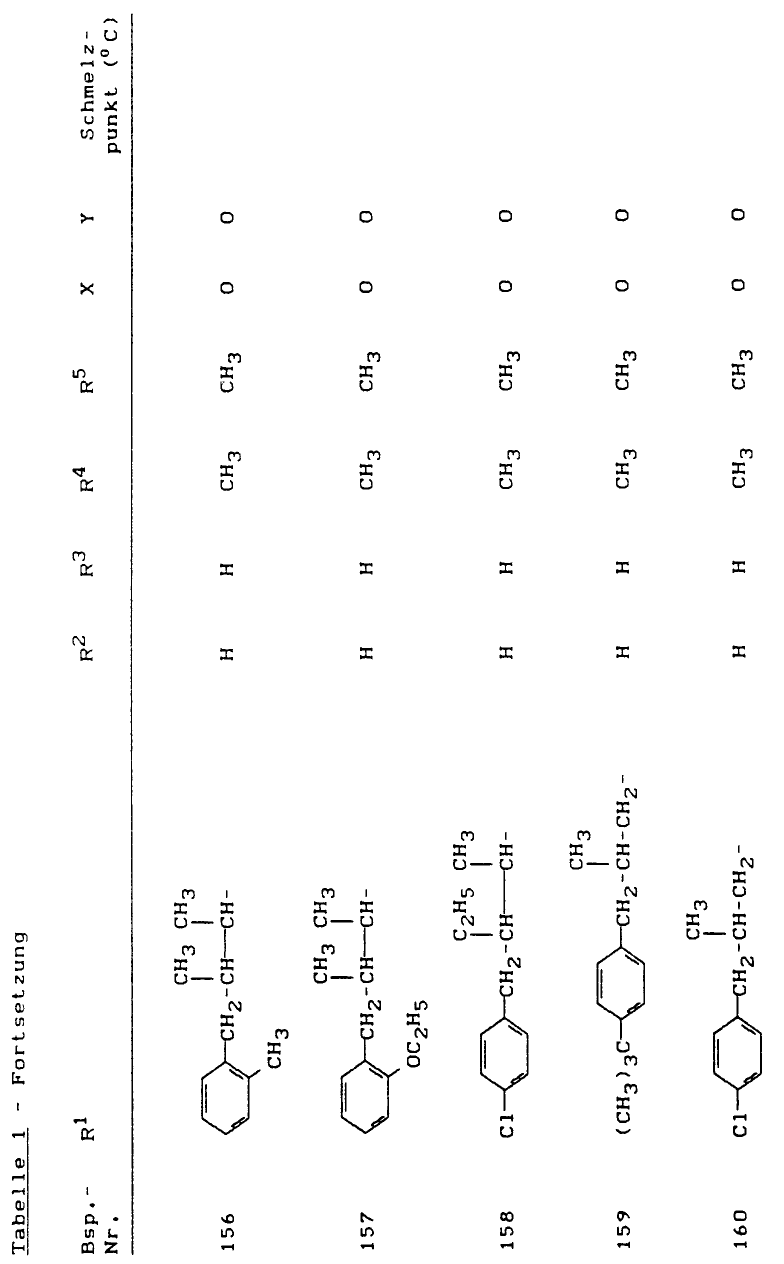

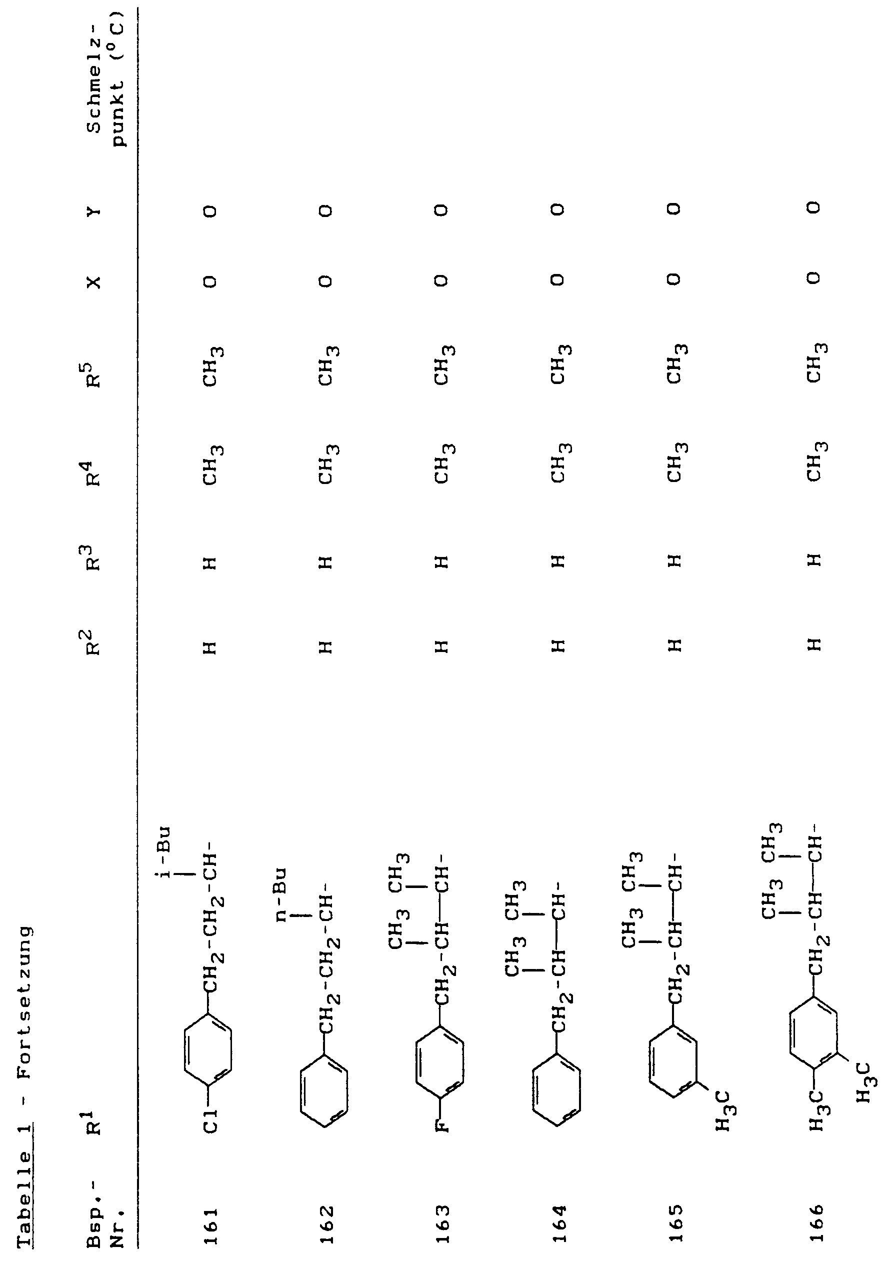

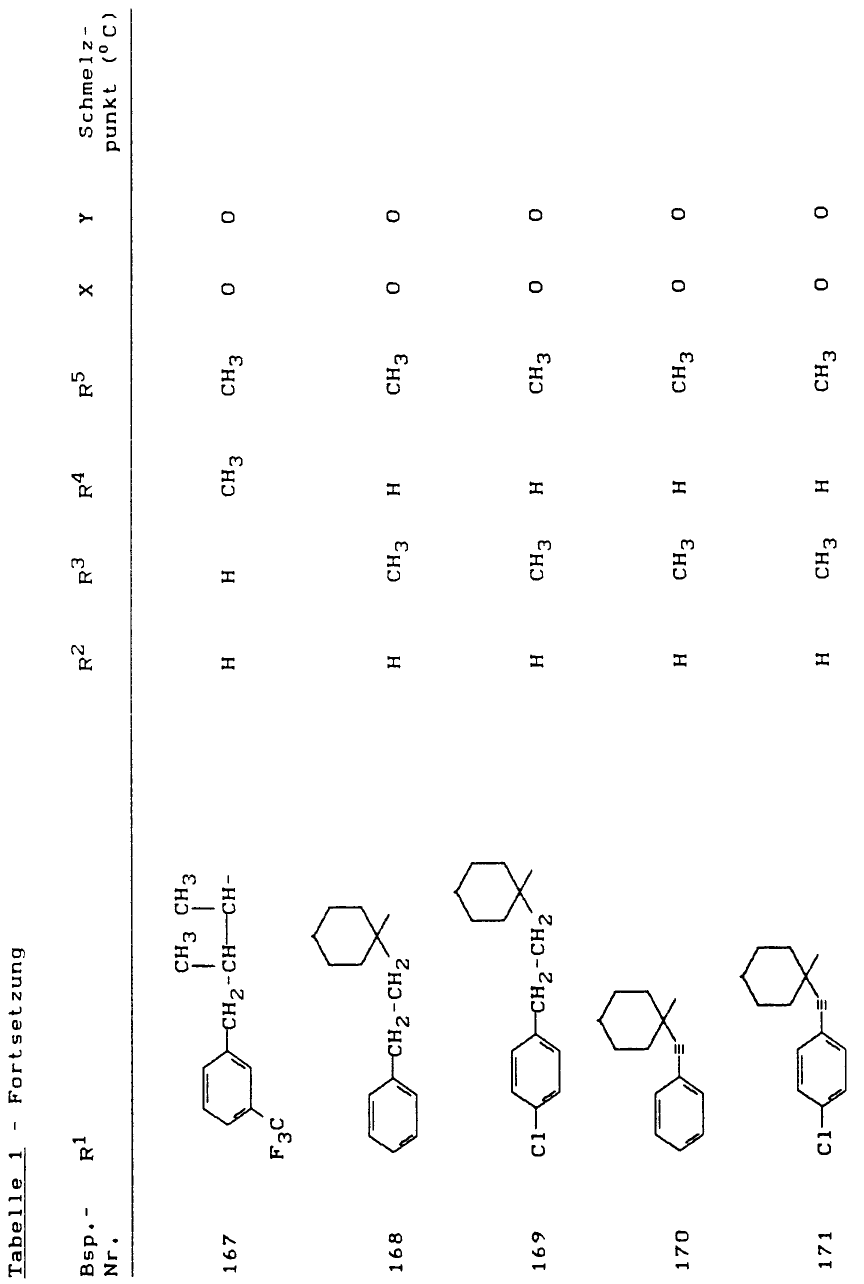

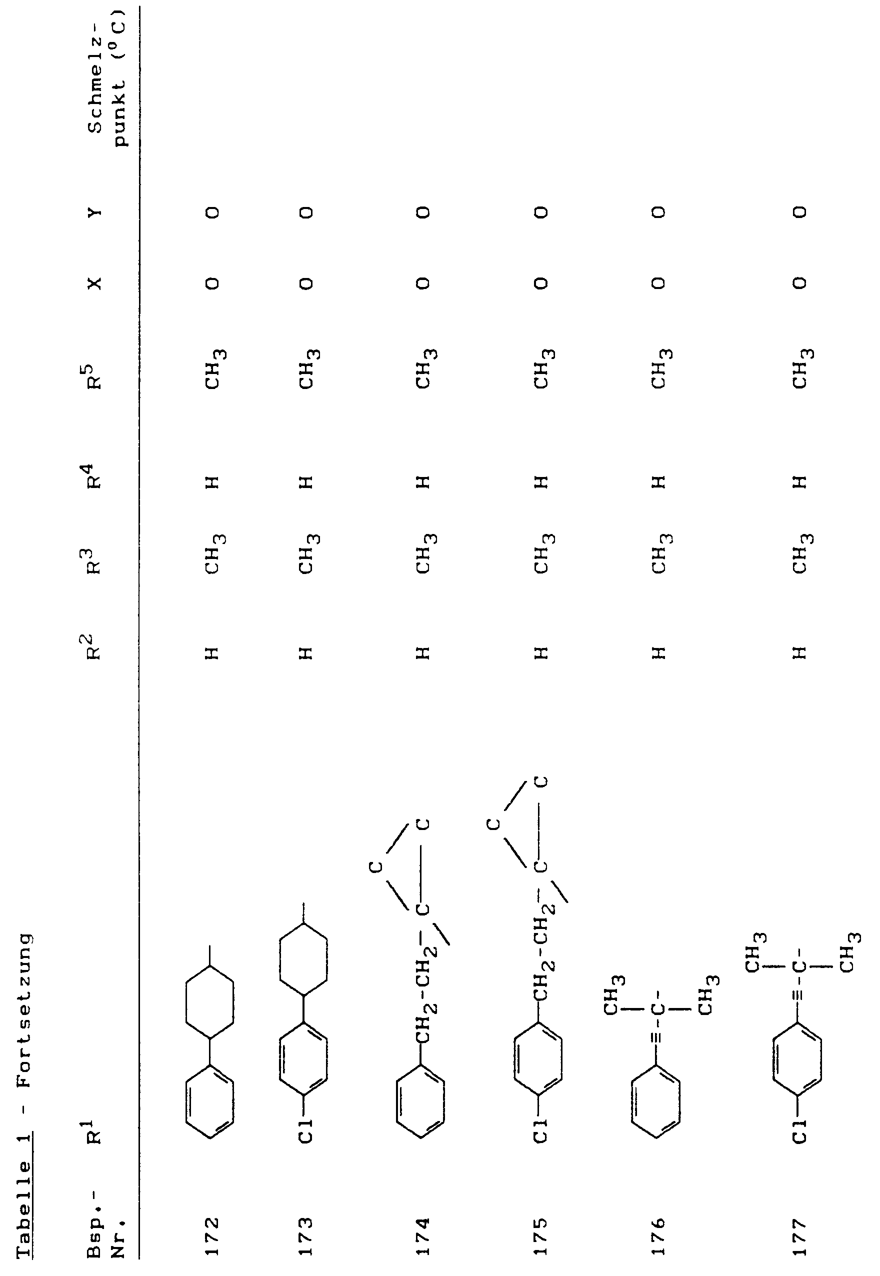

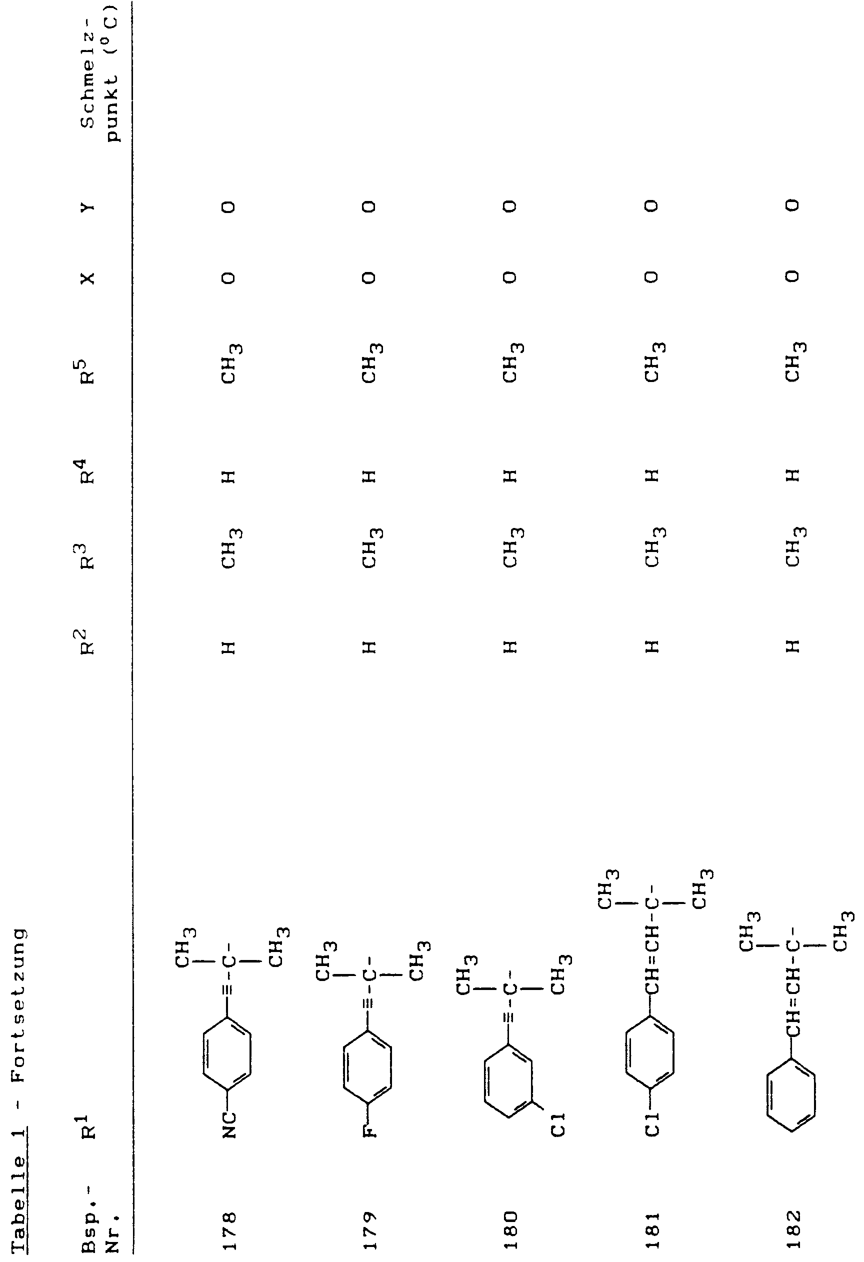

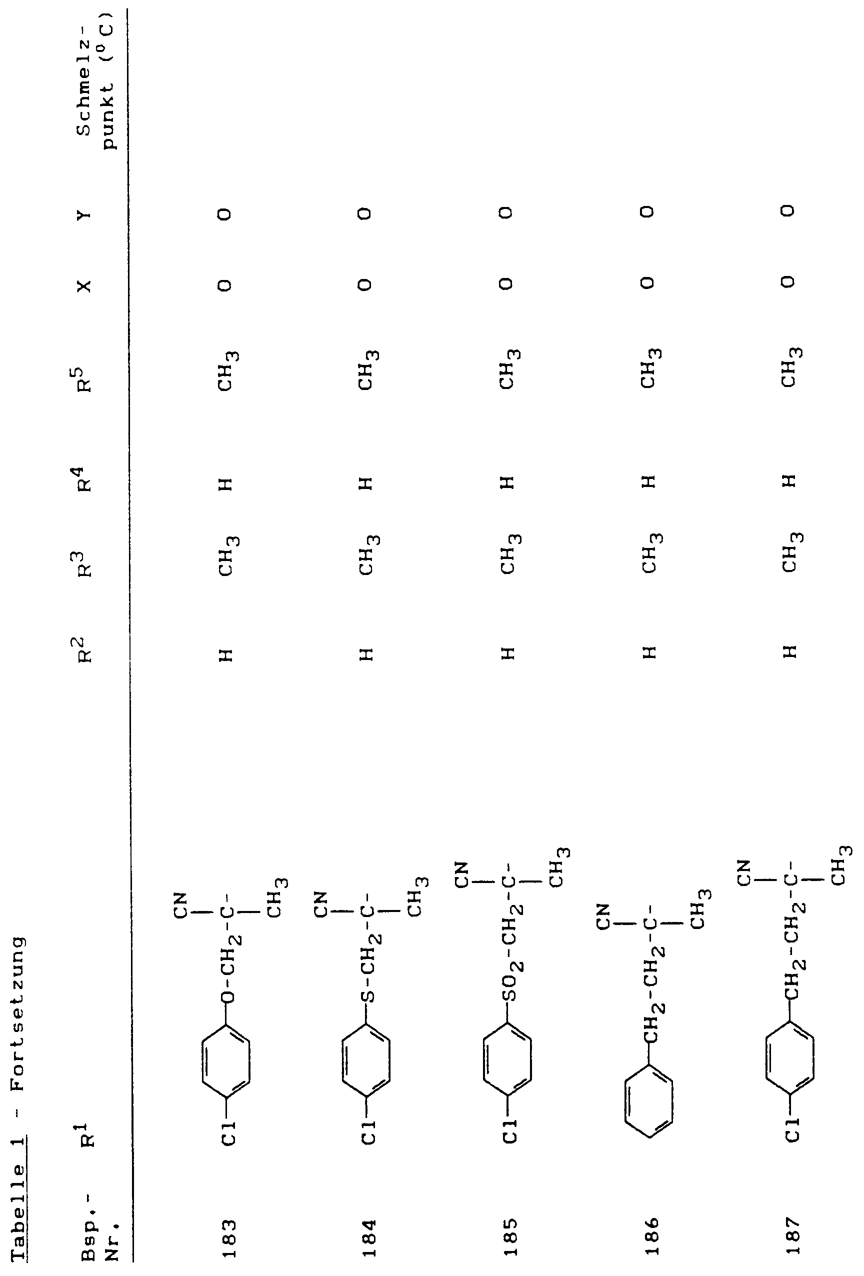

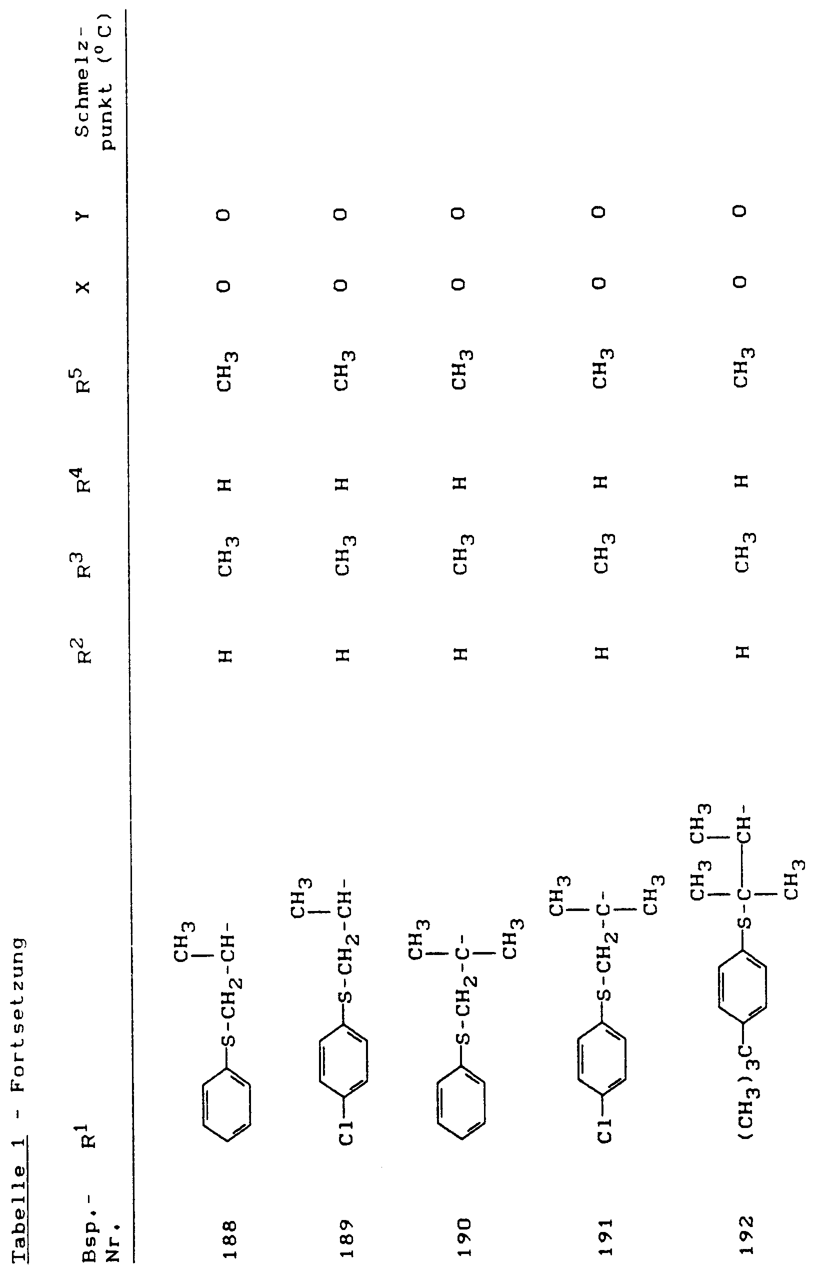

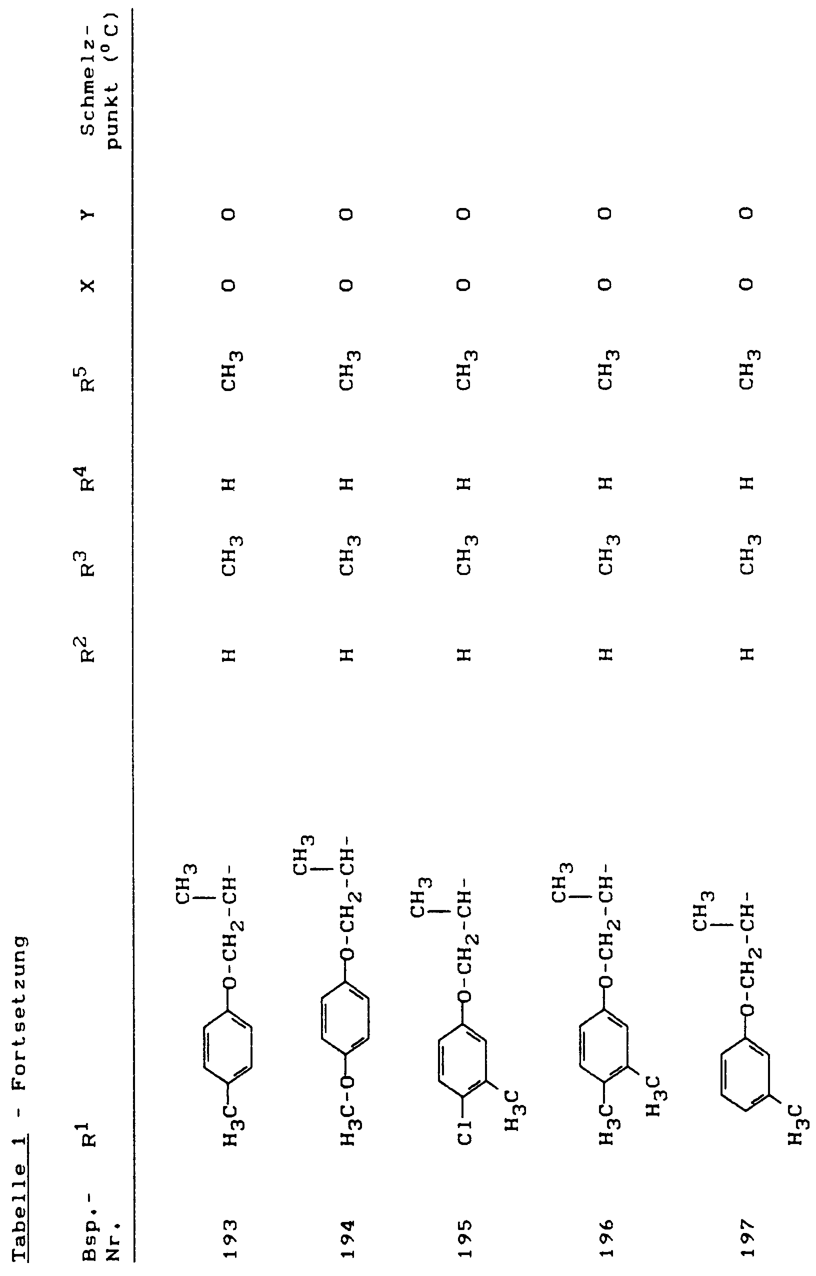

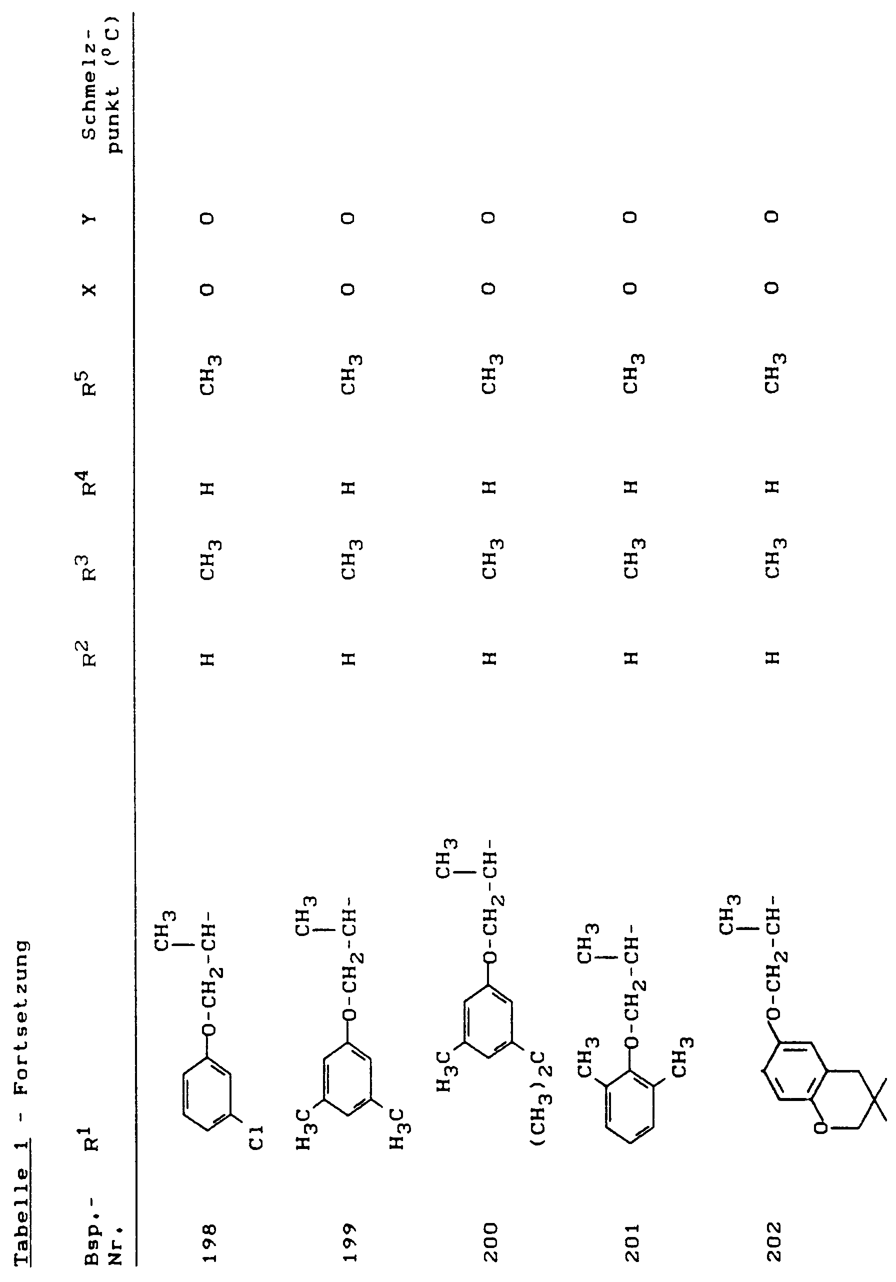

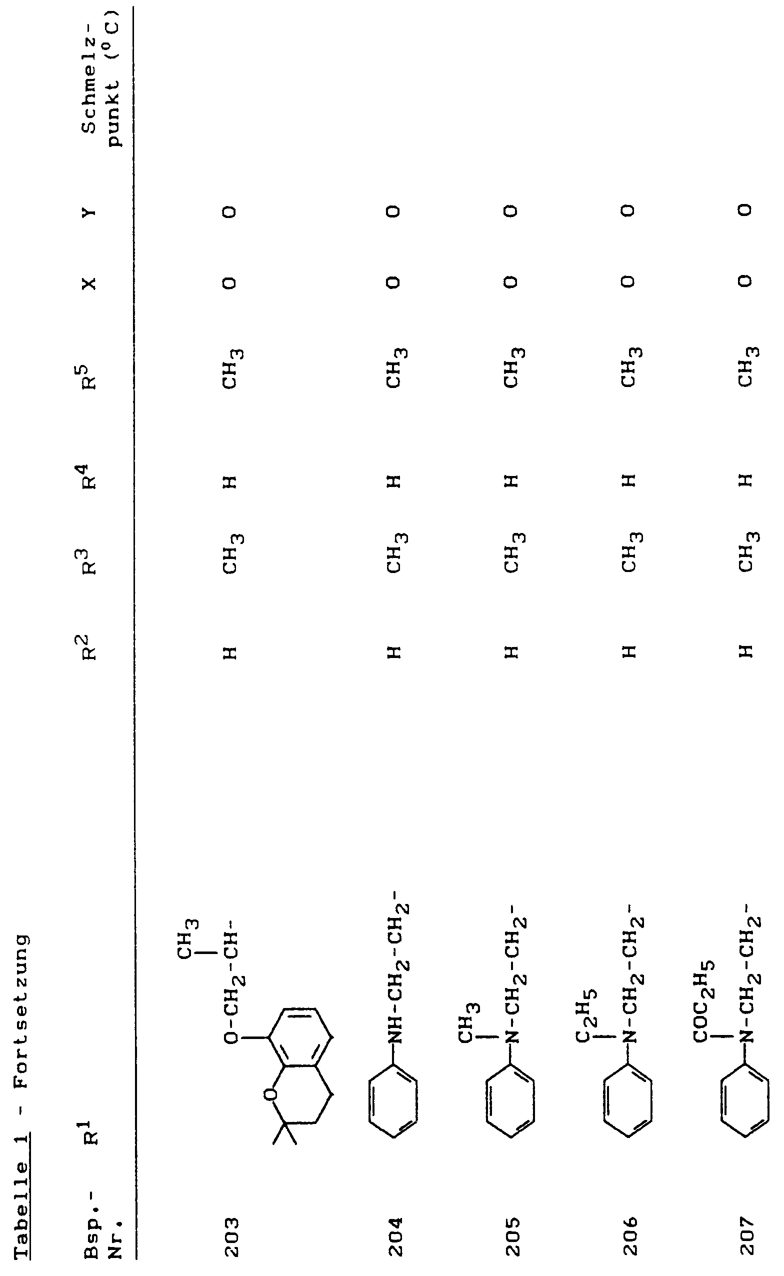

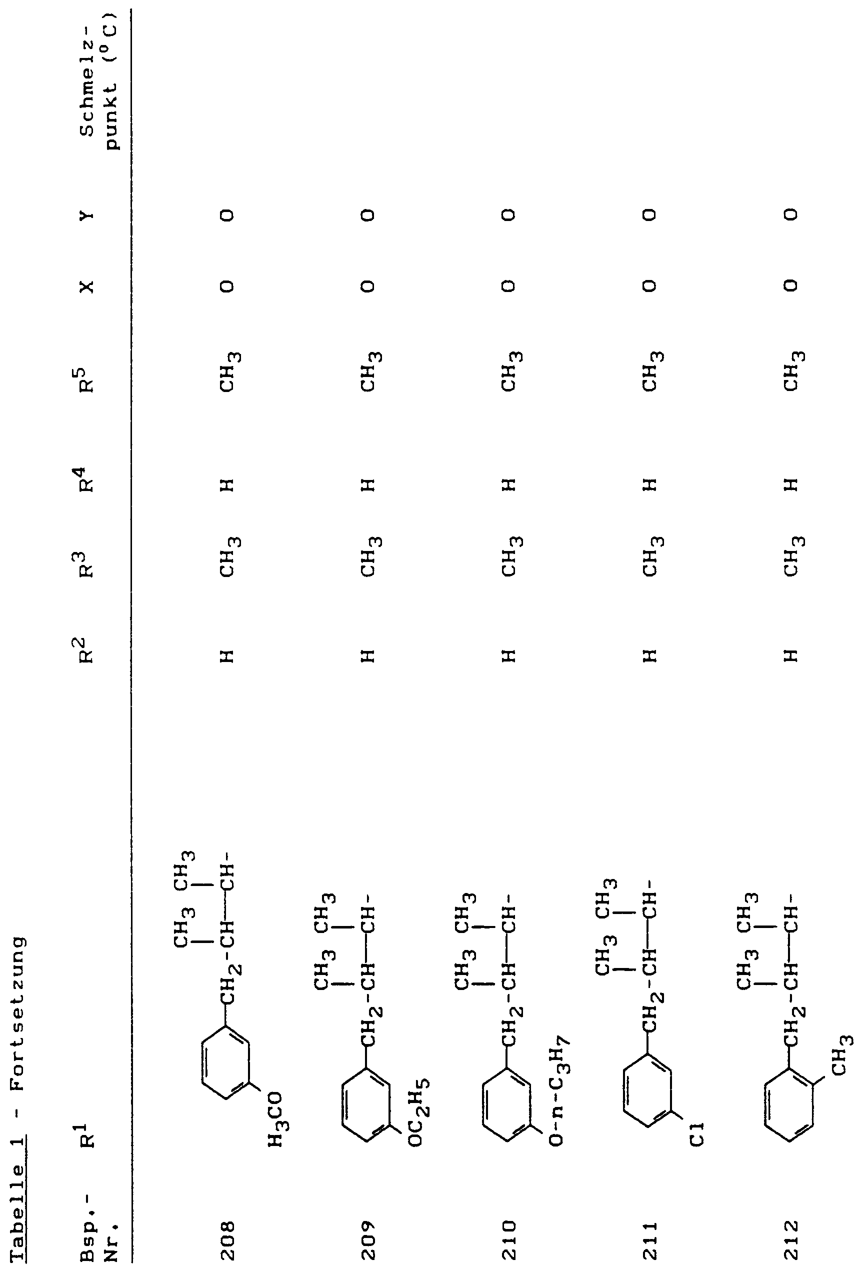

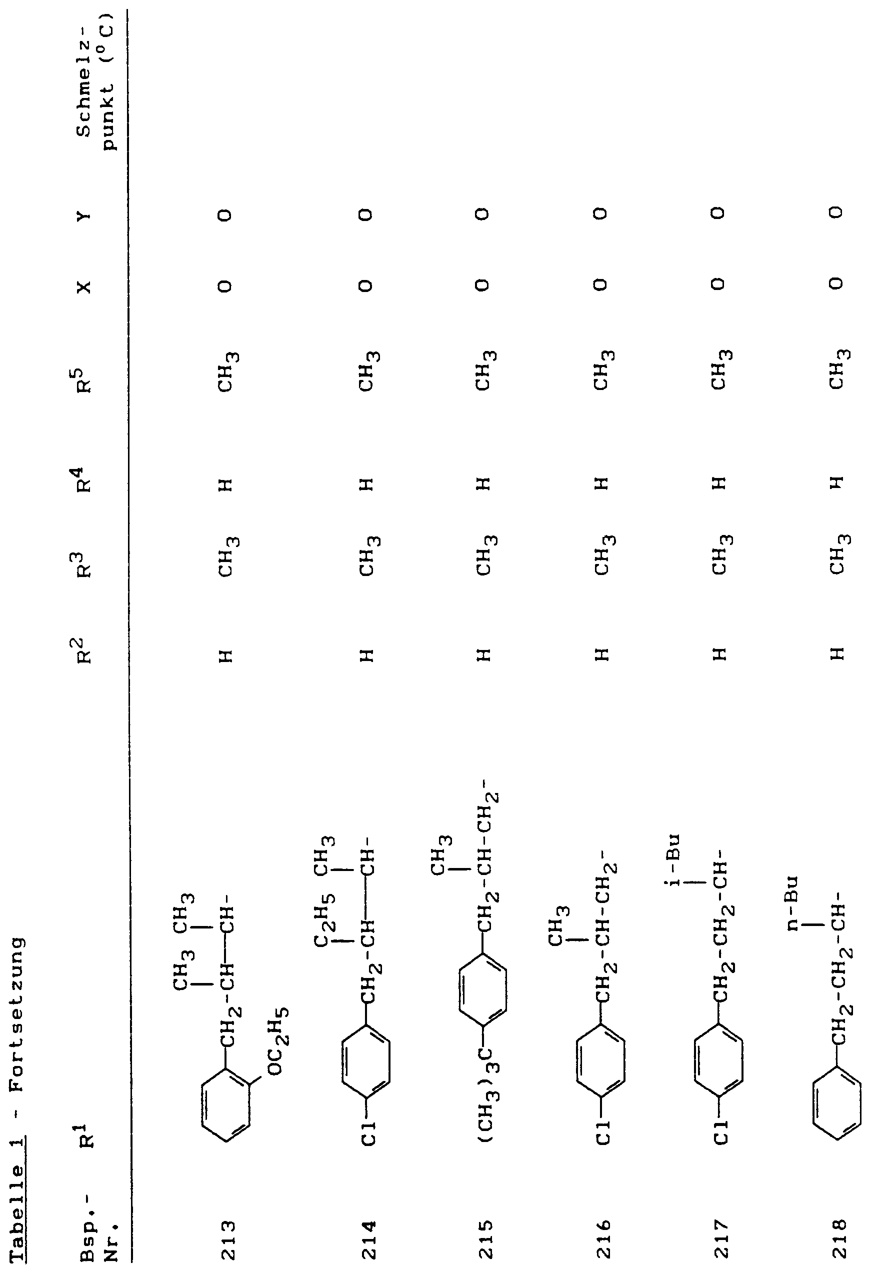

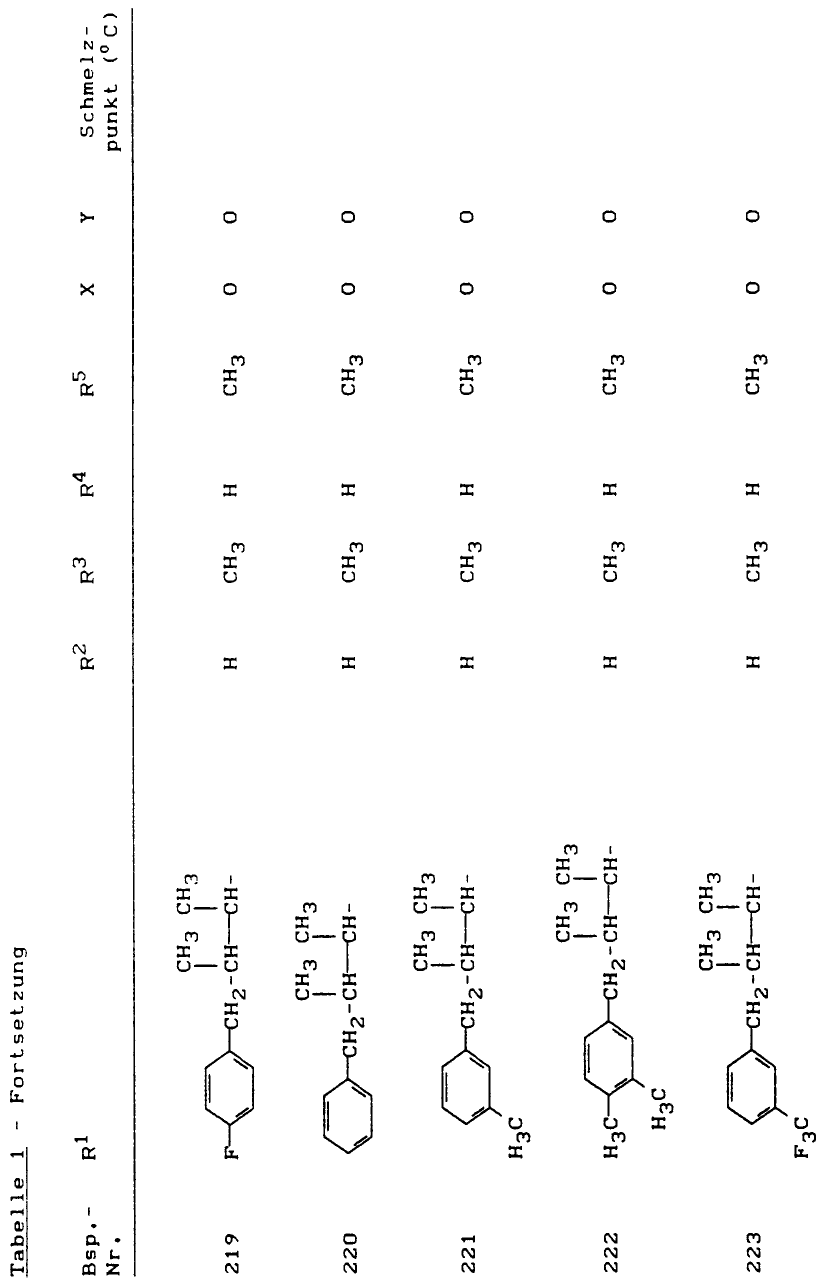

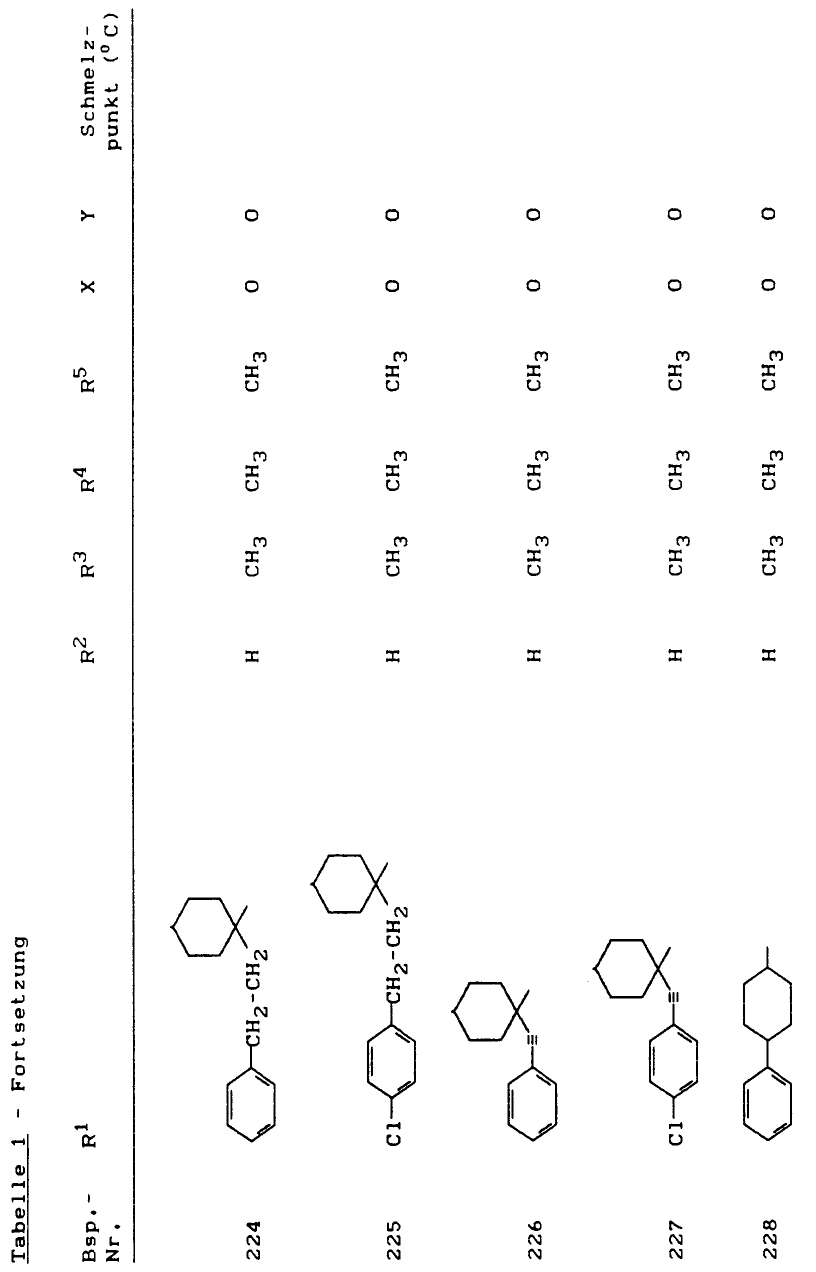

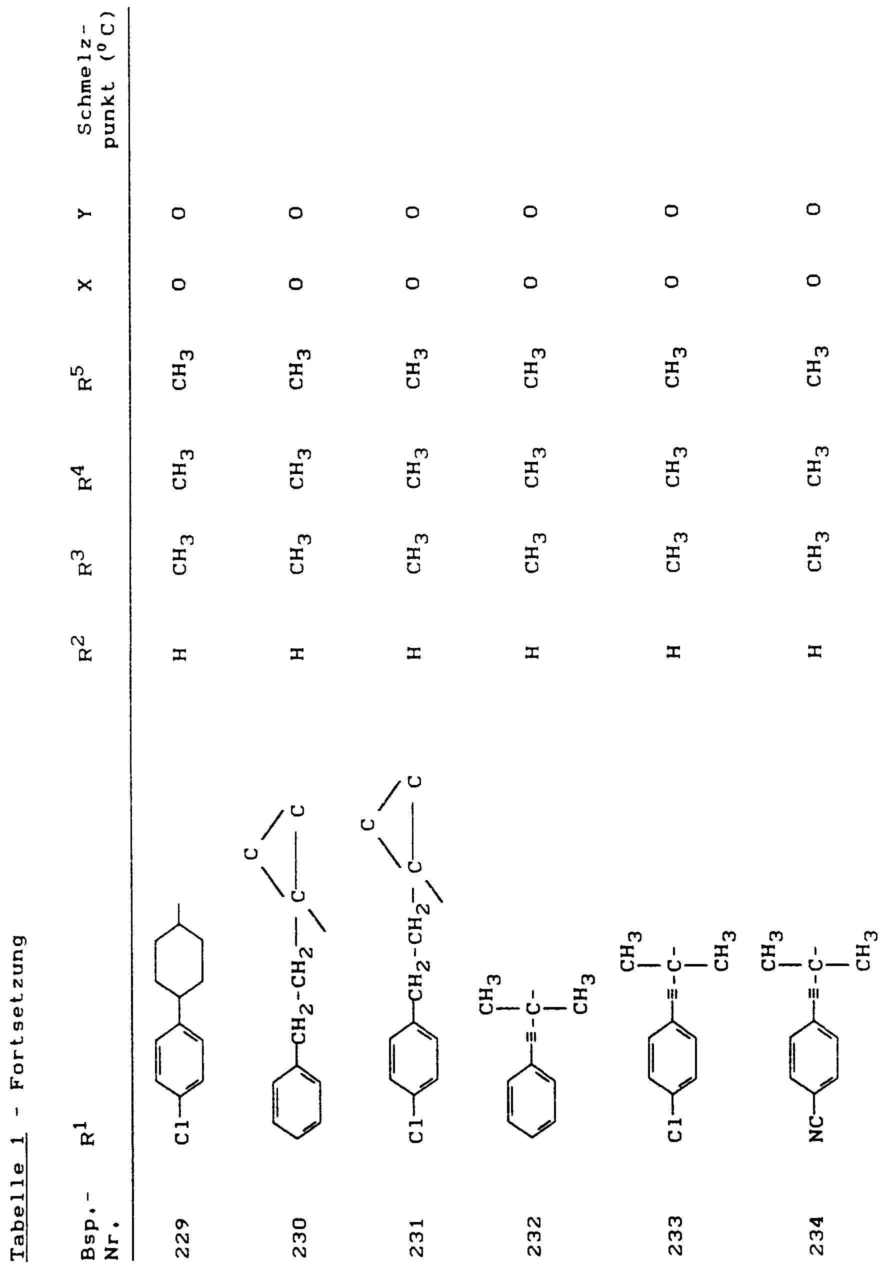

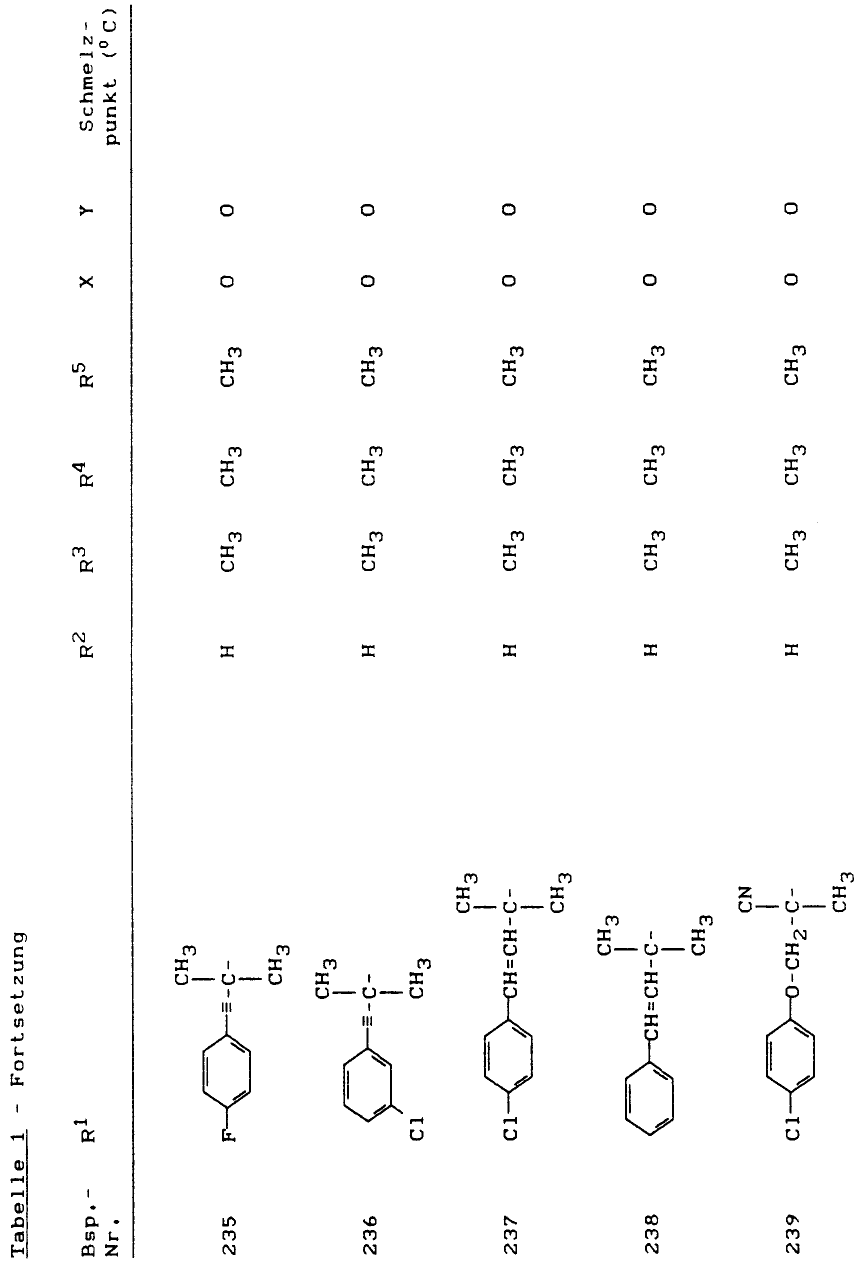

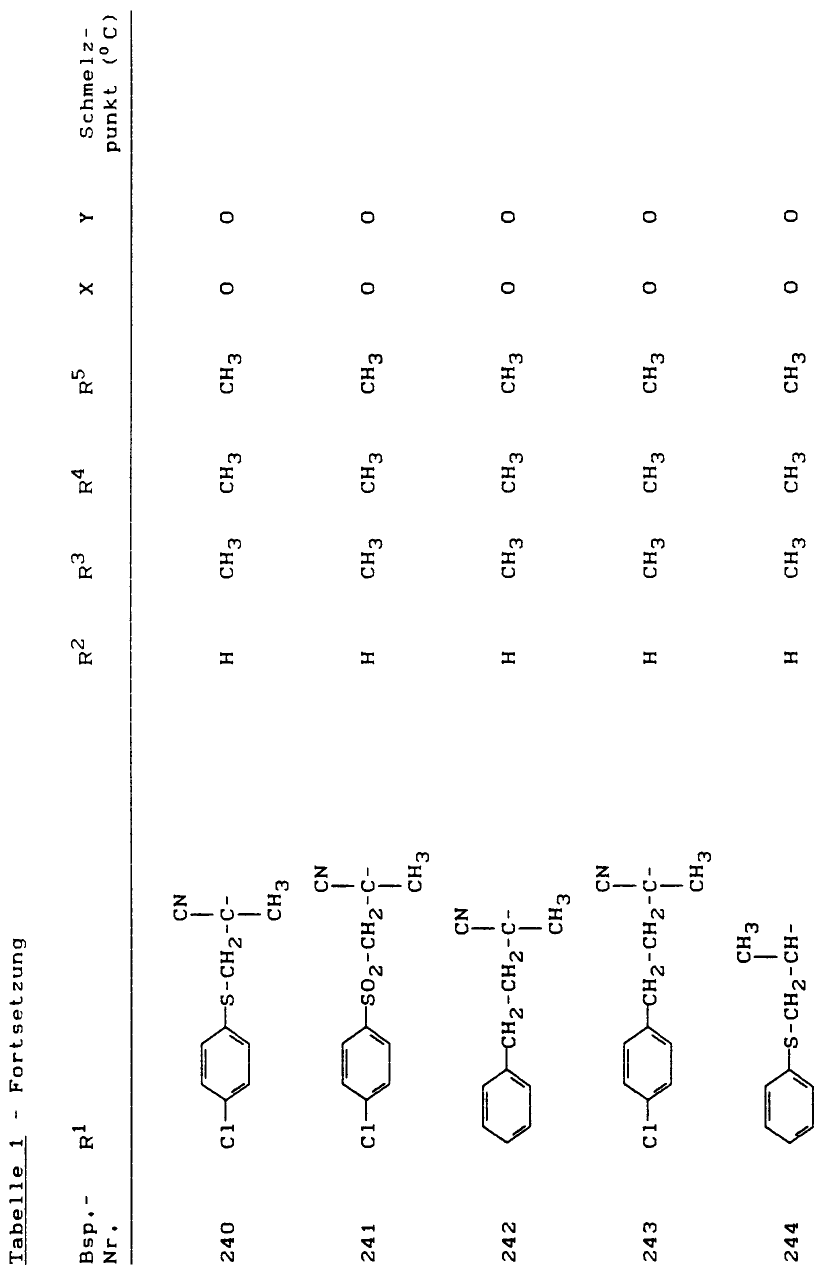

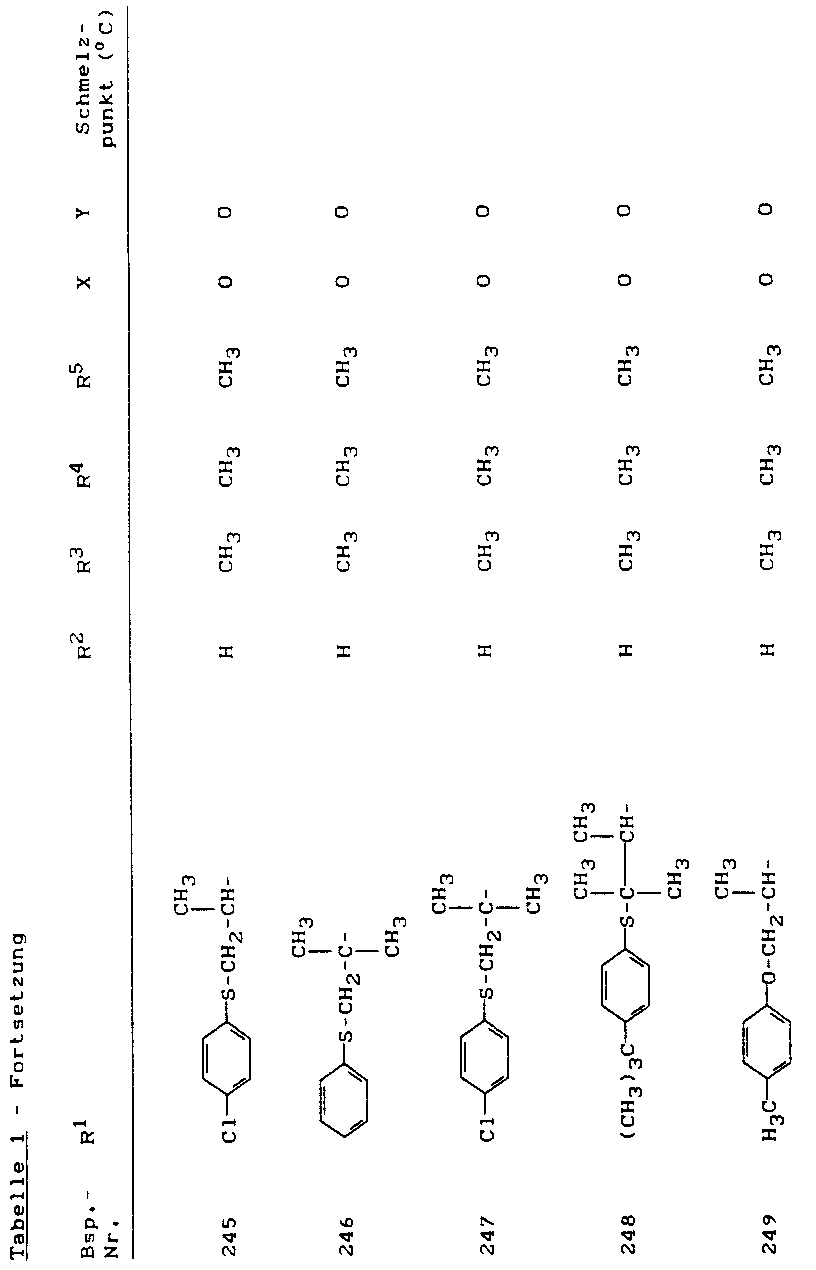

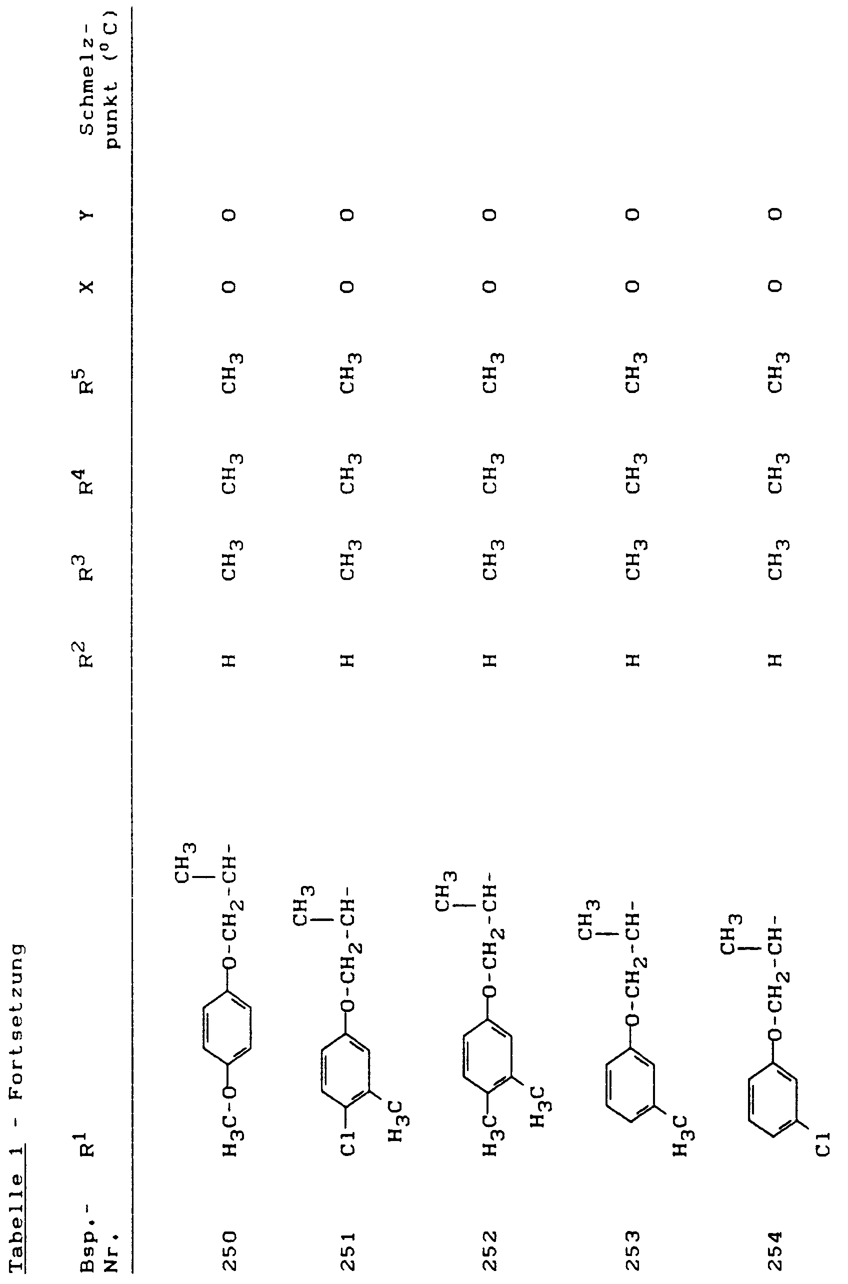

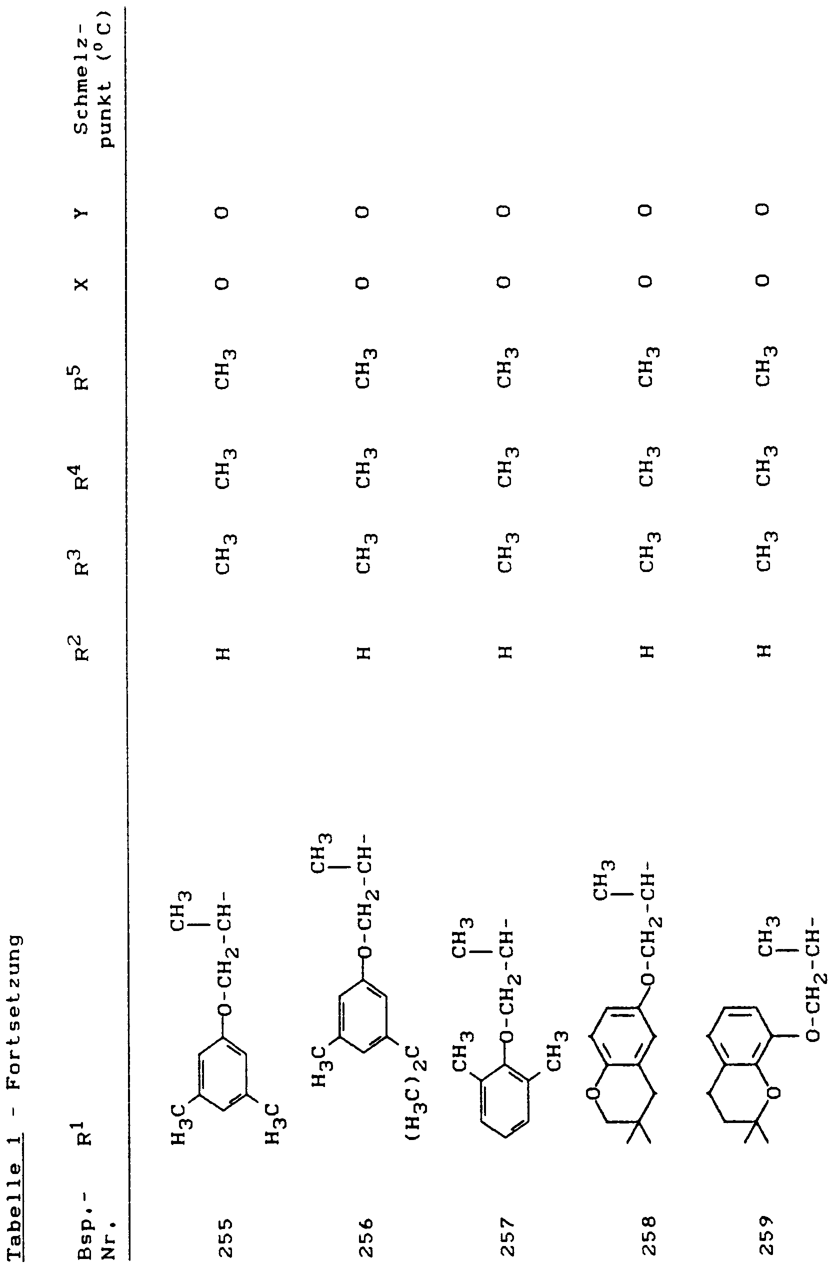

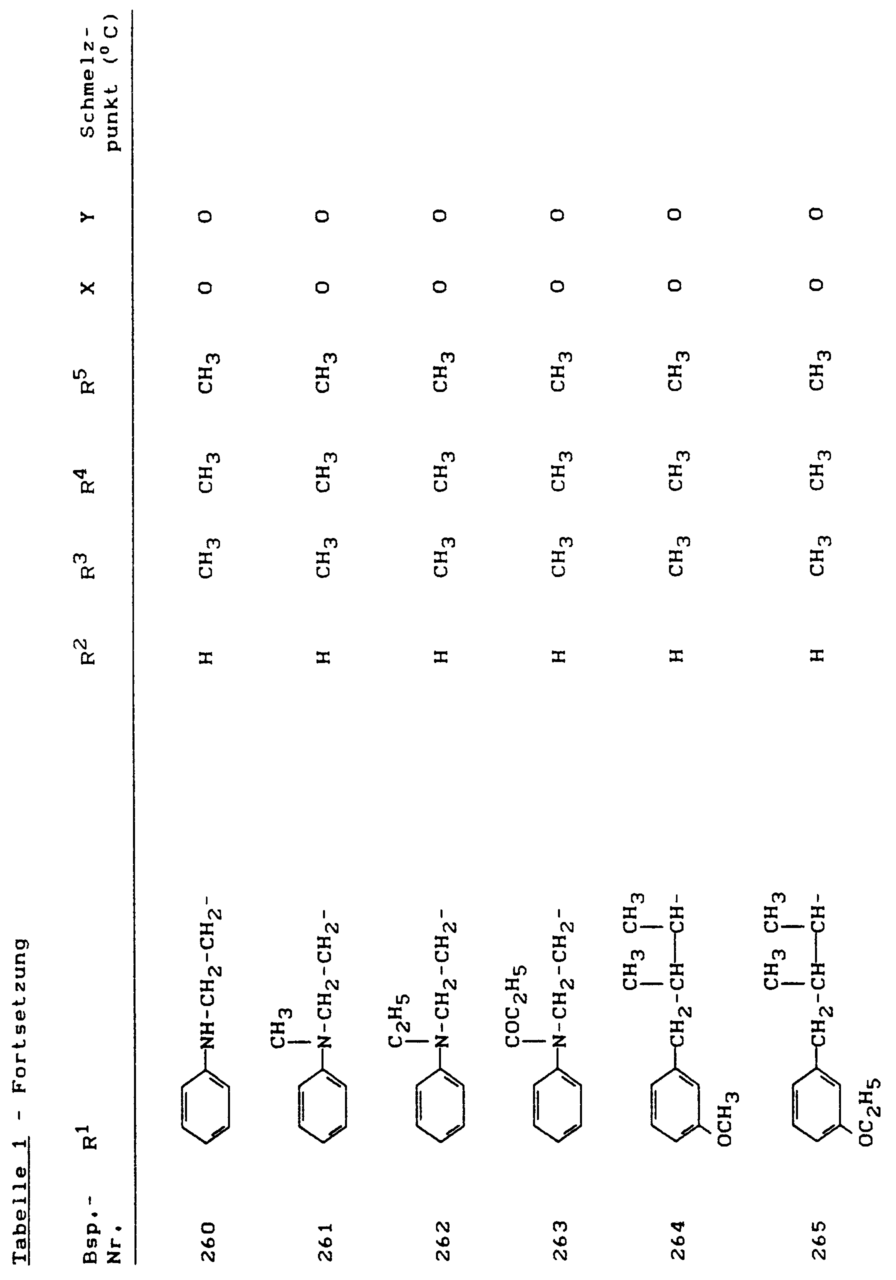

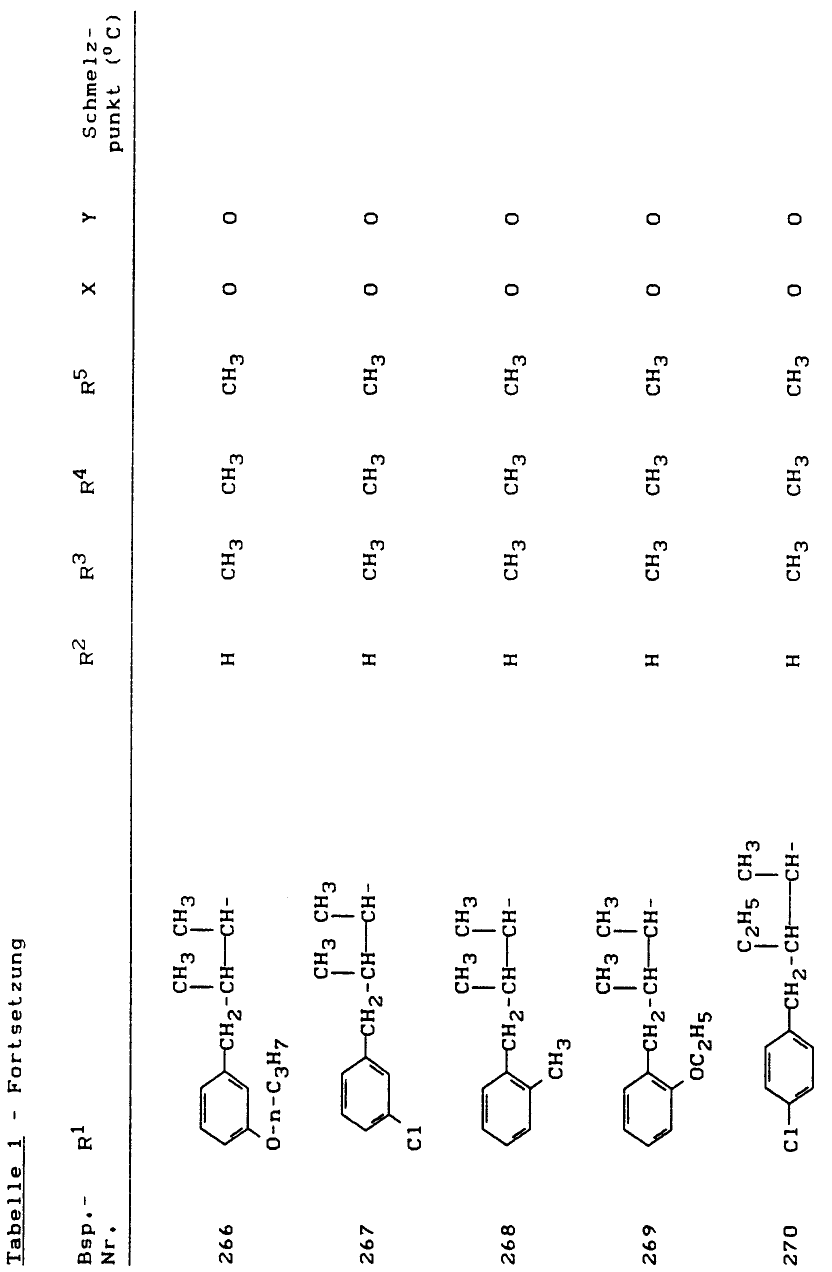

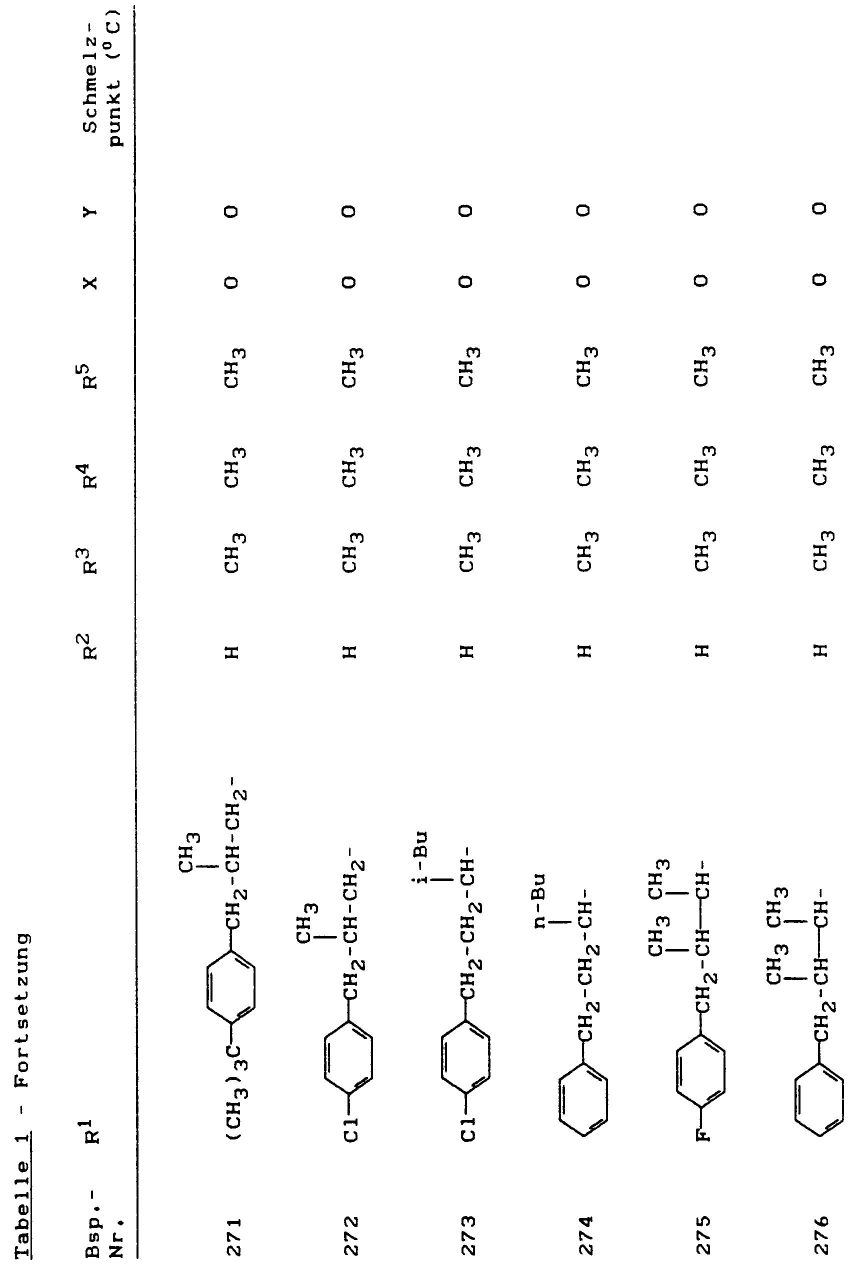

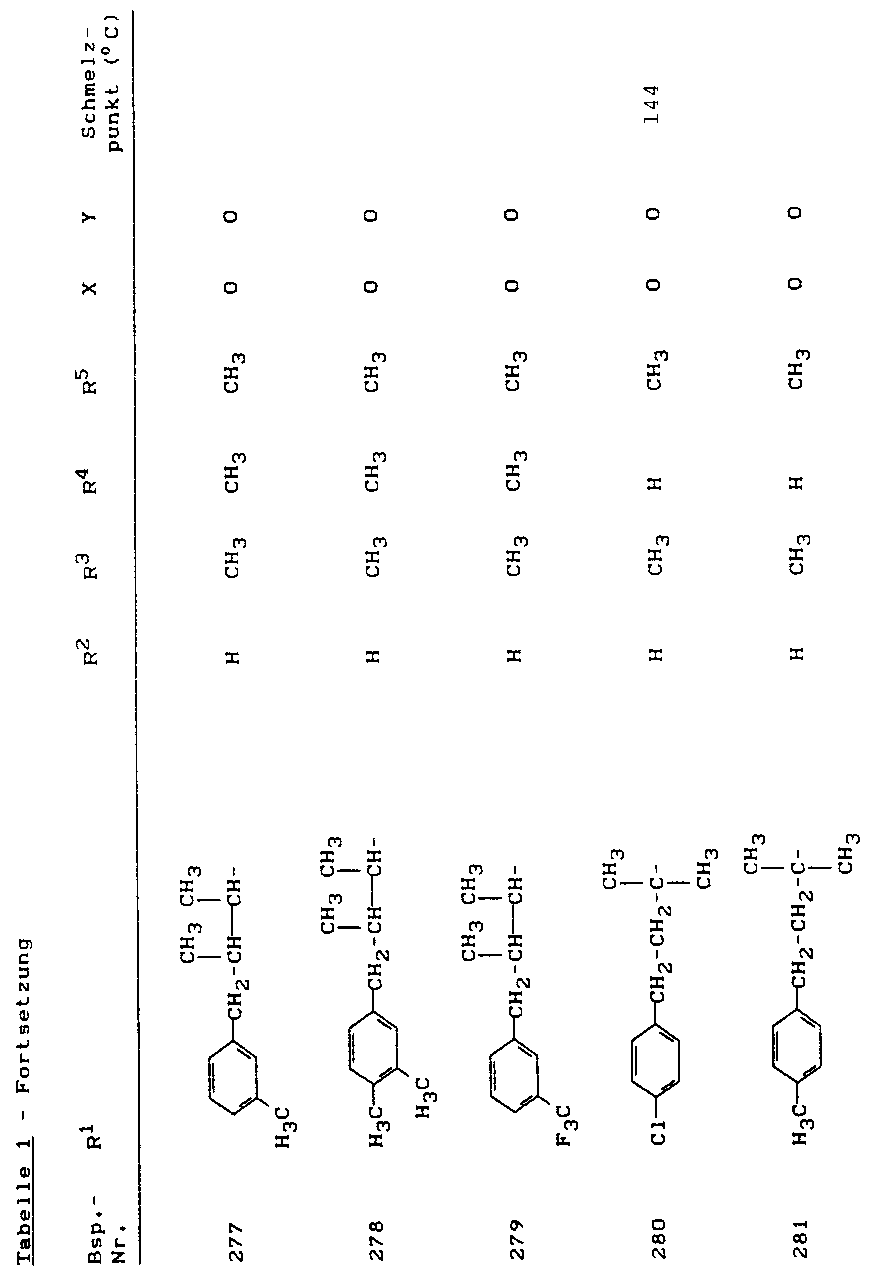

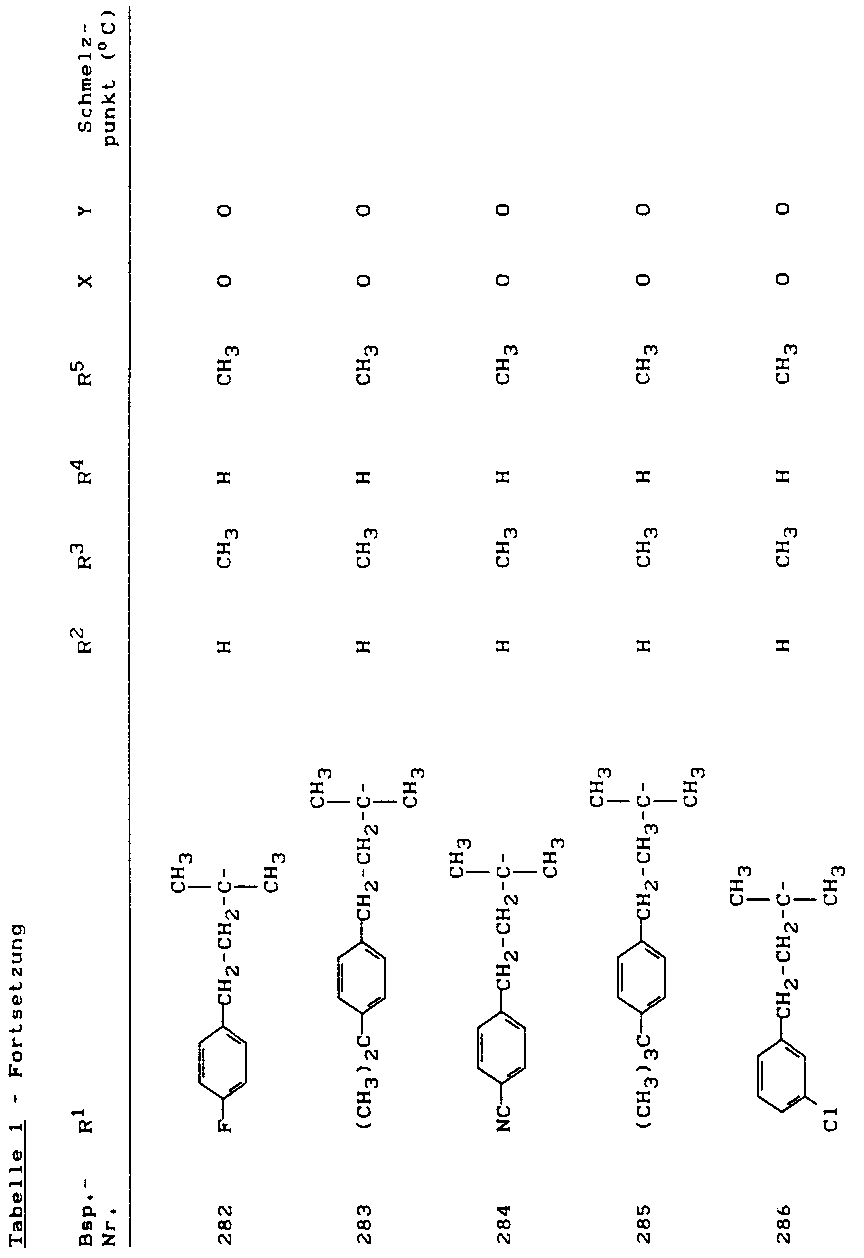

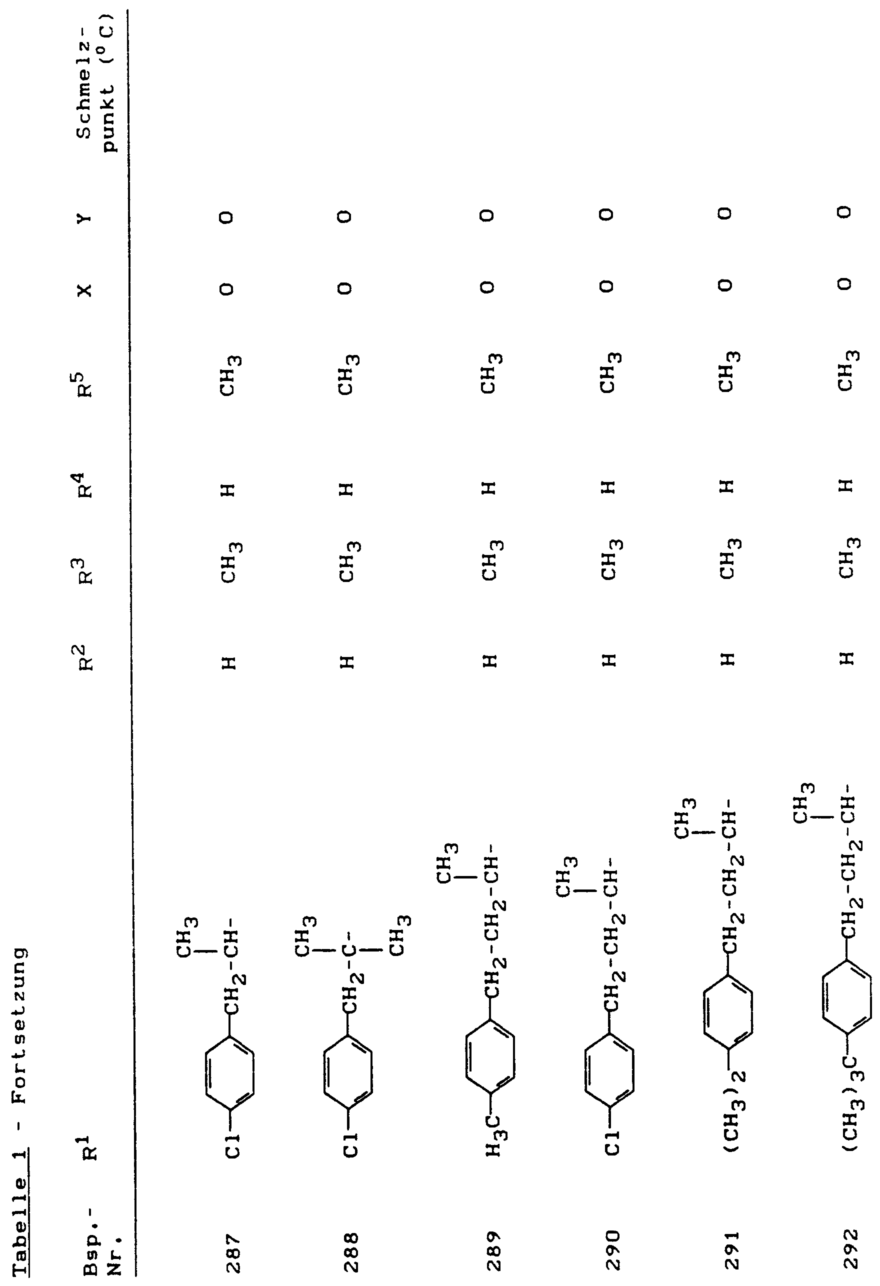

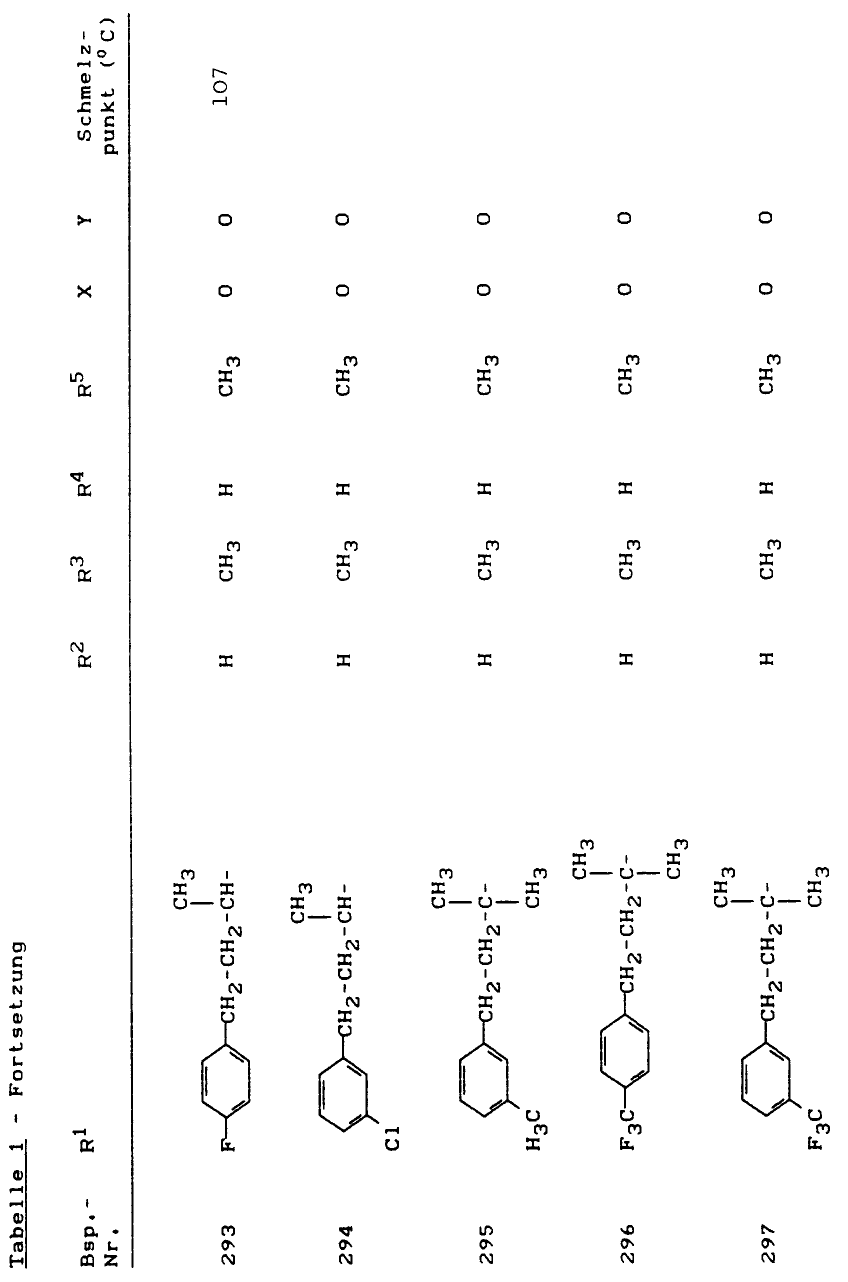

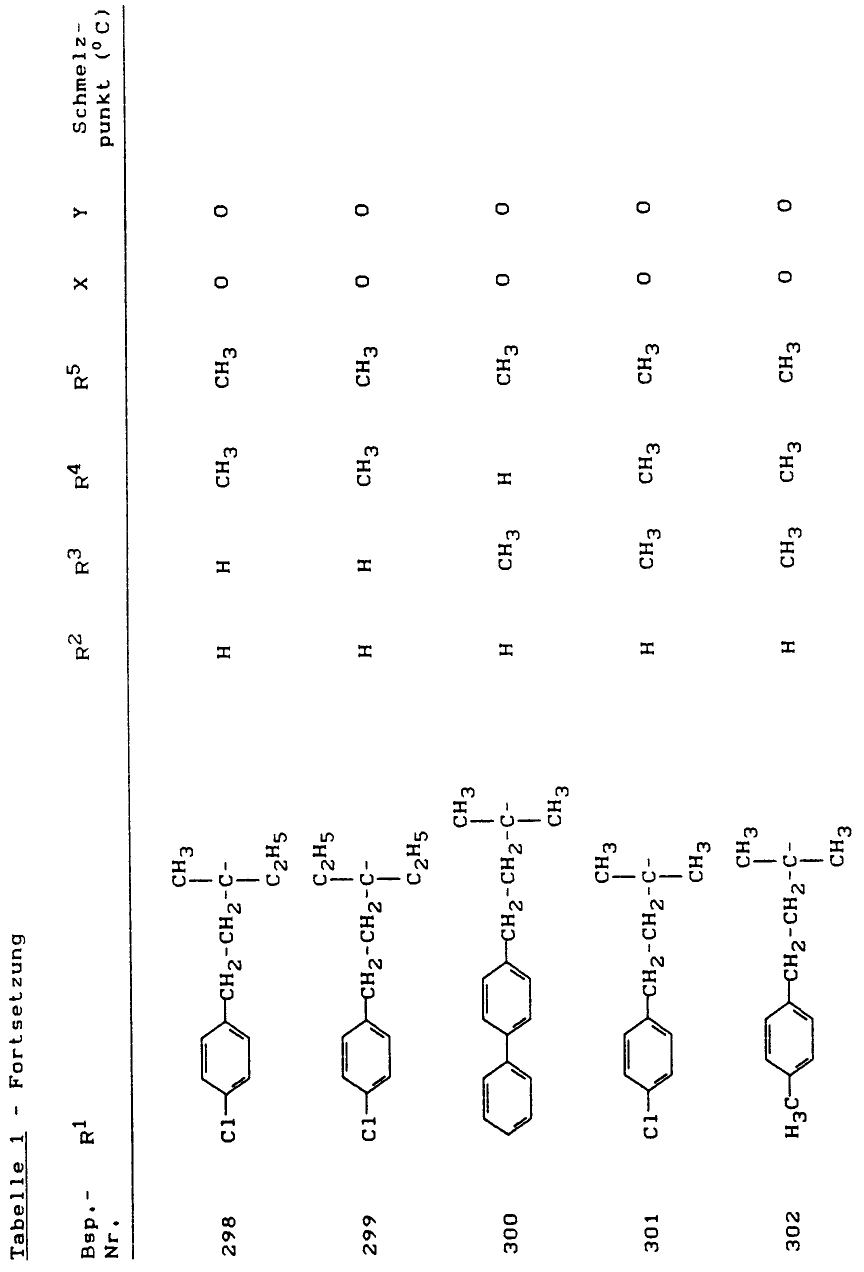

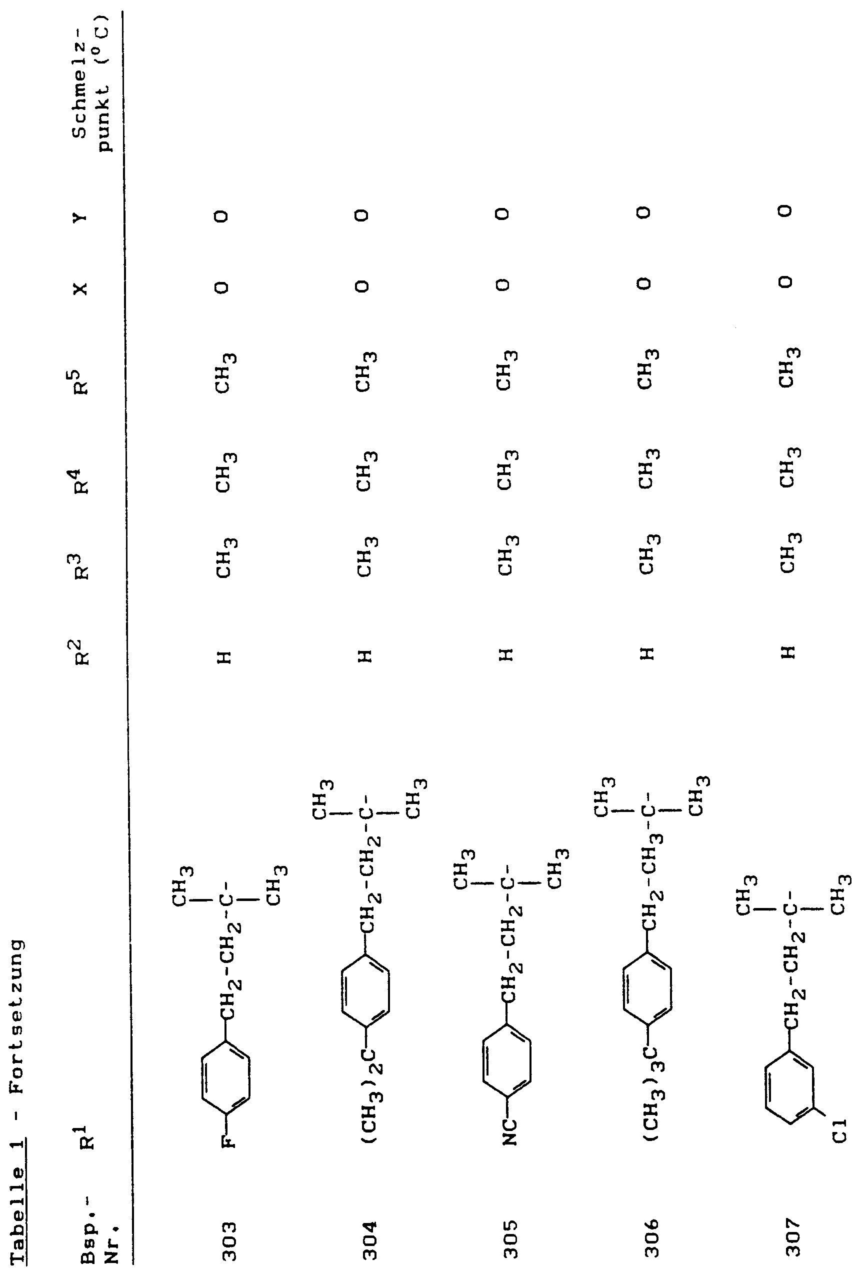

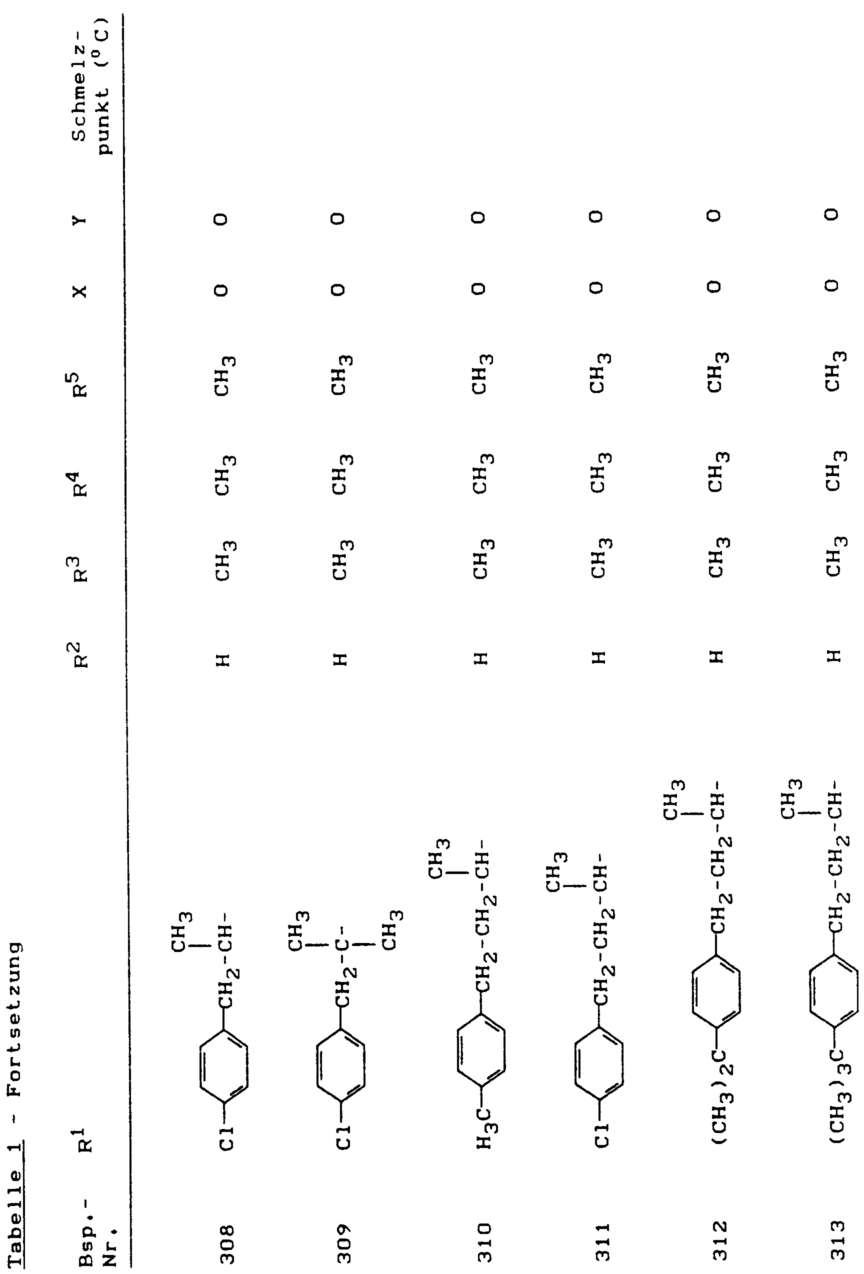

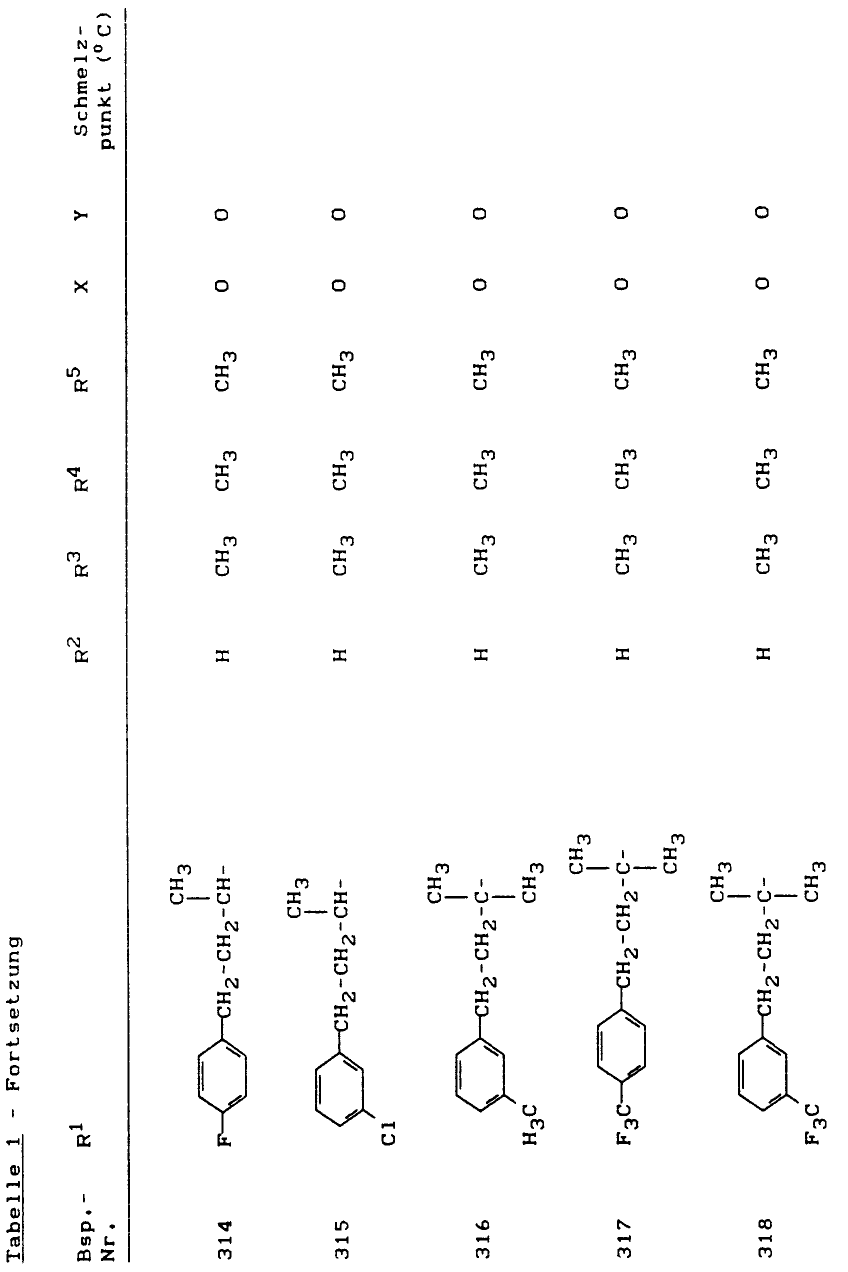

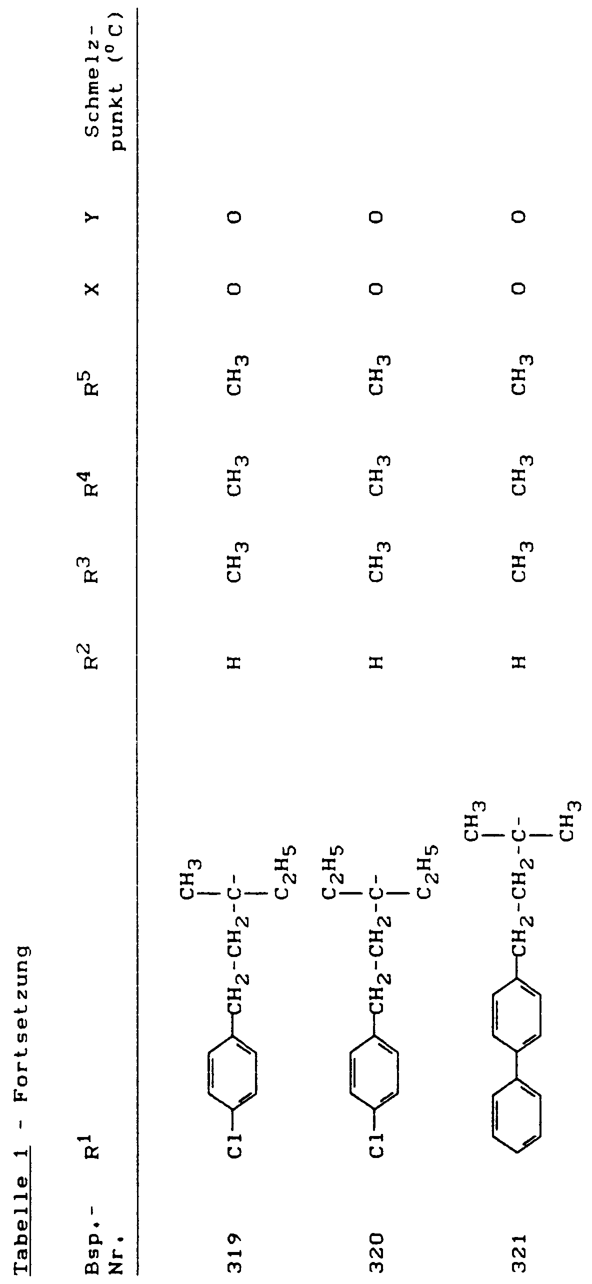

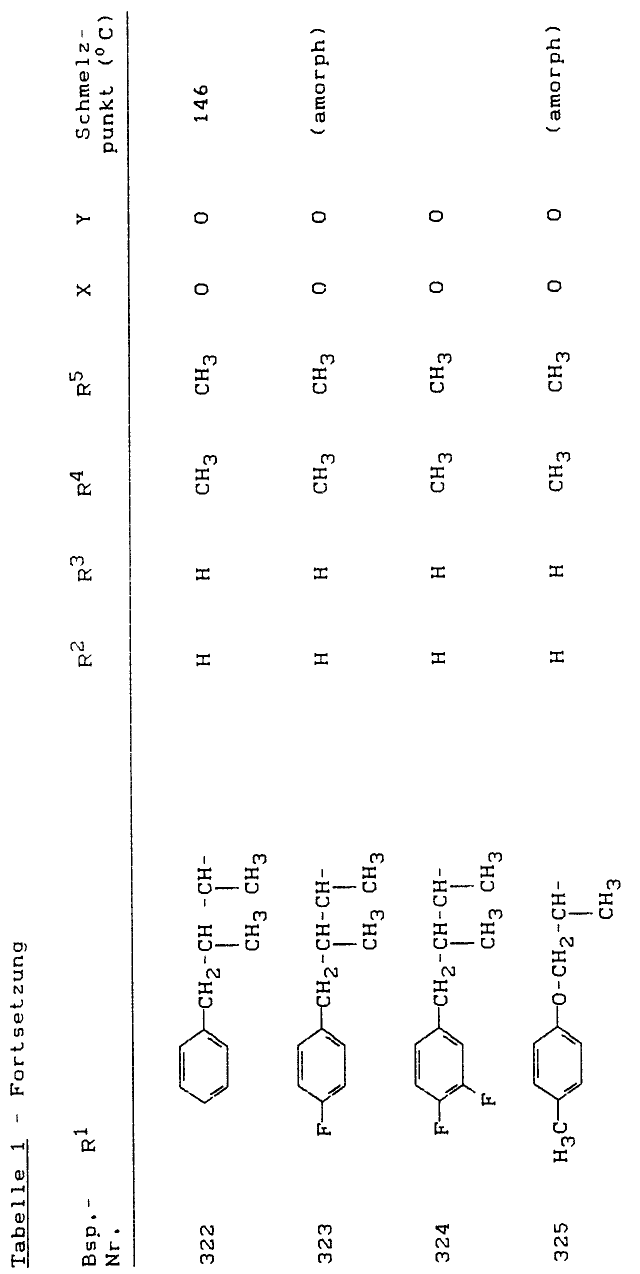

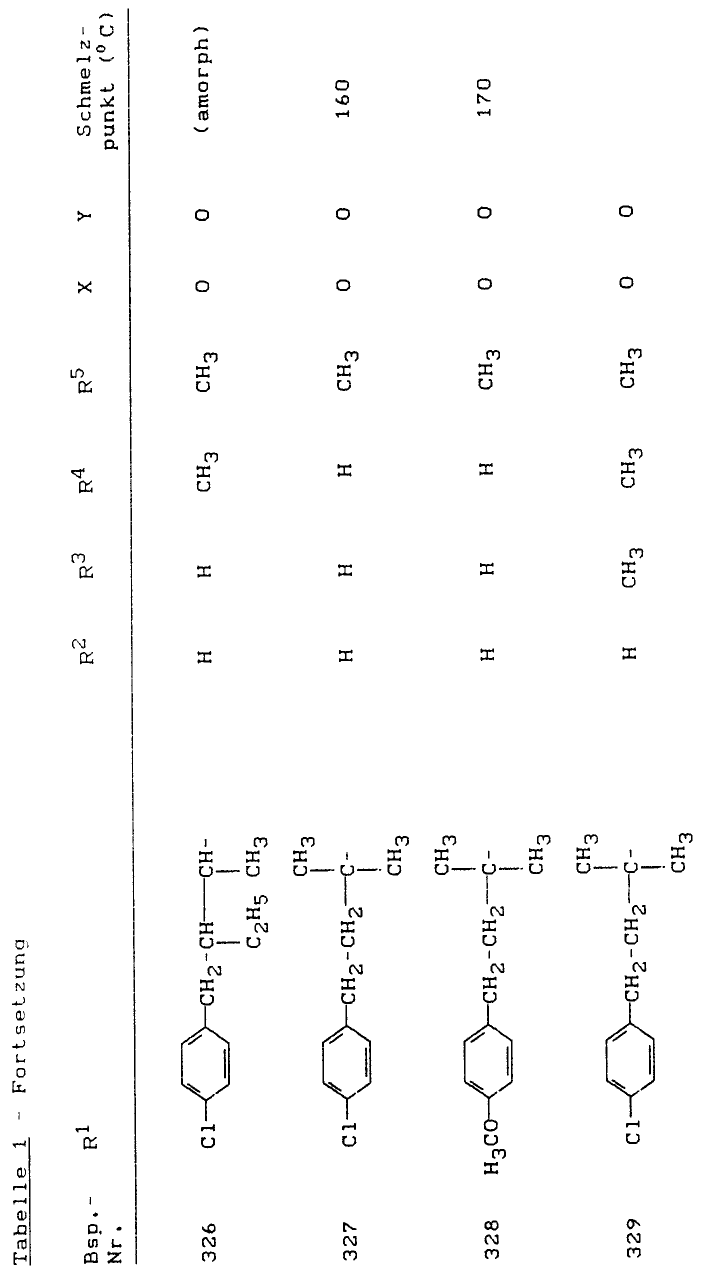

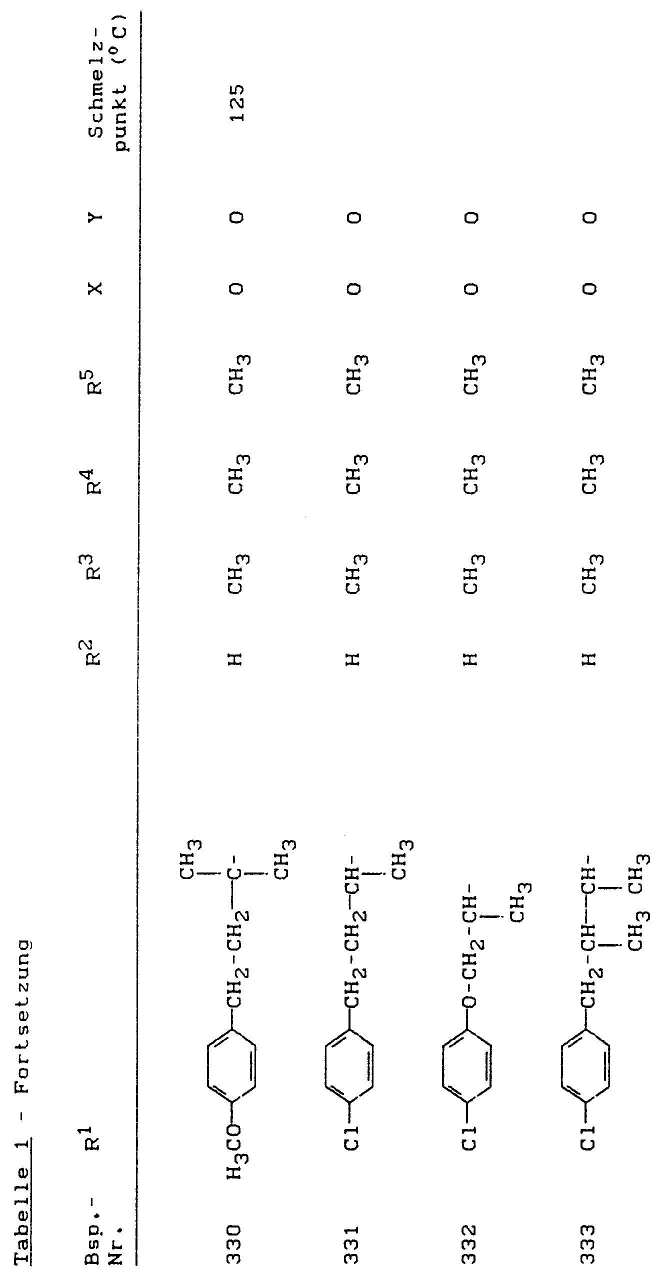

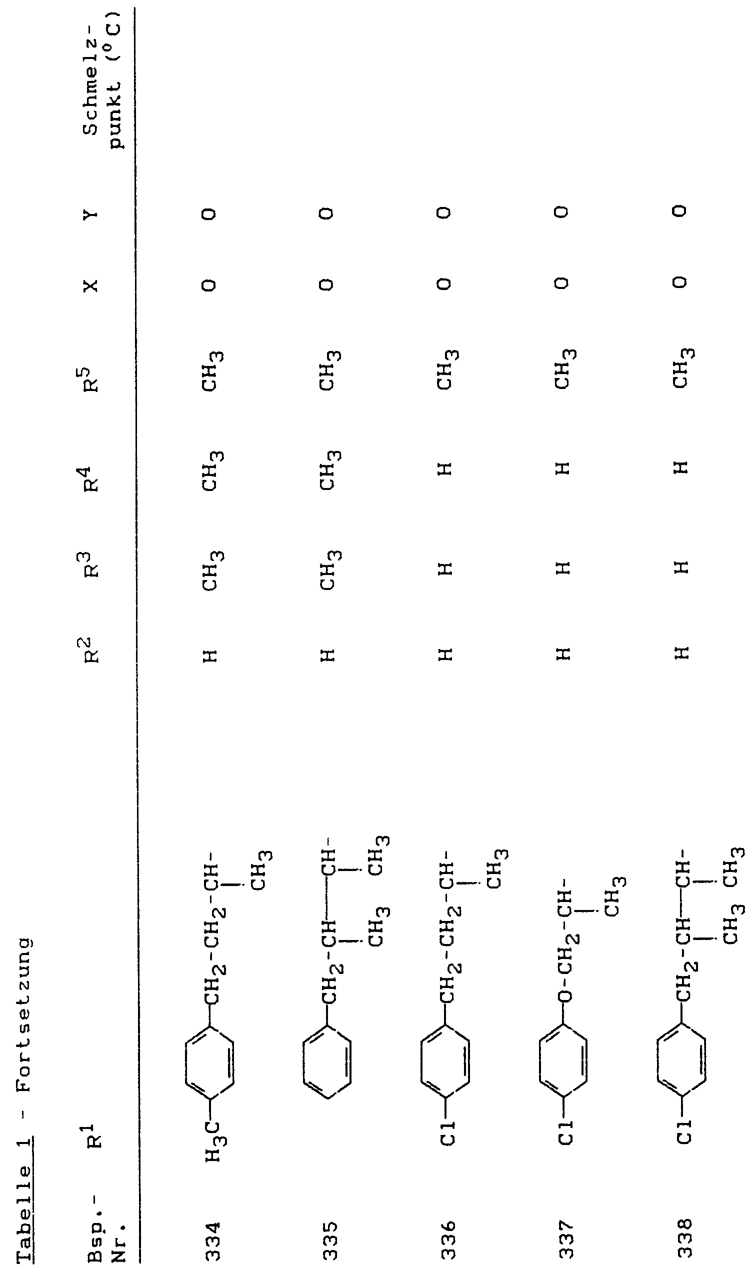

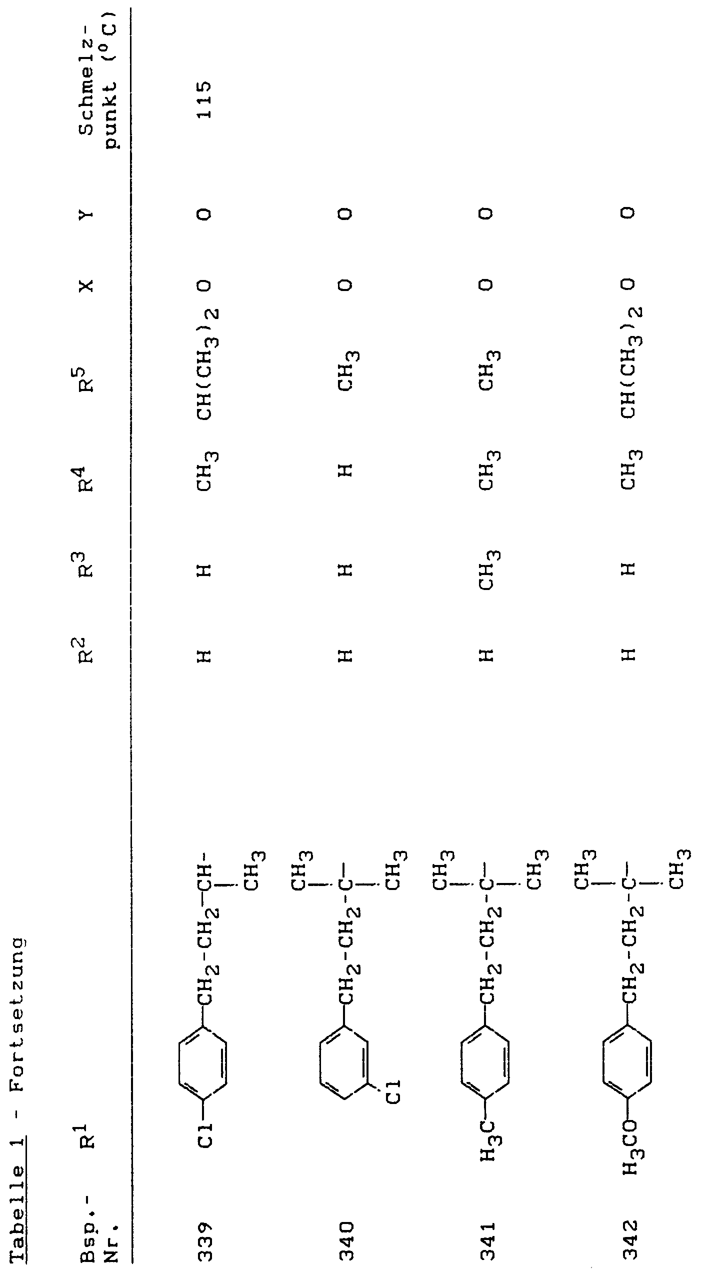

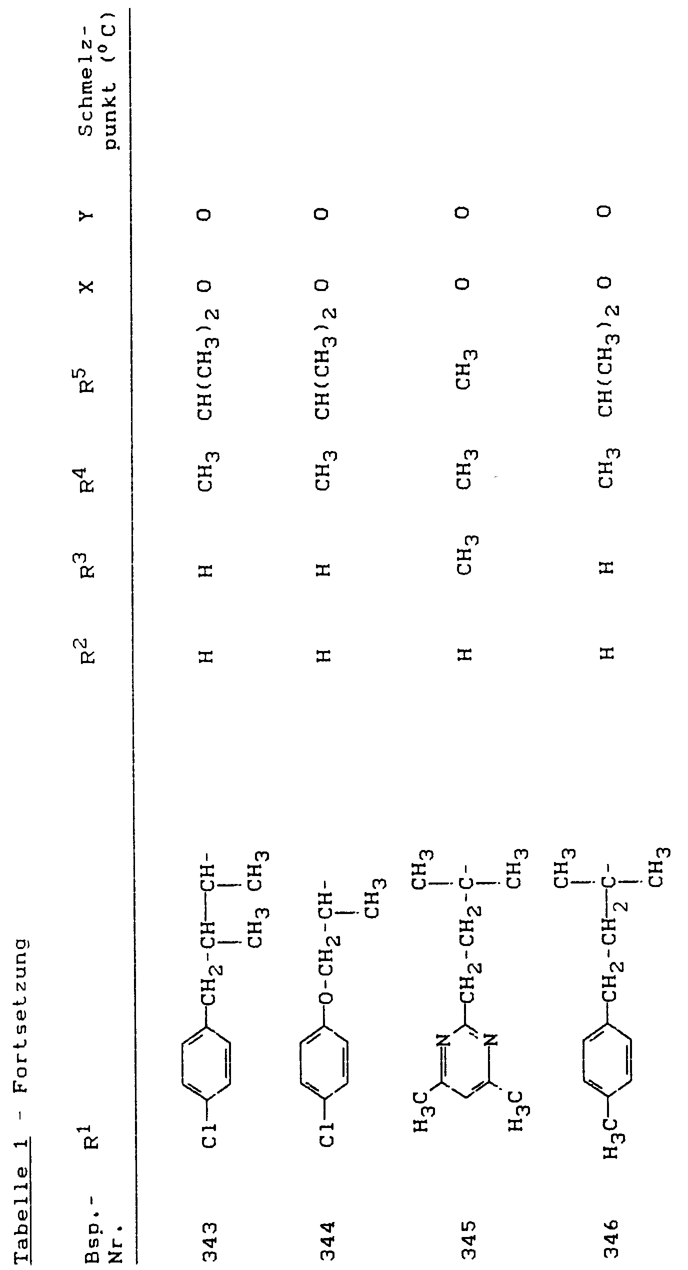

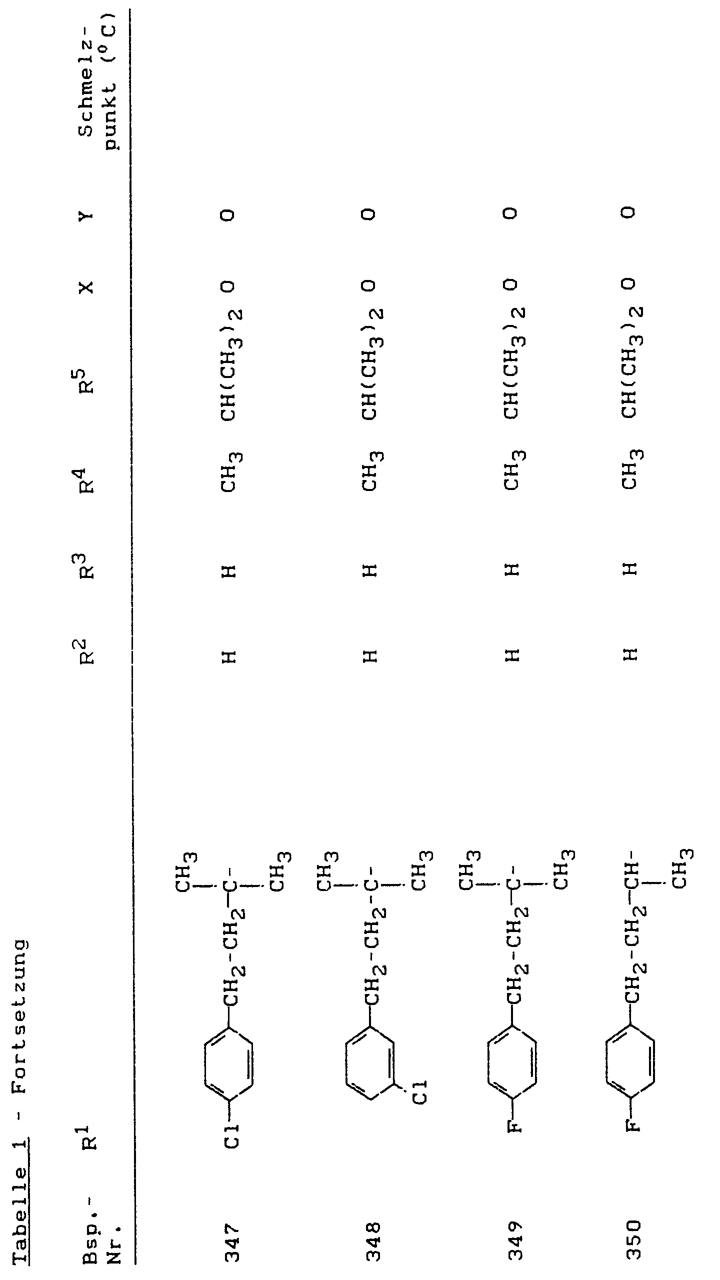

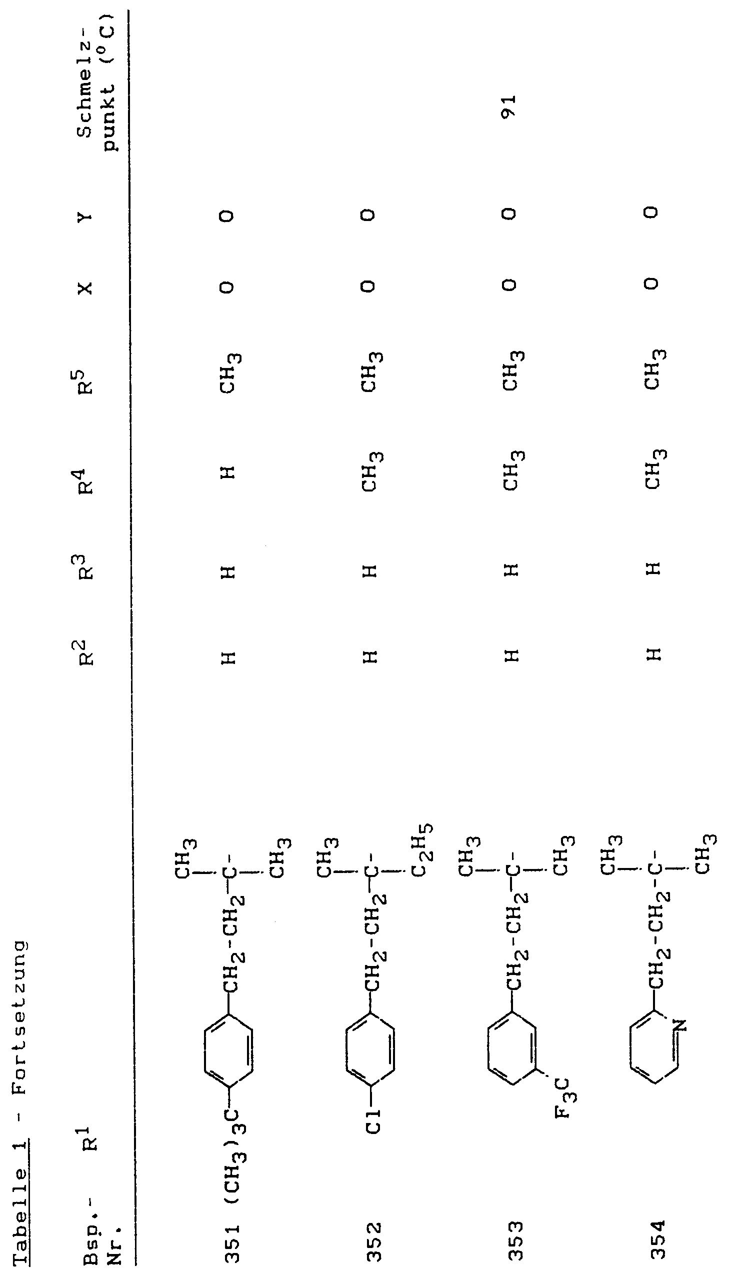

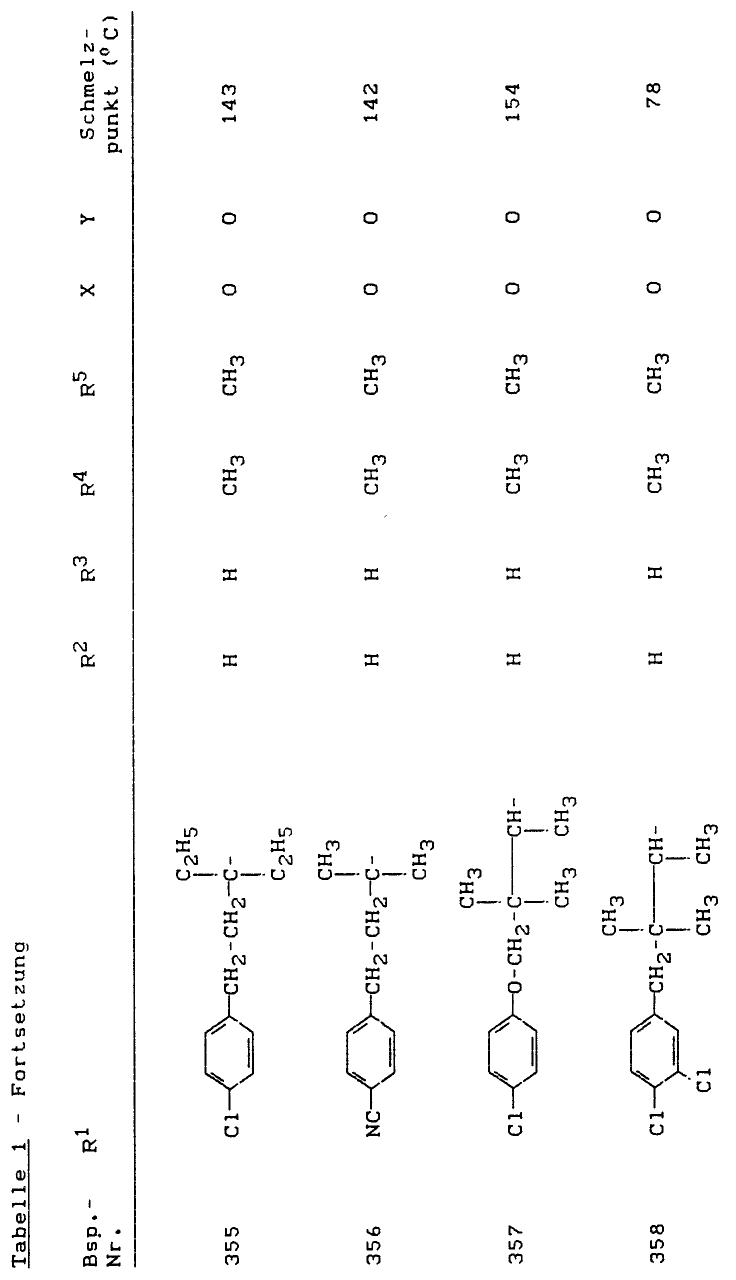

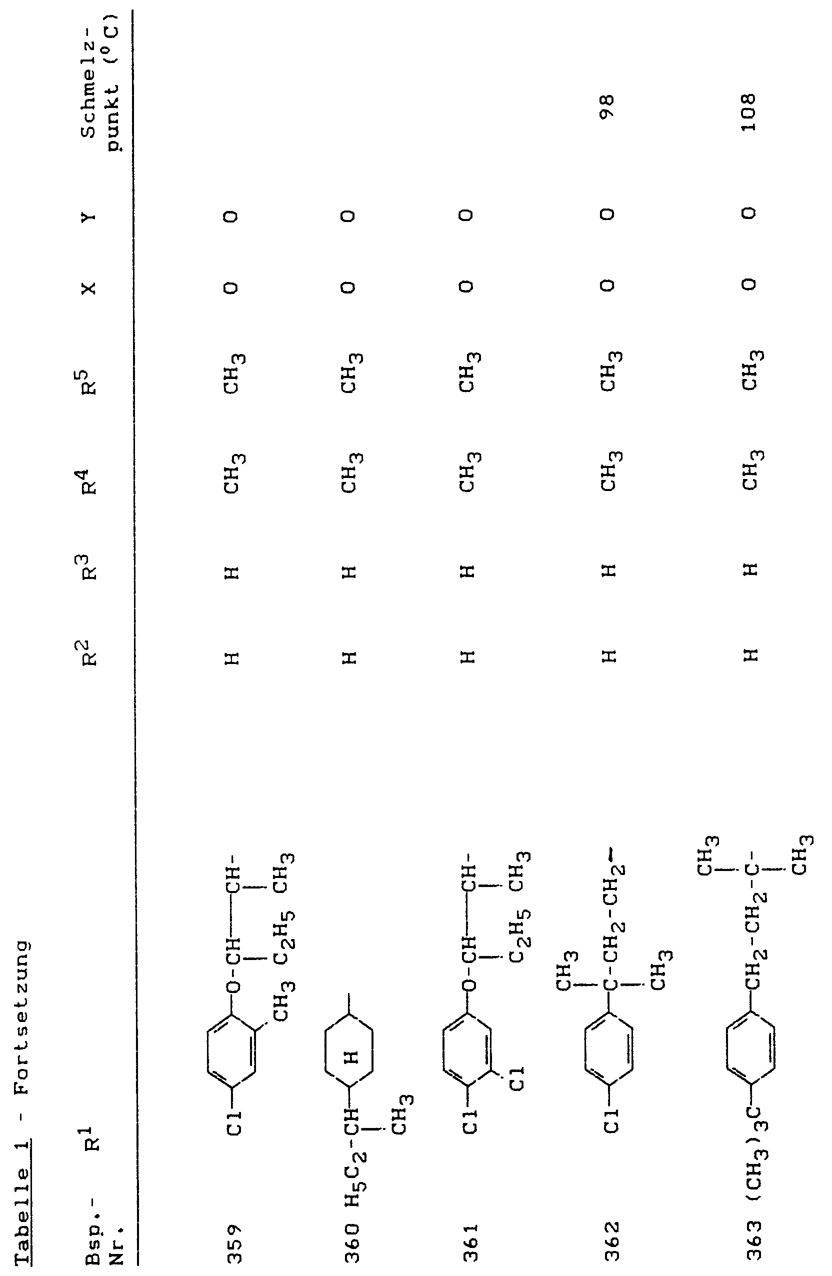

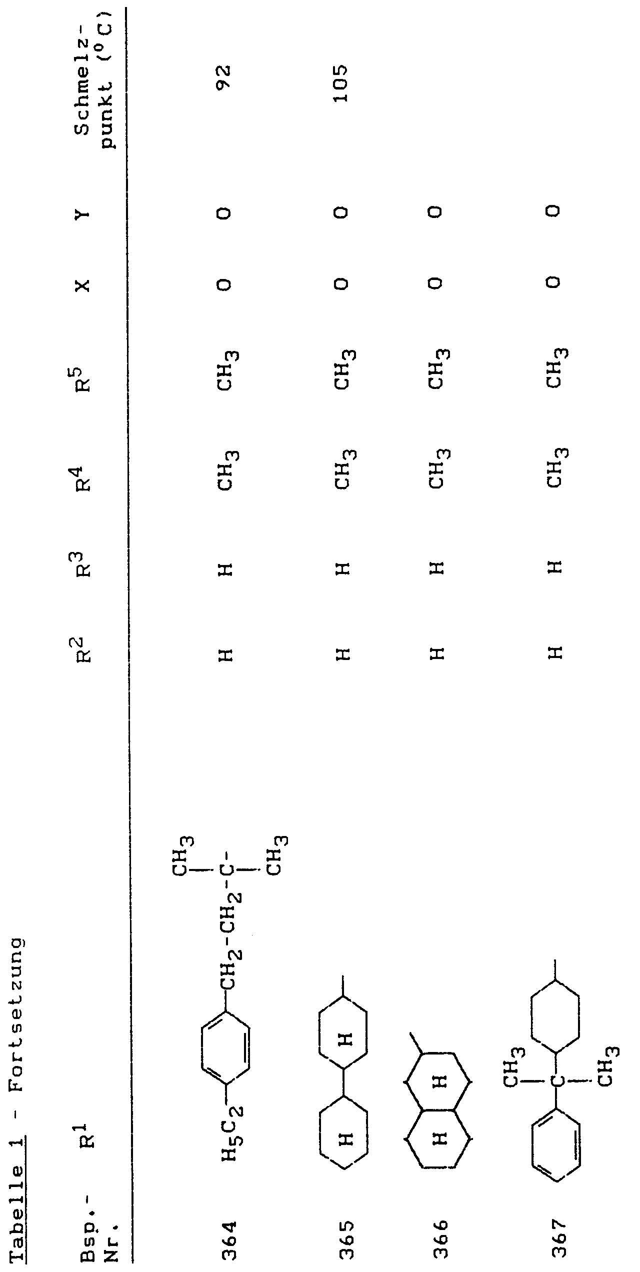

### Ausgangsstoffe der Formeln (II):

### Beispiel (II-1)

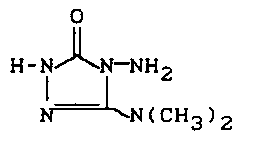

130 g (1.44 Mol) Carbodihydrazid werden in einer Mischung aus 470 g Phenol und 200 ml Chlorbenzol suspendiert, mit 153 g (1.44 Mol) Natriumcarbonat und 3.0 g (12 mMol) Dibutylzinnoxid versetzt und im Wasserstrahlvakuum auf 50°C bis 60°C erwärmt. Im Verlauf von 20 Minuten wird eine Lösung von 247 g (1.44 Mol) Tetramethylchlorformamidiniumchlorid in 400 g Phenol zugetropft, wobei Wasser abdestilliert. Dann wird bis zum Erreichen des Phenol-Siedepunktes Chlorbenzol im Wasserstrahlvakuum abdestilliert. Anschließend wird die Reaktionsmischung auf 160°C bis 180°C unter Normaldruck erhitzt, wobei Dimethylamin abgespalten wird. Nach 3 Stunden wird bei 190°C eine Stunde lang Phenol abdestilliert. Der verbleibende feste Rückstand wird im Soxleth-Extraktor mit 1.5 Liter Isopropanol extrahiert; die Isopropanol-Lösung wird eingedampft.

Man erhält 47 g (23% der Theorie) 4-Amino-5-dimethylamino-2,4-dihydro-3H-1,2,4-triazol-3-on als kristallinen Rückstand vom Schmelzpunkt 205°C.

### Beispiel (II-2)

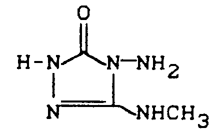

Eine Mischung aus 360 g (2.0 Mol) Carbodihydrazid, 212 g (2.0 Mol) Natriumcarbonat und 580 g Phenol wird auf 50°C bis 60°C erwärmt. Im Verlauf von 30 Minuten wird dann im Wasserstrahlvakuum eine Lösung von 314 g (2.0 Mol) Trimethyl-chlorformamidin-hydrochlorid in 314 g Phenol zugetropft. Dann wird das Reaktionsgemisch 60 Minuten bei 50°C bis 60°C gerührt und anschließend auf 190°C erwärmt, wobei Dimethylamin abgespaltet wird. Nach Ende der Gasentwicklung wird das Phenol im Wasserstrahlvakuum abdestilliert und der Rückstand aus Wasser umkristallisiert.

Man erhält 60 g (26% der Theorie) 4-Amino-5-methylamino-2,4-dihydro-3H-1,2,4-triazol-3-on vom Schmelzpunkt 220°C.

### Beispiel (II-3)

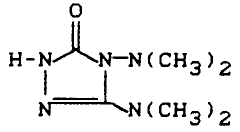

### Stufe 1:

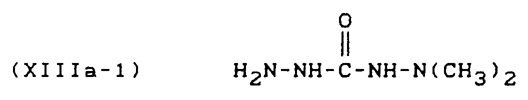

856 g (4.0 Mol) Kohlensäure-diphenylester werden unter Wasserkühlung vorgelegt und tropfenweise mit 245 g (4.0 Mol) N,N-Dimethylhydrazin versetzt. Dann wird langsam (innerhalb von 4 Stunden) auf 60°C erwärmt. Anschließend werden nach Abkühlen auf 20°C 200 g (4.0 Mol) Hydrazinhydrat dazu gegeben und die Reaktionsmischung wird 12 Stunden bei 20°C gerührt. Nach einstündigem Erwärmen auf 70°C bis 80°C werden flüchtige Komponenten im Wasserstrahlvakuum bis zu einer Sumpftemperatur von 100°C abdestilliert. Der verbleibende Rückstand enthält im wesentlichen eine Lösung von 1,1-Dimethyl-carbodihydrazid in Phenol, welche direkt für die nächste Stufe eingesetzt wird.

### Stufe 2:

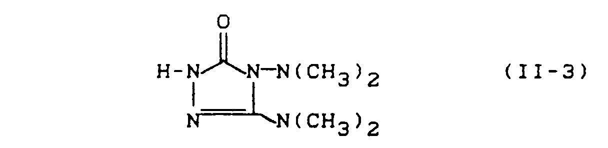

251 g der obigen Lösung aus 1,1-Dimethyl-carbodihydrazid in Phenol (ca. 0.82 Mol) werden mit 100 g Phenol verdünnt, mit 87 g (0.82 Mol) Natriumcarbonat versetzt und auf 50°C bis 60°C erwärmt. Im Ölpumpenvakuum wird dann eine Lösung von 140 g (0.82 Mol) Tetramethyl-chlorformamidiniumchlorid in 250 g Phenol im Verlauf von 75 Minuten zugetropft. Das Reaktionsgemisch wird dann unter Normaldruck 6 Stunden auf Rückflußtemperatur erhitzt (ca. 190°C bis 195°C), wobei Dimethylamin abgespalten wird. Dann wird im Ölpumpenvakuum destilliert und das Destillat erneut destilliert. Das nun erhaltene Destillat (40 g) wird in Xylol aufgenommen und auf -78°C abgekühlt. Das hierbei kristallin anfallende Produkt wird durch Absaugen isoliert.

Man erhält 22.8 g (16% der Theorie) 4.5-Bis-dimethylamino-2,4-dihydro-3H-1,2,4-triazol-3-on vom Schmelzpunkt 93°C.

### Beispiel (II-4)

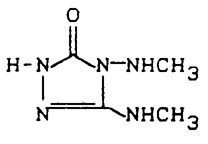

### Stufe 1:

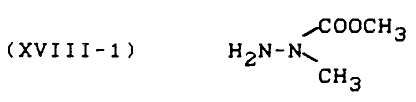

Eine Lösung von 94 g (2.0 Mol) Methylhydrazin in 100 ml Methanol wird auf 0°C bis -20°C abgekühlt und 268 g (2.0 Mol) Pyrokohlensäure-dimethylester kontinuierlich so zugetropft, daß die Temperatur von 0°C nicht überstiegen wird. Dann wird die Reaktionsmischung bis zum Ende der Gasentwicklung bei 80°C gerührt und anschließend im Wasserstrahlvakuum destilliert.

Man erhält 181 g (87% der Theorie) N-Methyl-N-methoxycarbonyl-hydrazin vom Siedepunkt 65°C/15 Torr.

### Stufe 2:

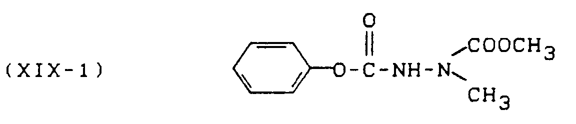

624 g (6.0 Mol) 1-Methyl-1-methoxycarbonyl-hydrazin und 334 g (3.15 Mol) Natriumcarbonat werden in 2 Liter Ethylenchlorid vorgelegt und unter Rühren werden 939 g (6.0 Mol) Chlorameisensäure-phenylester so zugetropft, daß die Temperatur von 20°C nicht überschritten wird. Dann wird das Gemisch noch 60 Minuten bei 60°C gerührt und das freigesetzte Natriumchlorid durch Absaugen abgetrennt. Vom Filtrat wird das Lösungsmittel im Wasserstrahlvakuum bei einer Sumpftemperatur von maximal 110°C abdestilliert.

Man erhält 1285 g (96% der Theorie) 1-Methyl-1-methoxycarbonyl-2-phenoxycarbonyl-hydrazin als öligen Rückstand, welcher allmählich kristallisiert.
Schmelzpunkt: 86°C.

### Stufe 3:

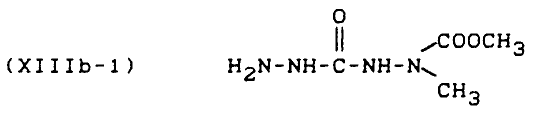

206 g (4.12 Mol) Hydrazinhydrat werden bei 20°C vorgelegt und eine auf 40°C erwärmte Lösung von 922 g (4.12 Mol) 1-Methyl-1-methoxycarbonyl-2-phenoxycarbonylhydrazin in 600 g Chlorbenzol wird in einem Guß dazu gegeben, worauf sich die Reaktionsmischung auf ca. 60°C erwärmt. Das Gemisch wird dann 4 Stunden bei 80°C gerührt und anschließend im Wasserstrahlvakuum bis zu einer Sumpftemperatur von ca. 100°C eingeengt. Der Rückstand, welcher im wesentlichen 1-Methyl-1-methoxycarbonyl-carbodihydrazid enthält wird direkt für die nächste Stufe eingesetzt.

### Stufe 4:

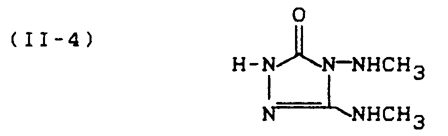

Eine Mischung aus 160 g (0.626 Mol) 1-Methyl-1-methoxycarbonyl-carbodihydrazid und 160 g Phenol wird auf 40°C erwärmt und mit 142 g (0.626 Mol) Methyliminokohlensäure-diphenylester versetzt. Nach Abklingen der exothermen Reaktion wird das Gemisch unter Wasserstrahlvakuum langsam auf 150°C erhitzt, wobei ca. 150 g Phenol abdestillieren. Nach Erkalten wird der Rückstand mit 100 ml Wasser und 120 g 50%iger Natronlauge verrührt und 60 Minuten unter Rückfluß erhitzt Dann werden 300 ml 18%ige Salzsäure hinzu gegeben und der Ansatz eingedampft. Der Rückstand wird im Ölpumpenvakuum destilliert und das Rohdestillat erneut destilliert.

Man erhält 45 g (50% der Theorie) 4,5-Bis-methylamino-2,4-dihydro-3H-1,2,4-triazol-3-on als öliges Produkt, welches in der Vorlage wachsartig erstarrt. Nach Verrühren mit Essigester werden 40 g (44%) weißkristallines Produkt vom Schmelzpunkt 135-137°C erhalten.

### Beispiel (II-5)

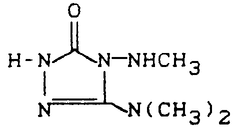

Wie unter Beispiel (II-4) - Stufe 3 - beschrieben, wird eine phenolische Lösung von 1-Methyl-1-methoxycarbonylcarbodihydrazid hergestellt. 384 g dieser Lösung (1.5 Mol) werden in 200 ml Chlorbenzol aufgenommen, mit 239 g (2.25 Mol) Natriumcarbonat versetzt und im Wasserstrahlvakuum auf 50°C erwärmt. Dabei wird eine Lösung von 244 g (1.5 Mol) Dichlormethylen-dimethylimmoniumchlorid in 566 g Phenol zugetropft, wobei Wasser azeotrop abdestilliert. Anschließend wird das Reaktionsgemisch noch 60 Minuten bei 120°C unter Normaldruck gerührt und dann heiß filtriert und mit Ethanol und Aceton gewaschen. Die organische Lösung wird eingeengt, der Rückstand mit 240 ml 50%iger Natronlauge unter Rückfluß erhitzt und nach Erkalten mit konz. Salzsäure neutralisiert. Nach Einengen im Wasserstrahlvakuum wird der Rückstand im Ölpumpenvakuum destilliert und das dabei erhaltene Rohdestillat erneut destilliert.

Man erhält 72 g öliges Produkt (Siedepunkt 165°C/1 mbar), das mit 300 ml Toluol in ein reines kristallines Produkt überführt wird.

Ausbeute: 60.0 g (25.5% der Theorie) 4-Methylamino-5-dimethylamino-2,4-dihydro-3H-1,2,4-triazol-3-on;
Schmelzpunkt: 129°C.

### Beispiel (II-6)

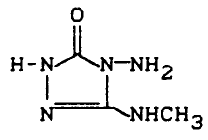

Eine Mischung aus 58 g (0.25 Mol) 1,3-Diamino-2-methylguanidin-hydroiodid, 53.5 g (0.25 Mol) Kohlensäure-diphenylester und 20 g Phenol wird unter Rühren auf 160°C erhitzt, bis die Mischung nahezu homogen geworden ist. Dann werden 34.5 g (0.25 Mol) Kaliumcarbonat portionsweise dazu gegeben, wobei Kohlendioxid freigesetzt wird. Nach 60 Minuten bei 160°C wird etwas abgekühlt und das Phenol im Wasserstrahlvakuum abdestilliert. Der Rückstand wird in 200 ml Wasser aufgenommen, mit Salzsäure neutralisiert und langsam mit Ethanol versetzt, wobei das Reaktionsgemisch kristallin anfällt.

Man erhält 24 g (74% der Theorie) 4-Amino-5-methylamino-2,4-dihydro-3H-1,2,4-triazol-3-on vom Schmelzpunkt 214°C.

Analog zu den Beispielen (II-1) bis (II-6) und entsprechend der allgemeinen Beschreibung der erfindungsgemäßen Herstellungsverfahren können auch die in der nachstehenden Tabelle 2 aufgeführten Verbindungen der Formel (II) hergestellt werden:

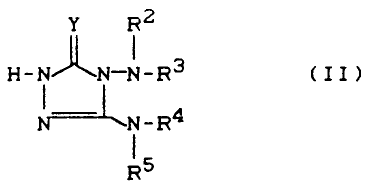

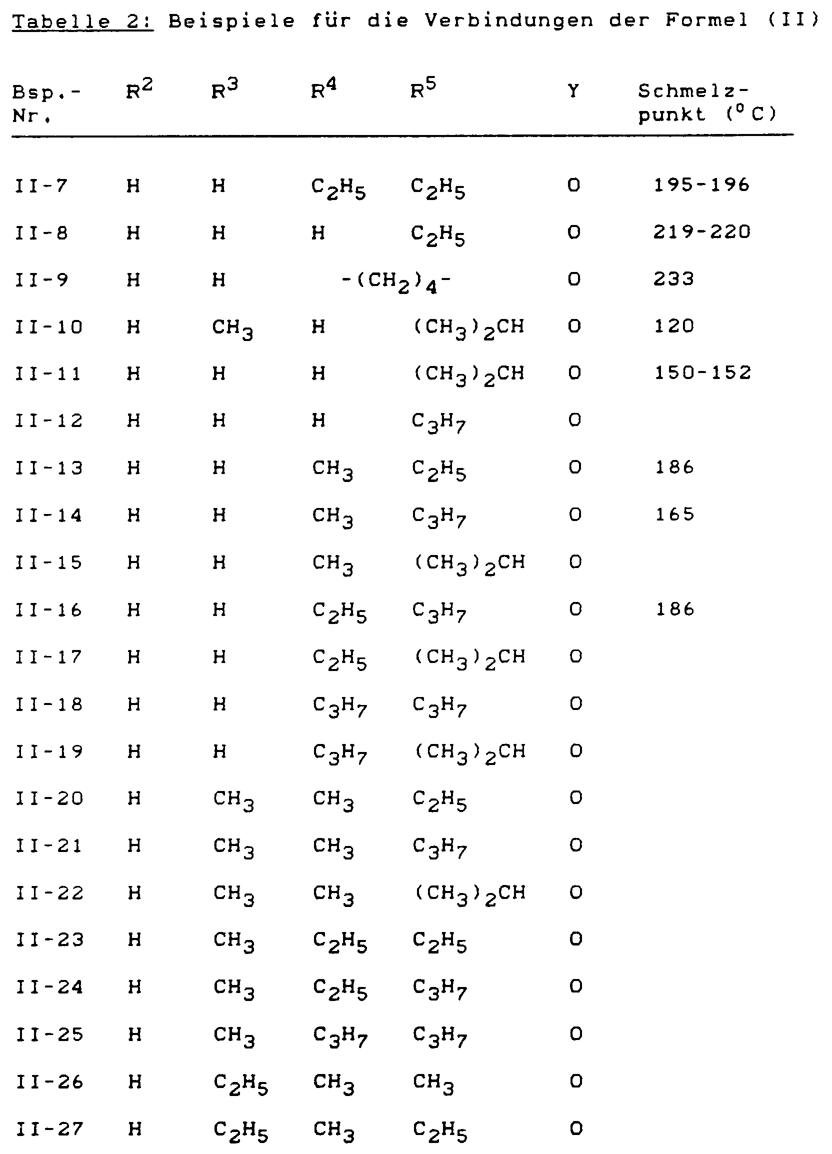

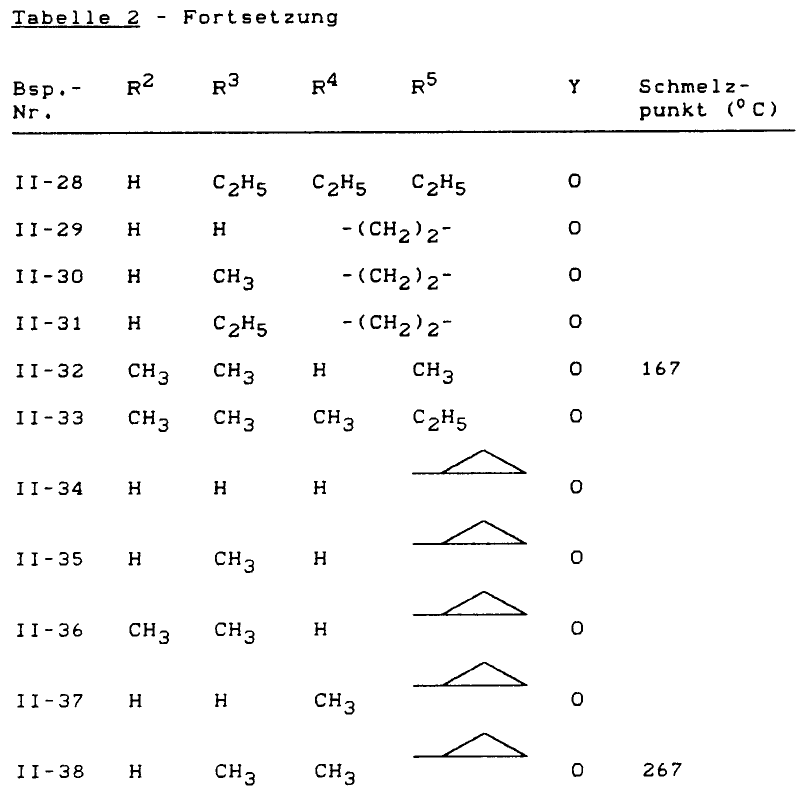

### Ausgangsstoffe der Formel (III):

### Beispiel (III-1)

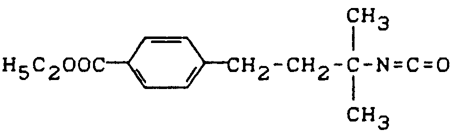

150 g (1,5 mol) Phosgen werden bei 15°C in 2,5 l Chlorbenzol vorgelegt und in diese Lösung unter Rühren eine Lösung von 235 g (1,0 mol) 1,1-Dimethyl-3-(4-ethoxycarbonyl-phenyl)-propylamin in 1,0 l Chlorbenzol tropfenweise eindosiert, wobei die Innentemperatur auf ca. 25°C ansteigt. Das Reaktionsgemisch wird dann auf ca 70°C erwärmt und unter weiterem Einleiten von Phosgen (ca. 50 g/h) und unter Rühren langsam zum Rückflußsieden (ca. 127°C) erhitzt und nach 30 Minuten unter Rückfluß phosgeniert. Aus der klaren Lösung wird ca. 1 l Chlorbenzol zusammen mit überschüssigem Phosgen abdestilliert. Schließlich wird im Wasserstrahlvakuum eingeengt und der Rückstand im Ölpumpenvakuum destilliert.

Man erhält 253 g (97% der Theorie) 1,1-Dimethyl-3-(4-ethoxycarbonyl-phenyl)-propyl-isocyanat vom Siedebereich 125°C-130°C/0,1 mbar.

Analog Beispiel (III-1) können beispielsweise auch die in der nachstehenden Tabelle 3 aufgeführten Verbindungen der Formel (III) hergestellt werden:

R¹-N=C=X (III)

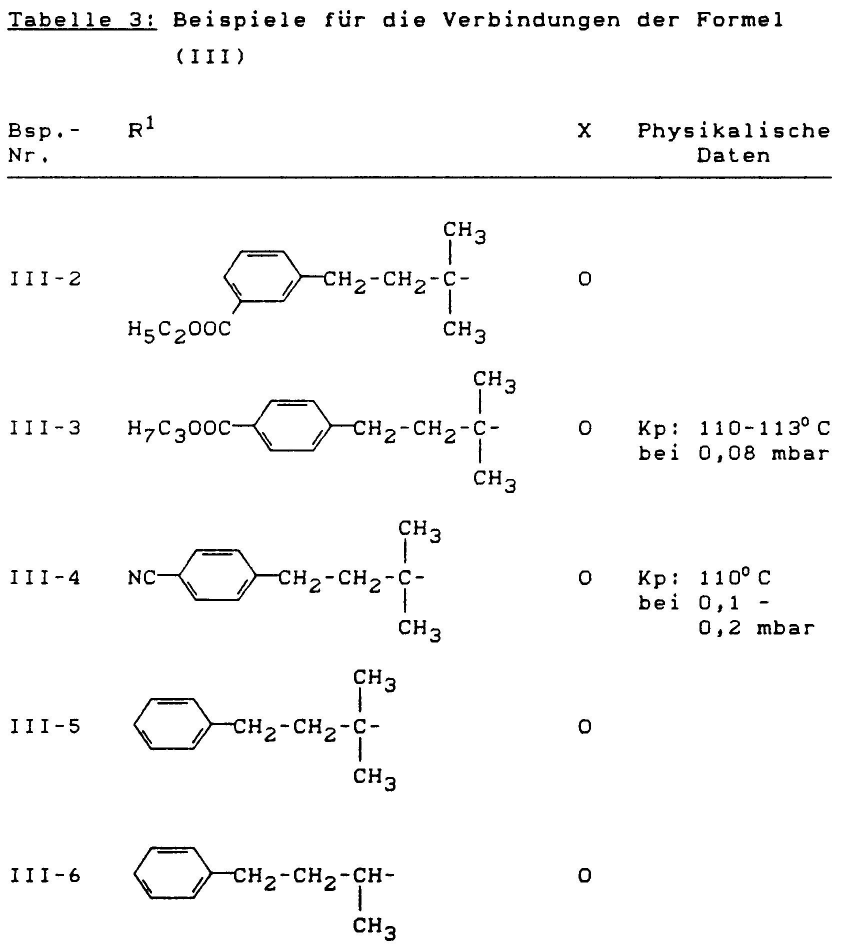

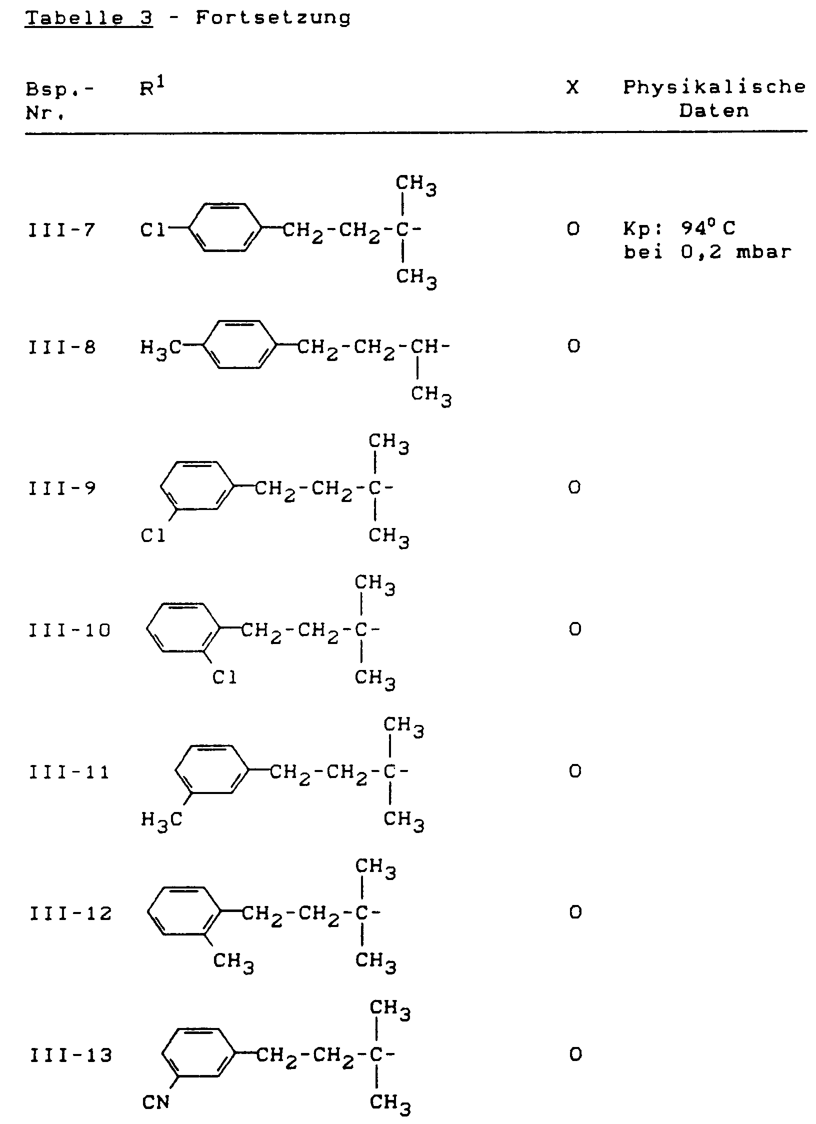

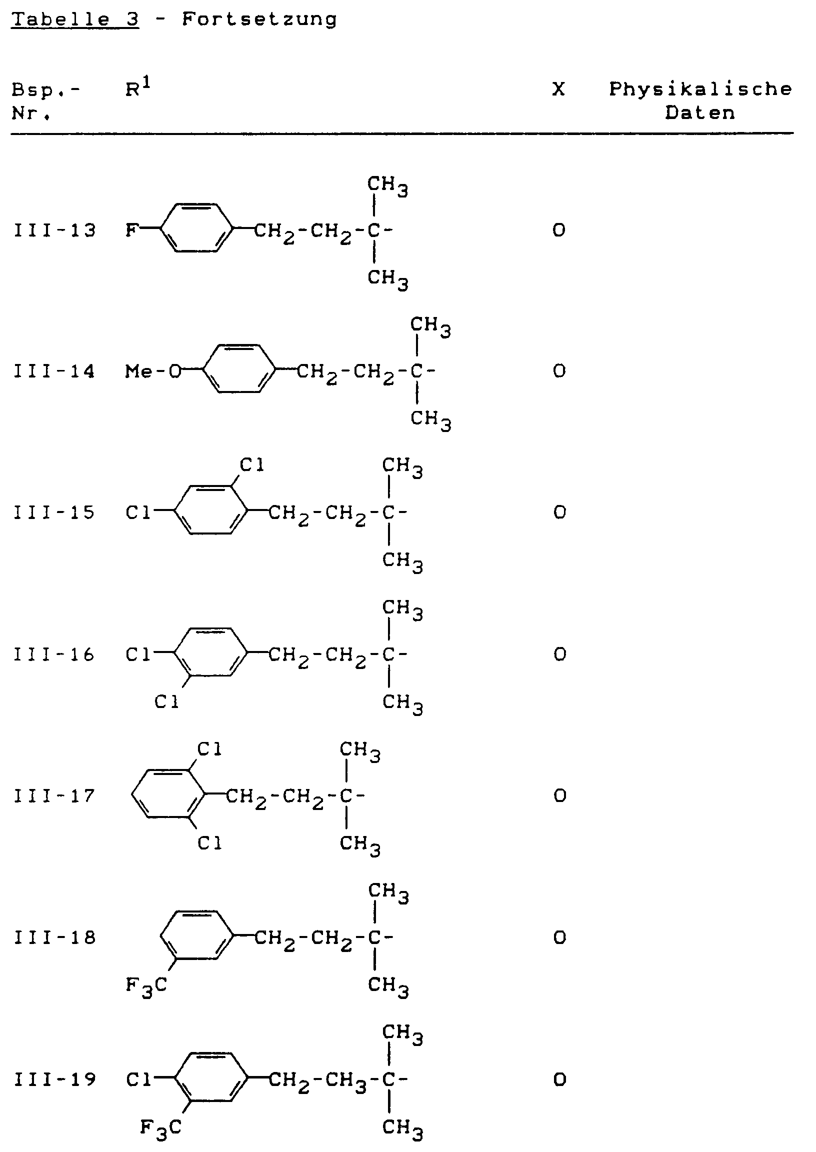

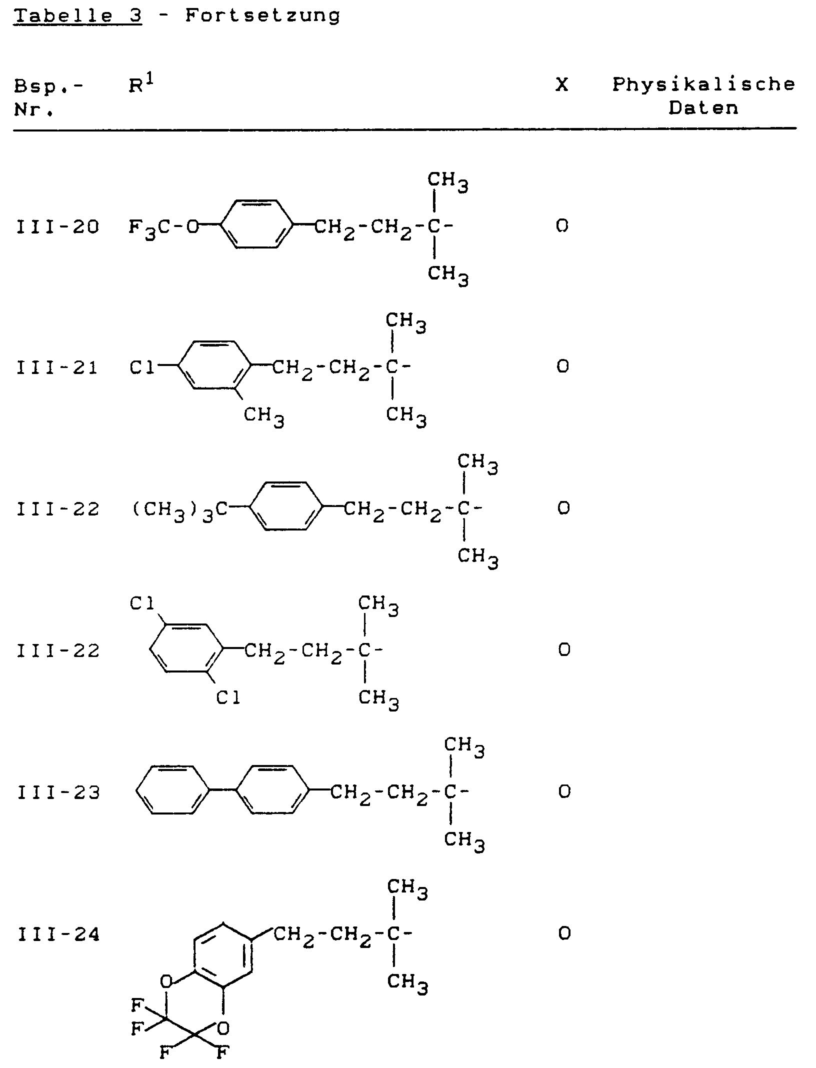

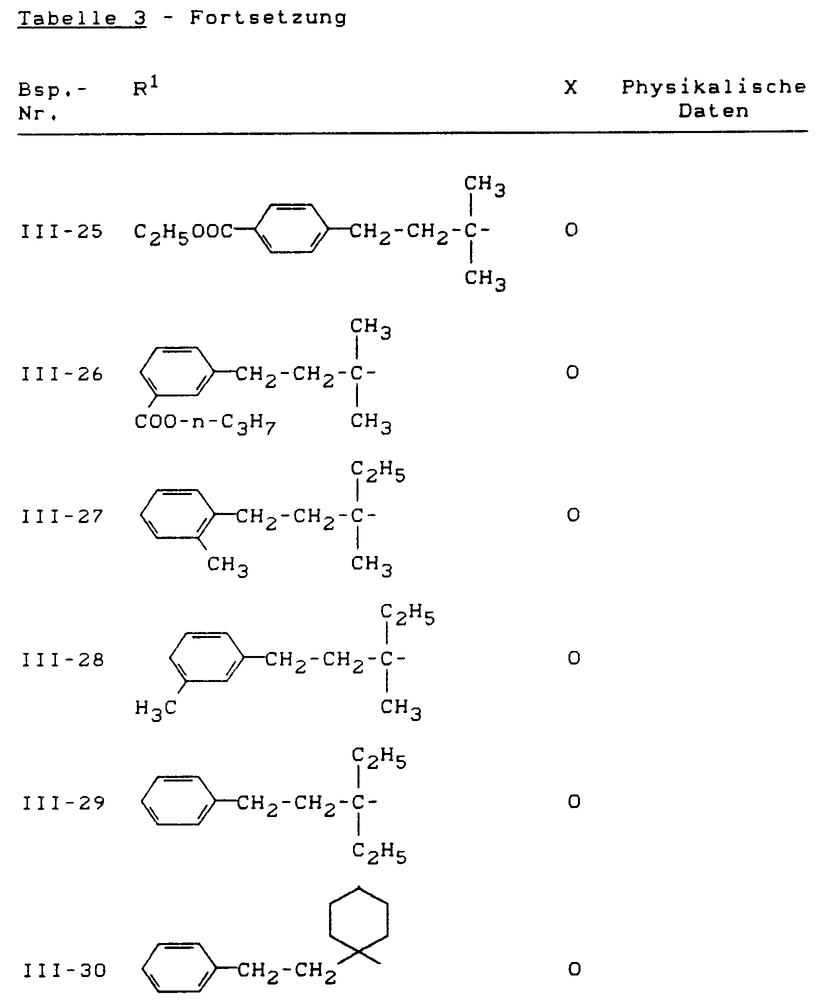

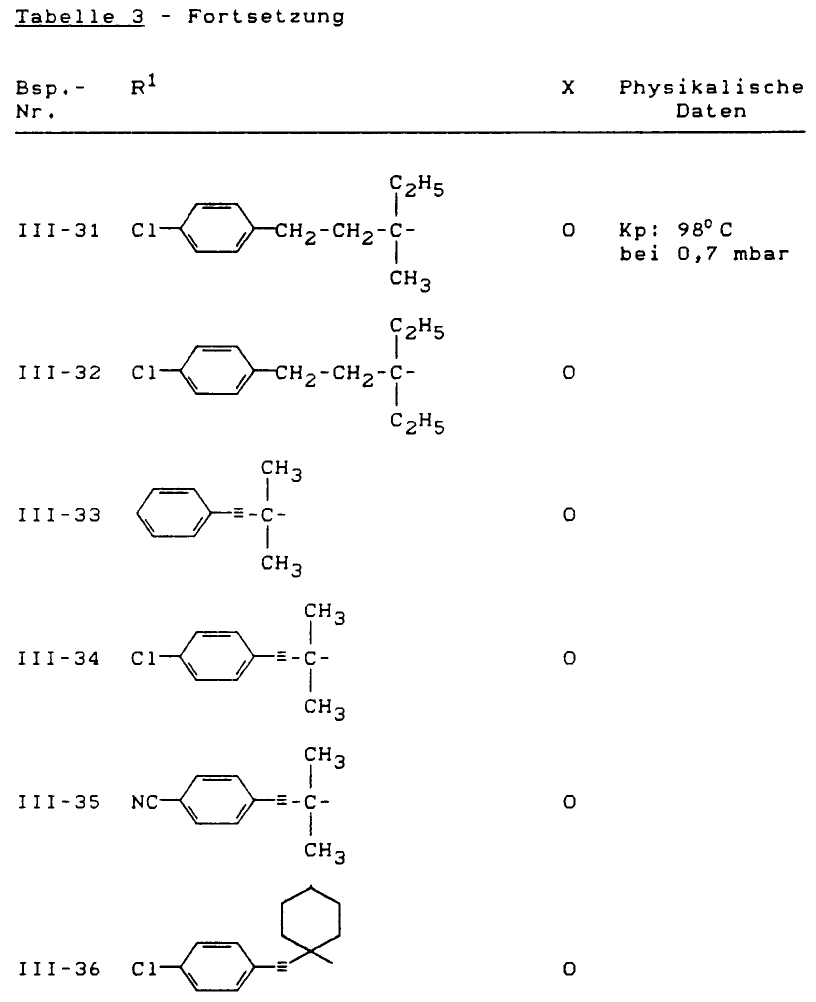

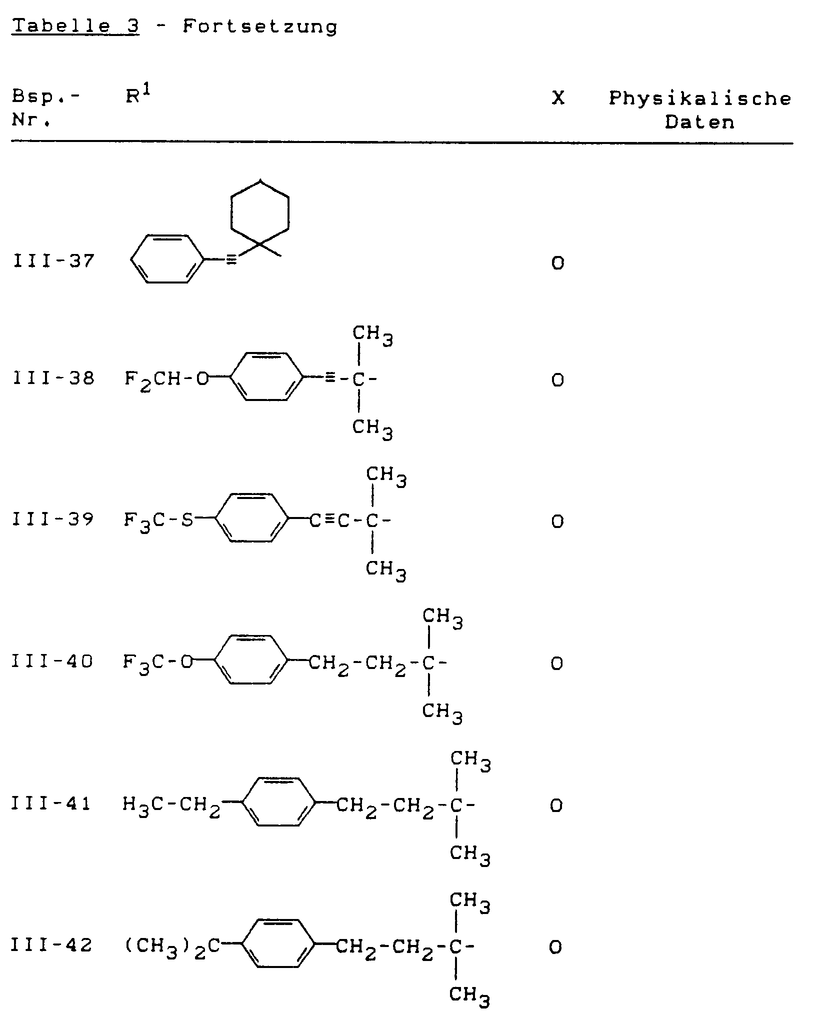

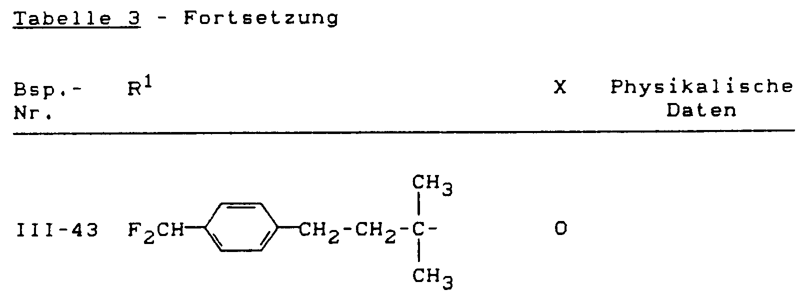

### Ausgangsstoffe der Formel (IV):

### Beispiel (IV-1)

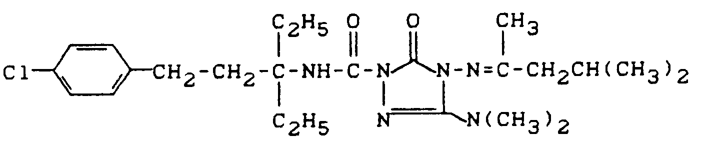

3,4 g (15 mMol) 4-(4-Methyl-2-pentyliden-amino)-5-dimethylamino-2,4-dihydro-3H-1,2,4-triazol-3-on werden in 100 ml Acetonitril gelöst und nacheinander mit 100 mg Diazabicycloundecen (DBU) und 3,8 g (15 mMol) 1-(4-Chlorphenyl)-3-ethylpent-3-yl-isocyanat versetzt. Das Reaktionsgemisch wird 2 Tage bei 20°C gerührt und anschließend eingeengt. Der Rückstand wird in Methylenchlorid aufgenommen, mit Wasser gewaschen, mit Natriumsulfat getrocknet und filtriert. Vom Filtrat wird das Lösungsmittel im Wasserstrahlvakuum sorgfältig abdestilliert. Der Rückstand kristallisiert beim Verreiben mit Petrolether.

Man erhält 4,3 g (60% der Theorie) [1-(4-Chlorphenyl)-3-ethylpent-3-yl-aminocarbonyl]-4-(4-methyl-2-pentylidenamino)-5-dimethylamino-2,4-dihydro-3H-1,2,4-triazol-3-on vom Schmelzpunkt 121°C.

Analog zu Beispiel (IV-1) und entsprechend der allgemeinen Beschreibung der erfindungsgemäßen Herstellungsverfahren können auch die in der nachstehenden Tabelle 4 aufgeführten Verbindungen der Formel (IV) hergestellt werden.

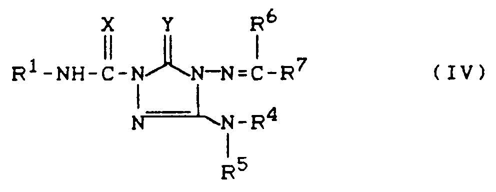

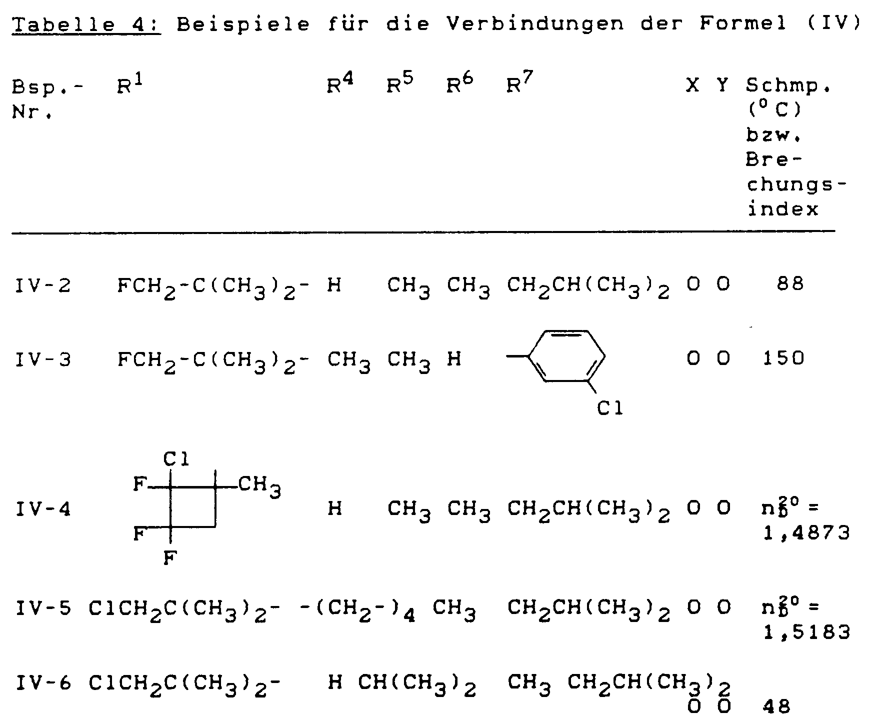

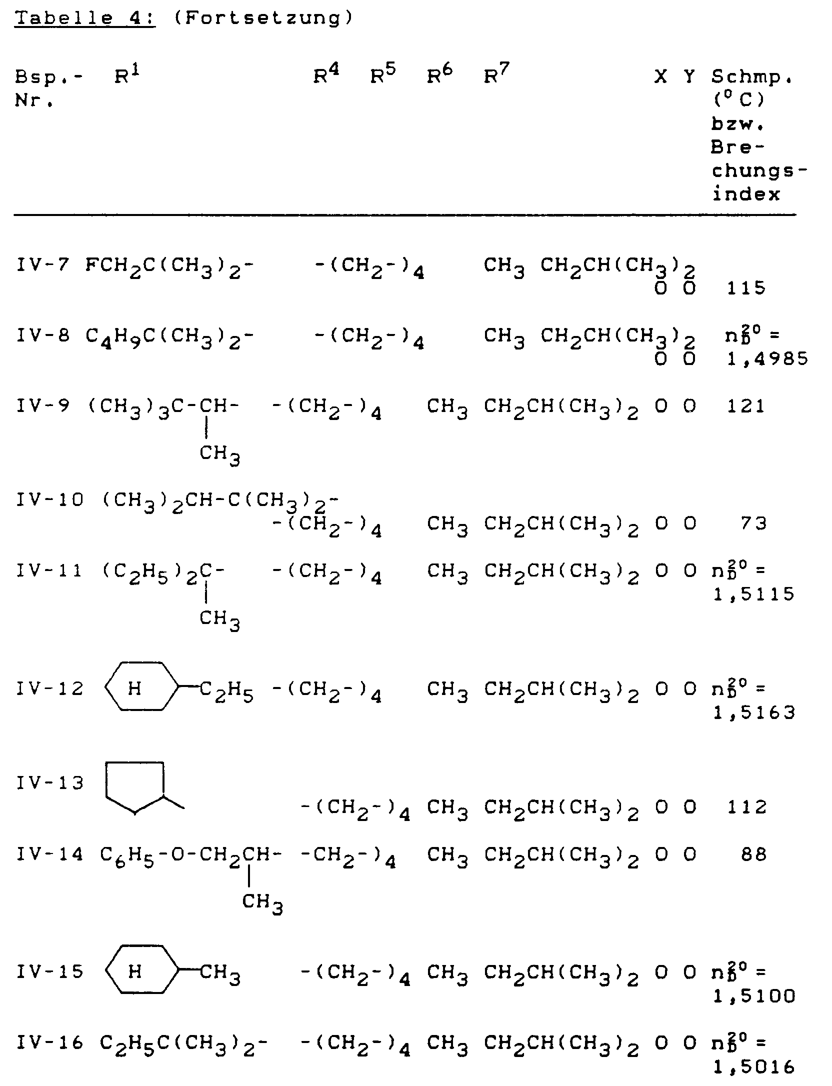

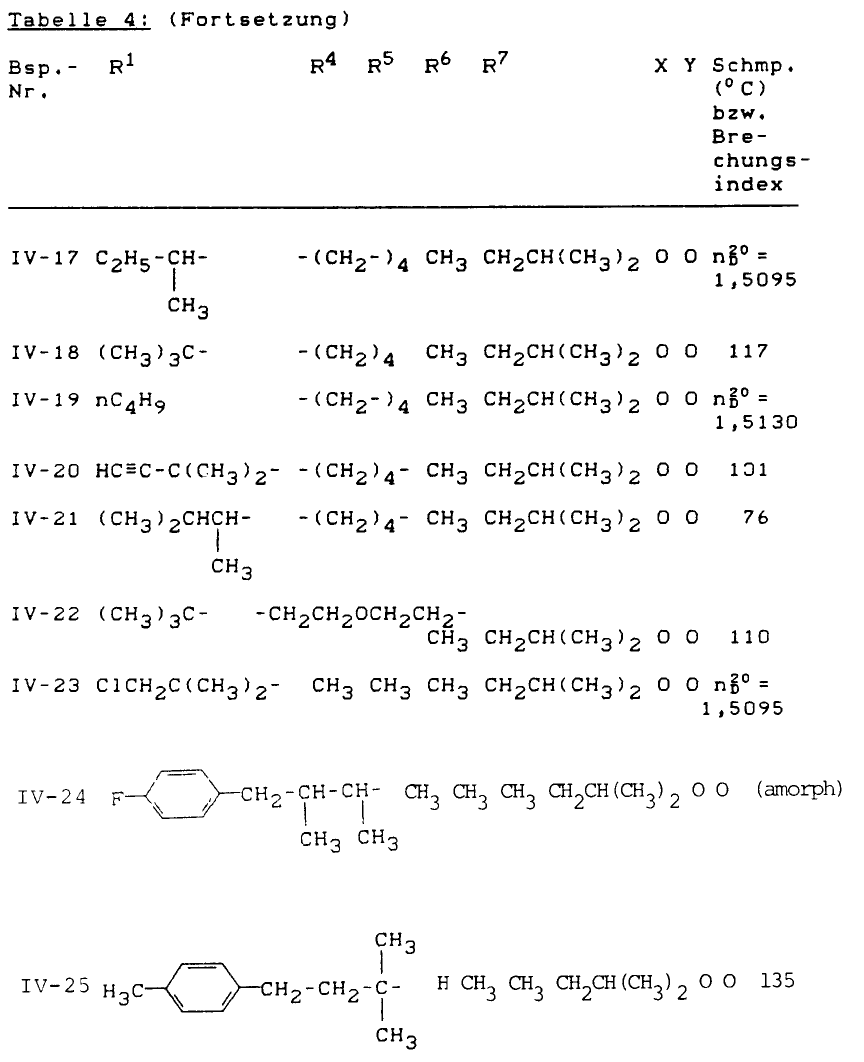

### Ausgangsstoffe der Formel (VI):

### Beispiel (VI-1)

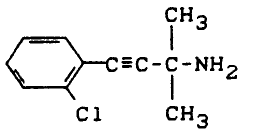

96,7 g (0,50 Mol) 1-Brom-2-chlor-benzol und 45,7 g (0,55 Mol) 3-Amino-3,3-dimethyl-1-propin werden in 500 ml Triethylamin vorgelegt. Nach Zugabe von 7,0 g (0,01 Mol) Palladium(II)-bis(triphenylphosphin)-dichlorid, 7,6 g (0,04 Mol) Kupfer(I)-iodid und 21,0 g (0,4 Mol) Triphenylphosphin wird das Reaktionsgemisch für 24 Stunden zum Rückflußsieden erhitzt. Dann wird filtriert und das Filtrat im Wasserstrahlvakuum eingeengt. Der Rückstand wird mit Methylenchlorid/Wasser (ca. 300 ml/300 ml) extrahiert, die organische Phase abgetrennt, mit Natriumsulfat getrocknet und filtriert. Vom Filtrat wird das Lösungsmittel im Wasserstrahlvakuum abdestilliert und das verbleibende Rohprodukt durch Destillation im Ölpumpenvakuum gereinigt.

Man erhält 74,1 g (75% der Theorie) 3-Amino-3,3-dimethyl-1-(2-chlor-phenyl)-1-propin vom Brechungsindex n$\frac{\text{21}}{\text{D}}$ = 1,5799.

### Beispiel (VI-2)

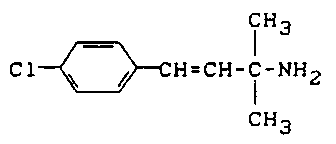

96,8 g (0,50 Mol) 3-Amino-3,3-dimethyl-1-(4-chlor-phenyl)-1-propin werden in einer Hydrierapparatur nach Parr mit 400 ml Tetrahydrofuran vermischt und mit 13,0 g Lindlar-Katalysator (5% Palladium auf Calciumcarbonat mit Blei dotiert) versetzt. Das Gemisch wird dann unter einem Wasserstoffdruck von 3 bar bei allmählich von 25°C auf 50°C aussteigender Temperatur bis zum Ende der berechneten Wasserstoff-Aufnahme (1 Moläquivalent nach etwa 15 Stunden) geschüttelt. Dann wird filtriert, das Filtrat im Wasserstrahlvakuum eingeengt und das verbleibende Rohprodukt durch Destillation im Ölpumpenvakuum gereinigt.

Man erhält 67,5 g (69% der Theorie) 3-Amino-3,3-dimethyl-1-(4-chlor-phenyl)-1-propen vom Brechungsindex n$\frac{\text{21}}{\text{D}}$ = 1,5528.

### Beispiel (VI-3)

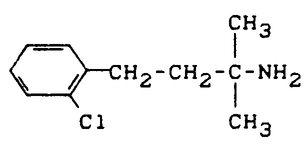

40,6 g (0,21 Mol) 3-Amino-3,3-dimethyl-1-(2-chlor-phenyl)-1-propin werden in einem Rührautoklaven mit 250 ml Tetrahydrofuran vermischt und mit 10 g Raney-Nickel versetzt. Wasserstoff wird dann bis zu einem Druck von 50 bar eindosiert und das Gemisch unter Rühren allmählich von 25°C auf 40°C erwärmt. Der Wasserstoffdruck wird jeweils nach Absinken auf 40 bar immer wieder auf 50 bar eingestellt, bis der Druck konstant bleibt. Anschließend wird filtriert, das Filtrat im Wasserstrahlvakuum eingeengt und das verbleibende Rohprodukt im Ölpumpenvakuum destilliert.

Man erhält 31,8 g (77% der Theorie) 3-Amino-3,3-dimethyl-1-(2-chlor-phenyl)-propan vom Brechungsindex n$\frac{\text{21}}{\text{D}}$ = 1,4817.

Analog zu den Beispielen (VI-1) bis (VI-3) können beispielsweise auch die in der nachstehenden Tabelle 5 aufgeführten Verbindungen der Formel (VI) hergestellt werden.

R¹-NH₂ (V)

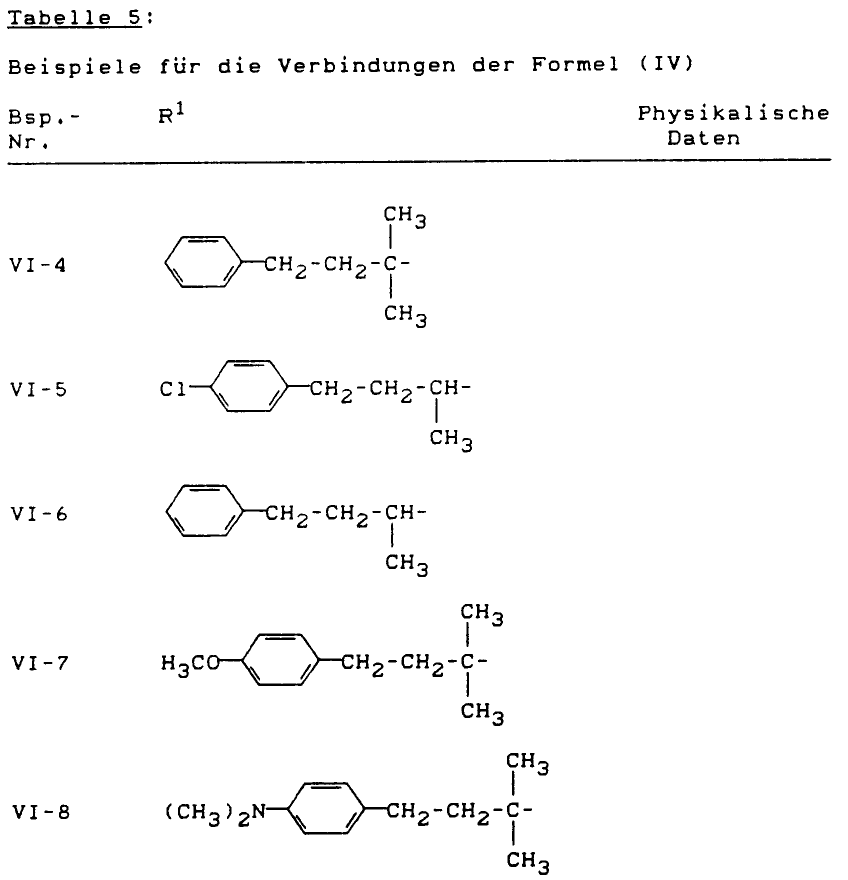

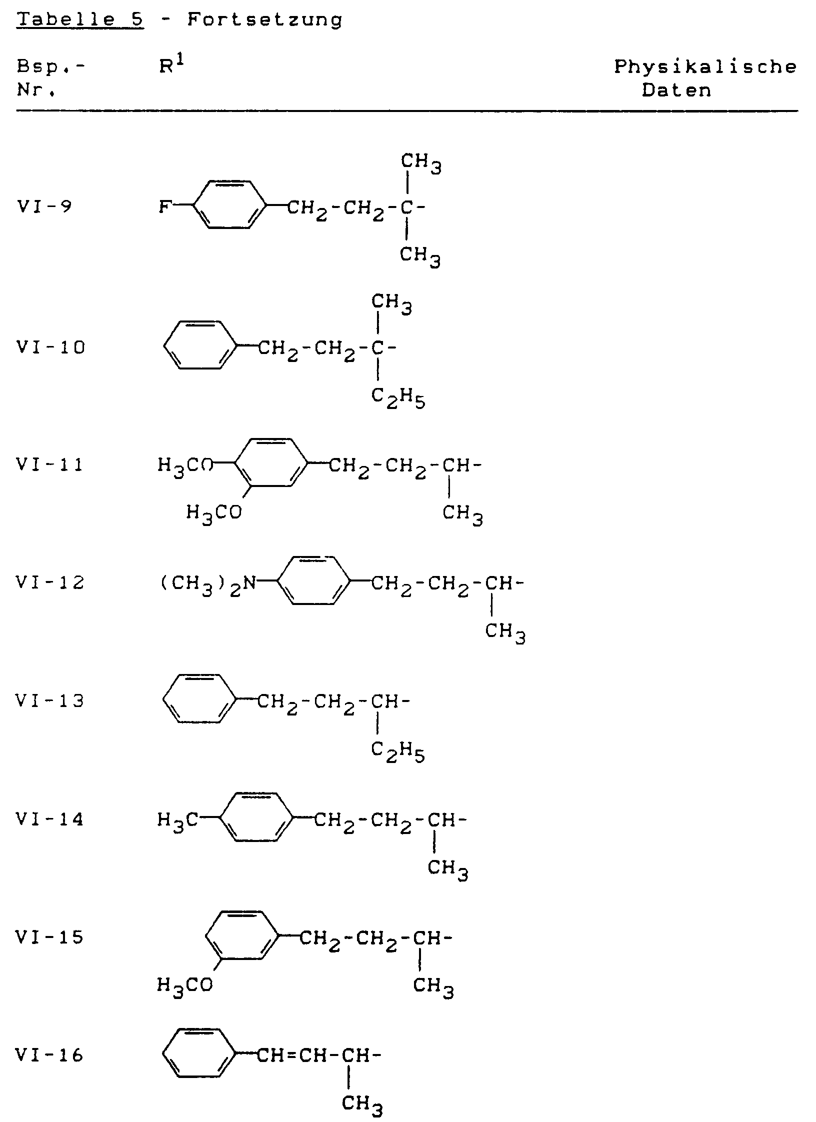

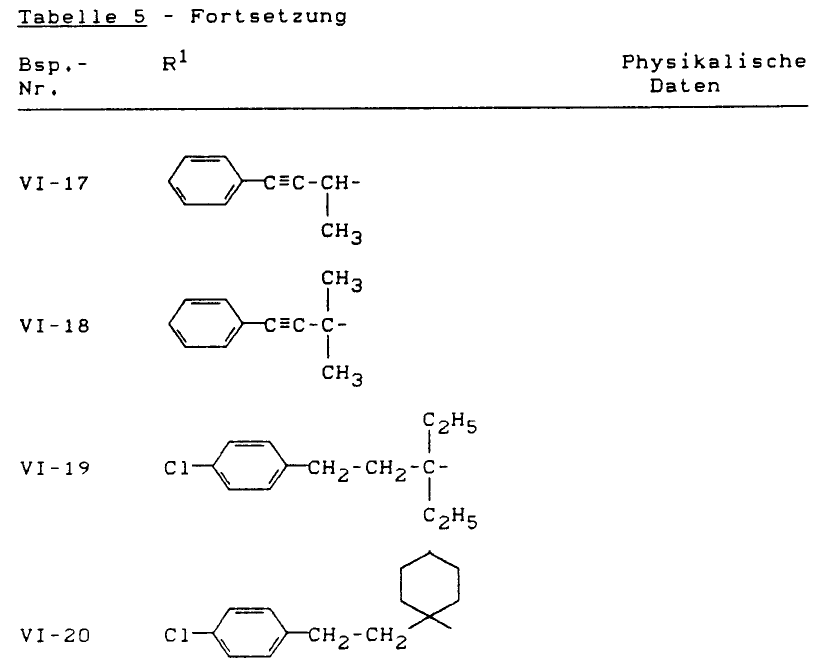

### Ausgangsstoffe der Formel (VIII):

### Beispiel (VIII-1)

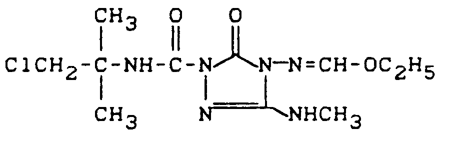

3,7 g (0.02 Mol) 4-Ethoxymethylenamino-5-methylamino-2,4-dihydro-3H-1,2,4-triazol-3-on werden in 100 ml Acetonitril gelöst und nacheinander mit 100 mg Diazabicycloundecen (DBU) und 2.8 g (0.02 Mol) Chlor-tert-butylisocyanat versetzt. Das Gemisch wird 12 Stunden bei 20°C gerührt und dann eingeengt. Der Rückstand wird in Methylenchlorid aufgenommen, mit Wasser gewaschen, mit Natriumsulfat getrocknet und filtriert. Vom Filtrat wird das Lösungsmittel im Wasserstrahlvakuum abdestilliert und der Rückstand durch Verreiben mit Diethylether zur Kristallisation gebracht.

Man erhält 5.2 g (82% der Theorie) 2-Chlor-tert-butylaminocarbonyl-4-ethoxymethylenamino-5-methylamino-2,4-dihydro-3H-1,2,4-triazol-3-on vom Schmelzpunkt 142°C.

Analog zu Beispiel VIII-1 und entsprechend der allgemeinen Beschreibung der erfindungsgemäßen Herstellungsverfahren können auch die in der nachstehenden Tabelle 6 aufgeführten Verbindungen der Formel (VIII) hergestellt werden.

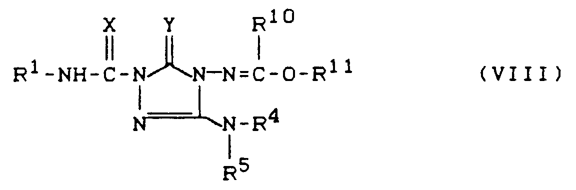

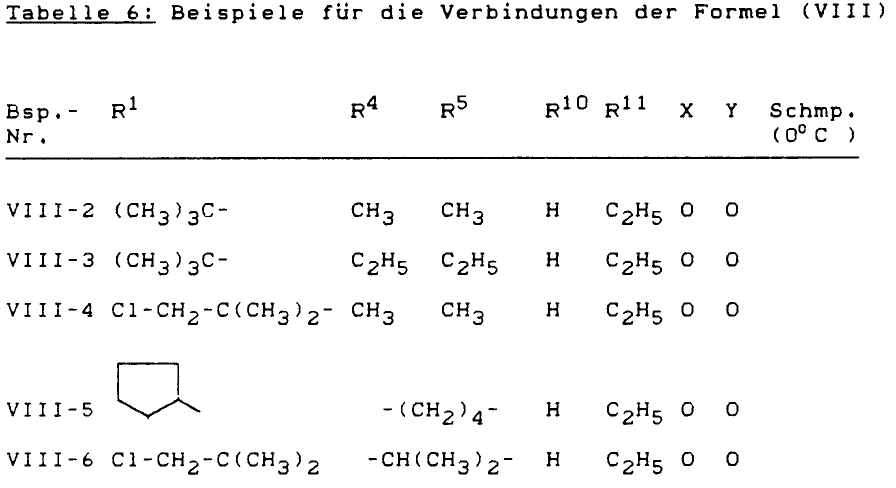

### Zwischenprodukte der Formel (XXI):

### Beispiel (XXI-1)

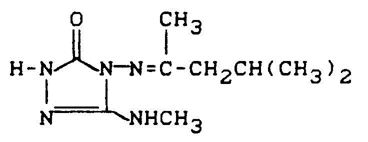

12.9 g (0.1 Mol) 4-Amino-5-methylamino-2,4-dihydro-3H-1,2,4-triazol-3-on werden mit 150 ml Methyl-isobutylketon und 100 mg p-Toluolsulfonsäure unter Rückfluß am Wasserabscheider erhitzt, bis praktisch kein Wasser mehr abgeschieden wird (ca. 2 Stunden). Man filtriert, engt das Filtrat ein und verreibt den Rückstand mit Petrolether.

Man erhält 8.3 g (39% der Theorie) 4-(4-Methyl-2-pentyliden-amino)-5-methylamino-2,4-dihydro-3H-1,2,4-triazol-3-on vom Schmelzpunkt 136°C.

Analog zu dem Beispiel (XXI-1) und entsprechend der allgemeinen Beschreibung der erfindungsgemäßen Herstellungsverfahren können auch die in der nachstehenden Tabelle 7 aufgeführten Verbindungen der Formel (XXI) hergestellt werden:

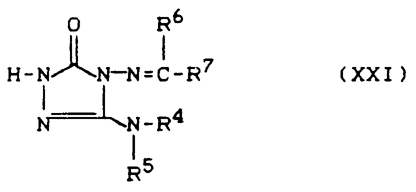

**Tabelle 7:**

| Beispiele für die Verbindungen der Formel (XXI) | | | | | | |
|---|---|---|---|---|---|---|
| Bsp.-Nr. | R⁴ | R⁵ | R⁶ | R⁷ | Y | Schmp. (°C) |
| XXI-2 | CH₃ | CH₃ | CH₃ | CH₃ | 0 | 197 |
| XXI-3 | CH₃ | CH₃ | CH₃ | (CH₃)₂CH-CH₂- | O | 105 |
| XXI-4 | -(CH₂)₄- | | CH₃ | (CH₃)₂CH-CH₂- | O | 133 |
| XXI-5 | H | C₂H₅ | CH₃ | (CH₃)₂CH-CH₂- | O | 105 |
| XXI-6 | H | (CH₃)₂CH | CH₃ | (CH₃)₂CH-CH₂- | O | 94 |
| XXI-7 | H | C₃H₇ | CH₃ | (CH₃)₂CH-CH₂- | O | |
| XXI-8 | CH₃ | C₂H₅ | CH₃ | (CH₃)₂CH-CH₂- | O | |
| XXI-9 | C₂H₅ | C₂H₅ | CH₃ | (CH₃)₂CH-CH₂- | O | 80 |
| XXI-10 | CH₃ | C₃H₇ | CH₃ | (CH₃)₂CH-CH₂- | O | |

### Zwischenprodukte der Formel (XXV):

### Beispiel (XXV-1)

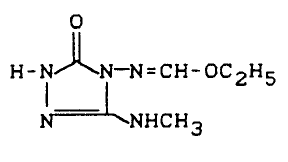

12.9 g (0.1 Mol) 4-Amino-5-methylamino-2,4-dihydro-3H-1,2,4-triazol-3-on werden mit 100 ml Orthoameisensäuretriethylester und 100 mg p-Toluolsulfonsäure 4 Stunden zum Rückflußsieden erhitzt. Dann wird eingeengt und mit Diethylether zur Kristallisation gebracht. Nach Umkristallisation aus Ethanol erhält man 10.0 g (54% der Theorie) 4-Ethoxymethylenamino-5-methylamino-2,4-dihydro-3H-1,2, 4-triazol-3-on vom Schmelzpunkt 169°C.

Analog zu Beispiel (XXV-1) und entsprechend der allgemeinen Beschreibung der erfindungsgemäßen Herstellungsverfahren können auch die in der nachstehenden Tabelle 8 aufgeführten Verbindungen der Formel (XXV) hergestellt werden:

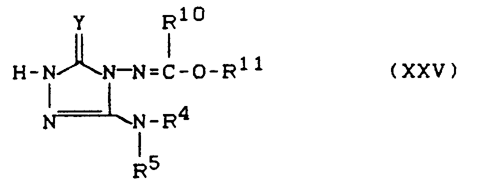

**Tabelle 8**

| Beispiele für die Verbindungen der Formel (XXV) | | | | | | |
|---|---|---|---|---|---|---|
| Bsp.-Nr. | R⁴ | R⁵ | R¹⁰ | R¹¹ | Y | Schmp. (°C) |
| XXV-2 | CH₃ | CH₃ | H | C₂H₅ | O | 72 |
| XXV-3 | CH₃ | C₂H₅ | H | C₂H₅ | O | |
| XXV-4 | CH₃ | C₃H₇ | H | C₂H₅ | O | |
| XXV-5 | H | CH₃ | CH₃ | C₂H₅ | O | |
| XXV-6 | -(CH₂)₄- | | H | C₂H₅ | O | |
| XXV-7 | C₂H₅ | C₂H₅ | H | C₂H₅ | O | |
| XXV-8 | H | CH(CH₂)₂ | H | C₂H₅ | O | |

### Anwendungsbeispiele:

In den folgenden Anwendungsbeispielen wurde die nachstehend aufgeführte Verbindung als Vergleichssubstanz eingesetzt:

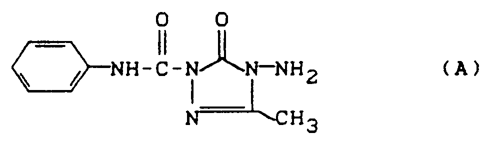

4-Amino-5-methyl-2-phenylaminocarbonyl-2,4-dihydro-3H-1,2,4-triazol-3-on
(bekannt aus EP-A 294666, Beispiel 122).

### Beispiel A

### Post-emergence-Test

| | | |
|---|---|---|
| Lösungsmittel: | 5 Gewichtsteile | Aceton |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung
Eine deutliche Überlegenheit in der Wirksamkeit gegenüber dem Stand der Technik zeigen in diesem Test z.B. die Verbindungen gemäß folgender Herstellungsbeispiele:
1, 2, 6, 7, 15, 16, 17, 18, 20, 21, 22, 23, 24, 25, 26, 27, 28, 30, 46, 50, 52, 53, 54, 55, 56, 57, 58, 59, 60, 61, 62, 63, 66, 71, 72, 73, 76, 77, 78, 79, 81, 82, 84, 85 und 87.

### Beispiel B

### Entlaubung und Austrocknung der Blätter bei Baumwolle

| | | |
|---|---|---|
| Lösungsmittel: | 30 Gewichtsteile | Dimethylformamid |
| Emulgator: | 1 Gewichtsteil | Polyoxyethylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

In diesem Test zeigen beispielsweise die Verbindungen gemäß Herstellungsbeispiel 15 und 16 an Baumwolle starke Entlaubung und Austrocknung der Blätter.

## Patentansprüche

1. Substituierte 4,5-Diamino-1,2,4-triazol-3-(thi)-one der allgemeinen Formel (I) 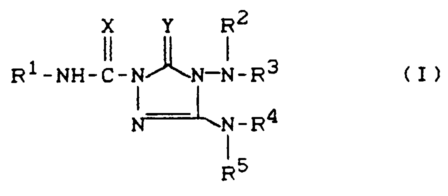 in welcher
R¹ für Alkyl, Alkenyl, Alkinyl, Halogenalkyl, Halogenalkenyl, Halogenalkinyl, Cyanalkyl, Hydroxyalkyl, Alkoxyalkyl, Aryloxyalkyl, Arylthioalkyl, Arylsulfinylalkyl, Arylsulfonylalkyl, Arylaminoalkyl, N-Alkyl-arylaminoalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Arylalkenyl, Arylalkinyl, Aroyl, Aryl, Aralkyloxy oder Aryloxy, für Alkoxy, Alkenyloxy oder Alkinyloxy steht,
R², R³, R⁴ und R⁵ gleich oder verschieden sind und unabhängig voneinander für Wasserstoff, Alkyl, Alkenyl, Alkinyl, Halogenalkyl, Halogenalkenyl, Halogenalkinyl, Cyanalkyl, Alkoxyalkyl, Alkylthioalkyl, für jeweils gegebenenfalls substituiertes Cycloalkyl, Cycloalkylalkyl, Aryl oder Aralkyl stehen, oder jeweils zwei dieser Reste (R² und R³ oder R⁴ und R⁵) zusammen für Alkandiyl stehen, und R⁵ auch für Alkoxy stehen kann,
X für Sauerstoff oder Schwefel steht und
Y für Sauerstoff oder Schwefel steht.

2. Substituierte 4,5-Diamino-1,2,4-triazol-3-(thi)one der allgemeinen Formel (I) gemäß Anspruch 1, bei welchen
R¹ für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 1 bis 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl, Phenylthioalkyl, Phenylsulfinylalkyl, Phenylsulfonylalkyl, Phenylaminoalkyl oder N-(C₁-C₄-Alkyl)-phenylaminoalkyl mit jeweils 1 bis 4 Kohlenstoffatomen im Alkylteil, Alkoxyalkyl, Alkoxycarbonylalkyl oder Alkoxycarbonylalkenyl mit jeweils 1 bis 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach bis dreifach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl-bzw. Cycloalkenylteil und gegebenenfalls 1 bis 6 Kohlenstoffatomen im Alkylteil steht, wobei als Substituenten jeweils infrage kommen: Halogen, Cyano, Phenyl, Cyclohexyl, Phenylethyl, Phenylisopropyl sowie jeweils geradkettiges oder verzweigtes Alkyl oder Halogenalkyl mit jeweils 1 bis 4 Kohlenstoffatomen und gegebenenfalls 1 bis 9 gleichen oder verschiedenenen Halogenatomen und geradkettiges oder verzweigtes Halogenalkenyl mit 1 bis 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; R¹ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen -insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; R¹ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach bis dreifach, gleich oder verschieden substituiertes Aralkyl, Arylalkenyl, Arylalkinyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil bzw. 2 bis 8 Kohlenstoffatomen im geradkettigen oder verzweigten Alkenylteil oder Alkinylteil steht, wobei die Wasserstoffatome des α-Kohlenstoffatoms durch Ethan-1,2-diyl, Propan-1,3-diyl, Butan-1,4-diyl oder Pentan-1,5-diyl ersetzt sein können und wobei als Alkylsubstituenten gegebenenfalls Halogen und Cyano infrage kommen und wobei als Arylsubstituenten jeweils infrage kommen: Halogen, Cyano, Nitro, Amino, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder R¹ für Benzyl mit im Phenylteil ankondensierter -O-CH₂-O- Gruppe steht,
R², R³, R⁴ und R⁵ gleich oder verschieden sind und unabhängig voneinander für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 1 bis 13 gleichen oder verschiedenen Halogenatomen, Cyanoalkyl mit 1 bis 8 Kohlenstoffatomen, Alkoxyalkyl oder Alkylthioalkyl mit jeweils bis zu 4 Kohlenstoffatomen in den einzelnen Alkylteilen, für jeweils gegebenenfalls einfach bis dreifach gleich oder verschieden substituiertes Cycloalkyl oder Cycloalkylalkyl mit jeweils 3 bis 8 Kohlenstoffatomen im Cycloalkylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen, die Reste R² bis R⁵ weiter für jeweils gegebenenfalls einfach bis dreifach gleich oder verschieden substituiertes Aryl oder Aralkyl stehen mit jeweils 6 oder 10 Kohlenstoffatomen im Arylteil und gegebenenfalls 1 bis 4 Kohlenstoffatomen im geradkettigen oder verzweigten Alkylteil, wobei als Substituenten infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 Halogenatomen, in welcher weiter auch jeweils zwei dieser Reste - R² und R³ oder R⁴ und R⁵ - zusammen für geradkettiges oder verzweigtes Alkandiyl mit 2 bis 6 Kohlenstoffatomen stehen können, und R⁵ auch für geradkettiges oder verzweigtes Alkoxy mit 1 bis 8 Kohlenstoffatomen stehen kann,
X für Sauerstoff oder Schwefel steht und
Y für Sauerstoff oder Schwefel steht.

3. Substituierte 4,5-Diamino-1,2,4-triazol-3-(thi)one der allgemeinen Formel (I) gemäß Anspruch 1, bei welchen
R¹ für Methyl, Ethyl, n- oder i-Propyl, n-, i-, s-oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl-bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n-oder i-Propyl, n-, i-, s-oder t-Butyl, Cyano, Methandiyl, Ethandiyl, Butandiyl oder Butadiendiyl oder Dichlorallyl;
R¹ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen: 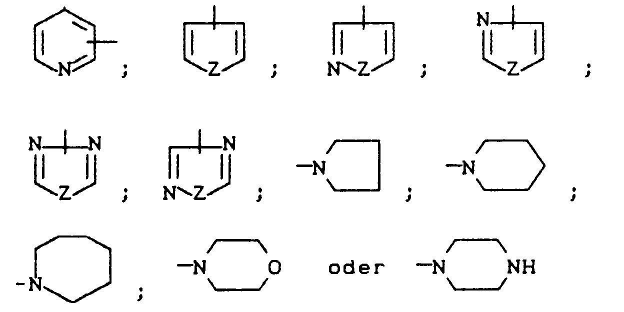 wobei Z jeweils für Sauerstoff oder Schwefel steht und wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
R¹ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylpropenyl, Phenylpropinyl, Phenylbutyl, Pnenylbutenyl, Phenylbutinyl, Phenylpentyl, Phenylpentenyl, Phenylpentinyl, Phenylhexyl, Phenylhexenyl, Phenylhexinyl, Phenylheptyl, Phenylheptenyl, Phenylheptinyl, Phenyloctyl, Phenyloctenyl, Phenyloctinyl, Phenylcyanmethyl, Phenylcyanethyl, Phenylcyanpropyl, Benzyloxy, Phenylethyloxy, Phenoxy, Phenoxyethyl, Phenoxypropyl, Phenoxy-i-propyl, Phenoxyisobutyl, Phenoxy-tert-butyl, Phenylthioisobutyl, Phenylthio-tert-butyl, Phenylthioethyl, Phenylthiopropyl, Phenylthio-i-propyl, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Difluormethyl, Trifluormethyl, Difluormethoxy, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, n- oder i-Propoxycarbonyl, Cyclohexyl oder Phenoxy,
R² für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
R³ für Wasserstoff, Methyl, Ethyl, Propyl, Butyl, Allyl, Propargyl, Cyanoethyl, Methoxyethyl oder gegebenenfalls durch Chlor substituiertes Benzyl steht, oder zusammen mit R² für Butan-1,4-diyl oder Pentan-1,5-diyl steht,
R⁴ für Wasserstoff, Methyl, Ethyl, Propyl, Isopropyl, Butyl, Isobutyl, sec-Butyl, Allyl, Propargyl, Cyanoethyl, Methoxyethyl, Cyclopropyl, Cyclopentyl, Cyclohexyl, Phenyl oder Benzyl steht,
R⁵ für Wasserstoff, Methyl, Ethyl, Propyl, Putyl, Allyl, Propargyl, Cyanoethyl, Methoxyethyl, Methoxy oder Benzyl steht, oder zusammen mit R⁴ für Butan-1,4-diyl oder Pentan-1,5-diyl steht,
X für Sauerstoff oder Schwefel steht und
Y für Sauerstoff oder Schwefel steht.

4. Substituierte 4,5-Diamino-1,2,4-triazol-3-(thi)one der allgemeinen Formel (I) gemäß Anspruch 1, bei welchen
R¹ für jeweils gegebenenfalls einfach bis dreifach, gleich oder verschieden substituiertes 1-Methyl-3-phenyl-propyl, 1-Ethyl-3-phenylpropyl, 1-Propyl-3-phenyl-propyl, 1-Isopropyl-3-phenyl-propyl, 2-Methyl-3-phenyl-propyl, 1,1-Dimethyl-3-phenyl-propyl, 1-Methyl-1-ethyl-3-phenyl-propyl, 1,1-Diethyl-3-phenylpropyl, 1-Methyl-1-propyl-3-phenyl-propyl, 1-Methyl-3-phenyl-2-propenyl, 1-Ethyl-3-phenyl-2-propenyl, 1-Propyl-3-phenyl-2-propenyl, 1-Isopropyl-3-phenyl-2-propenyl, 1,1-Dimethyl-3-phenyl-2-propenyl, 1-Methyl-1-ethyl-3-phenyl-2-propenyl, 1,1-Diethyl-3-phenyl-2-propenyl, 1-Methyl-1-propyl-3-phenyl-2-propenyl, 1-Methyl-3-phenyl-2-propinyl, 1-Ethyl-3-phenyl-2-propinyl, 1-Propyl-3-phenyl-2-propinyl, 1-Isopropyl-3-phenyl-2-propinyl, 1,1-Dimethyl-3-phenyl-2-propinyl, 1-Methyl-1-ethyl-3-phenyl-2-propinyl, 1,1-Diethyl-3-phenyl-2-propinyl, 1-Methyl-1-propyl-3-phenyl-2-propinyl, 1,2-Dimethyl-3-phenyl-propyl, 2-Ethyl-1-methyl-3-phenyl-propyl, 1,2,2-Trimethyl-3-phenylpropyl, 1,3,3-Trimethyl-3-phenyl-propyl oder 1,1,2,2-Tetramethyl-3-phenyl-propyl steht, wobei als Phenylsubstituenten jeweils infrage kommen: Fluor, Chlor, Brom, Methyl, Ethyl, n-oder i-Propyl, n-, i-, s- oder t-Butyl, Trifluormethyl, Phenyl, Methoxy, Ethoxy, Propoxy, Isopropoxy, Difluormethoxy, Trifluormethoxy, Difluormethyl, Methoxycarbonyl, Ethoxycarbonyl, Propoxycarbonyl, Cyano, Methylthio, Ethylthio, Propylthio, Isopropylthio, Difluormethylthio, Trifluormethylthio, Methylsulfinyl, Methylsulfonyl und Trifluormethylsulfonyl, weiterhin
R² für Wasserstoff steht,
R³ für Wasserstoff oder Methyl steht,
R⁴ für Wasserstoff, Methyl oder Ethyl steht,
R⁵ für Wasserstoff, Methyl, Ethyl, n- oder iso-Propyl steht, oder R⁴ und R⁵ zusammen für Butan-1,4-diyl stehen,
X für Sauerstoff oder Schwefel steht und
Y für Sauerstoff steht,

5. Verfahren zur Herstellung von substituierten 4,5-Diamino-1,2,4-triazol-3-(thi)onen der allgemeinen Formel (I) gemäß Anspruch 1, 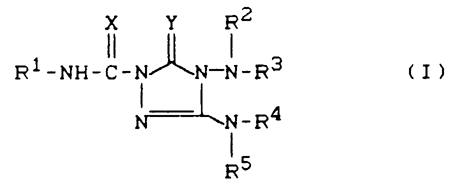 in welcher
R¹ für Alkyl, Alkenyl, Alkinyl, Halogenalkyl, Halogenalkenyl, Halogenalkinyl, Cyanalkyl, Hydroxyalkyl, Alkoxyalkyl, Aryloxyalkyl, Arylthioalkyl, Arylsulfinylalkyl, Arylsulfonylalkyl, Arylaminoalkyl, N-Alkyl-arylaminoalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Arylalkenyl, Arylalkinyl, Aroyl, Aryl, Aralkyloxy oder Aryloxy, für Alkoxy, Alkenyloxy oder Alkinyloxy steht,
R², R³, R⁴ und R⁵ gleich oder verschieden sind und unabhängig voneinander für Wasserstoff, Alkyl, Alkenyl, Alkinyl, Halogenalkyl, Halogenalkenyl, Halogenalkinyl, Cyanalkyl, Alkoxyalkyl, Alkylthioalkyl, für jeweils gegebenenfalls substituiertes Cycloalkyl, Cycloalkylalkyl, Aryl oder Aralkyl stehen, oder jeweils zwei dieser Reste (R² und R³ oder R⁴ und R⁵) zusammen für Alkandiyl stehen, und R⁵ auch für Alkoxy stehen kann,
X für Sauerstoff oder Schwefel steht und
Y für Sauerstoff oder Schwefel steht,
dadurch gekennzeichnet, daß man
(a) 4,5-Diamino-1,2,4-triazol-3-(thi)one der allgemeinen Formel (II) 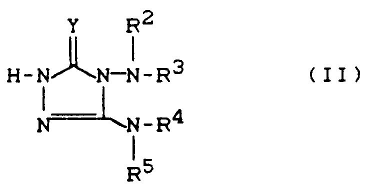 in welcher
R², R³, R⁴, R⁵ und Y die oben angegebenen Bedeutungen haben,
mit Iso(thio)cyanaten der allgemeinen Formel (III)
R¹-N=C=X (III)
in welcher
R¹ und X die oben angegebenen Bedeutungen haben,
gegebenenfalls in Gegenwart eines Verdünnungsmittels und gegebenenfalls in Gegenwart eines Reaktionshilfsmittels umsetzt,
oder daß man
(b) 4-Alkylidenamino-5-amino-1,2,4-triazol-3-(thi)one
der allgemeinen Formel (IV) 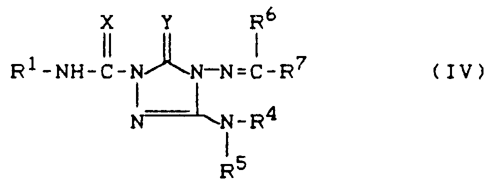 in welcher
R¹, R⁴, R⁵, X und Y die oben angegebenen Bedeutungen haben und
R⁶ und R⁷ gleich oder verschieden sind und unabhängig voneinander für Wasserstoff, Alkyl, Aralkyl oder Aryl stehen,
mit wäßrigen Säuren, gegebenenfalls in Gegenwart organischer Lösungsmittel, umsetzt,
oder daß man
(c) substituierte 1,2,4-Triazol-3-(thi)one der allgemeinen Formel (V) 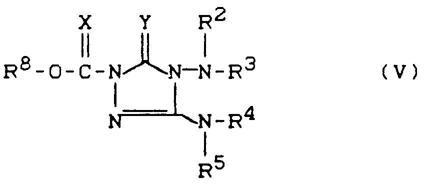 in welcher
R², R³, R⁴, R⁵, X und Y die oben angegebenen Bedeutungen haben und
R⁸ für Alkyl, Aralkyl oder Aryl steht,
mit Aminoverbindungen der allgemeinen Formel (VI)
R¹-NH₂(VI)
in welcher
R¹ die oben angegebene Bedeutung hat,
gegebenenfalls in Gegenwart eines Verdünnungsmittels und gegebenenfalls in Gegenwart eines Reaktionshilfsmittels umsetzt,
oder daß man
(d) 4,5-Diamino-1,2,4-triazol-3-(thi)one der allgemeinen Formel (II) 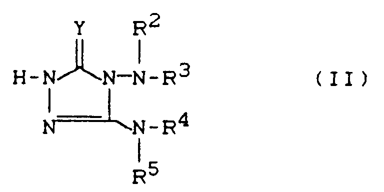 in welcher
R², R³, R⁴, R⁵ und Y die oben angegebenen Bedeutungen haben,
mit (Thio)Urethanen der allgemeinen Formel (VII) 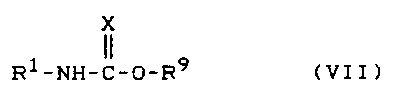 in welcher
R¹ und X die oben angegebenen Bedeutungen haben und
R⁹ für Alkyl, Aralkyl oder Aryl steht,
gegebenenfalls in Gegenwart eines Verdünnungsmittels und gegebenenfalls in Gegenwart eines Reaktionshilfsmittels umsetzt,
oder daß man
(e) 4-Oxyalkylidenamino-5-amino-1,2,4-triazol-3-(thi)one der allgemeinen Formel (VIII) 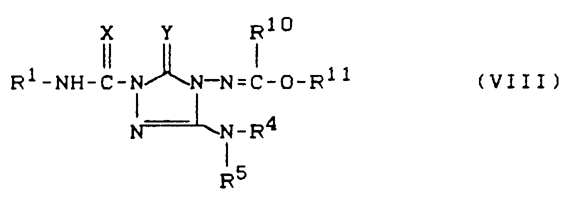 in welcher
R¹, R⁴, R⁵, X und Y die oben angegebenen Bedeutungen haben und
R¹⁰ und R¹¹ gleich oder verschieden sind und unabhängig voneinander für Alkyl, Alkenyl, Alkinyl, Cycloalkyl, Aralkyl oder Aryl stehen, sowie R¹⁰ auch für Wasserstoff steht,
mit Hydridkomplexen der allgemeinen Formel (IX)
M¹M²H₄(IX)
in welcher
M¹ für Lithium, Natrium oder Kalium steht und
M² für Bor oder Aluminium steht,
gegebenenfalls in Gegenwart eines Verdünnungsmittels umsetzt,
oder daß man
(f) 4-Alkylidenamino-5-amino-1,2,4-triazol-3-(thi)one der allgemeinen Formel (IV) 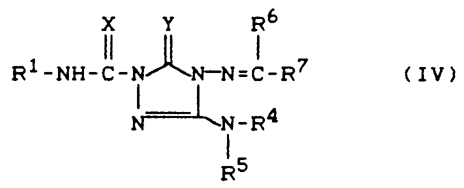 in welcher
R¹, R⁴, R⁵, X und Y die oben angegebenen Bedeutungen haben und
R⁶ und R⁷ gleich oder verschieden sind und unabhängig voneinander für Wasserstoff, Alkyl, Aralkyl oder Aryl stehen,
mit Reduktionsmitteln, gegebenenfalls in Gegenwart von Katalysatoren und gegebenenfalls in Gegenwart von Verdünnungsmitteln umsetzt.

6. Herbizide Mittel, gekennzeichnet durch einen Gehalt an mindestens einem substituierten 4,5-Diamino-1,2,4-triazol-3-(thi)on-Derivat der Formel (I) gemäß den Ansprüchen 1 bis 5.

7. Verfahren zur Bekämpfung von unerwünschten Pflanzen, dadurch gekennzeichnet, daß man substituierte 4,5-Diamino-1,2,4-triazol-3-(thi)one der Formel (I) gemäß den Ansprüchen 1 bis 5 auf unerwünschte Pflanzen und/oder ihren Lebensraum einwirken läßt.

8. Verwendung von substituierten 4,5-Diamino-1,2,4-triazol-3-(thi)onen der Formel (I) gemäß den Ansprüchen 1 bis 5 zur Bekämpfung von unerwünschten Pflanzen.

9. Verfahren zur Herstellung von herbiziden Mitteln, dadurch gekennzeichnet, daß man substituierte 4,5-Diamino-1,2,4-triazol-3-(thi)one der Formel (I) gemäß den Ansprüchen 1 bis 5 mit Streckmitteln und/oder oberflächenaktiven Substanzen vermischt.

10. Iso(thio)cyanate der Formel (III)
R¹-N=C=X (III)
in welcher
R¹ für jeweils gegebenenfalls einfach bis dreifach, gleich oder verschieden substituiertes 1-Ethyl-3-phenyl-propyl, 1-Propyl-3-phenylpropyl, 1-Isopropyl-3-phenyl-propyl, 2-Methyl-3-phenyl-propyl, 1,1-Dimethyl-3-phenyl-propyl, 1,2-Dimethyl-3-phenyl-propyl, 1,1-Diethyl-3-phenyl-propyl, 1-Methyl-1-propyl-3-phenyl-propyl, 1,2,2-Trimethyl-3-phenyl-propyl, 1-Methyl-3-phenyl-2-propenyl, 1-Ethyl-3-phenyl-2-propenyl, 1-Propyl-3-phenyl-2-propenyl, 1-Isopropyl-3-phenyl-2-propenyl, 1,1-Dimethyl-3-phenyl-2-propenyl, 1-Methyl-1-ethyl-3-phenyl-2-propenyl, 1,1-Diethyl-3-phenyl-2-propenyl, 1-Methyl-1-propyl-3-phenyl-2-propenyl, 1-Methyl-3-phenyl-2-propinyl, 1-Ethyl-3-phenyl-2-propinyl, 1-Propyl-3-phenyl-2-propinyl, 1-Isopropyl-3-phenyl-2-propinyl, 1,1-Dimethyl-3-phenyl-2-propinyl, 1-Methyl-1-ethyl-3-phenyl-2-propinyl, 1,1-Diethyl-3-phenyl-2-propinyl, 1-Methyl-1-propyl-3-phenyl-2-propinyl, 1,2-Dimethyl-3-phenyl-propyl, 2-Ethyl-1-methyl-3-phenyl-propyl, 1,2,2-Trimethyl-3-phenyl-propyl, 1,3,3-Trimethyl-3-phenyl-propyl oder 1,1,2,2-Tetramethyl-3-phenyl-propyl steht, wobei als Phenylsubstituenten jeweils infrage kommen: Fluor, Chlor, Brom, Methyl, Ethyl, n-oder i-Propyl, n-, i-, s- oder t-Butyl, Trifluormethyl, Phenyl, Methoxy, Ethoxy, Propoxy, Isopropoxy, Difluormethoxy, Trifluormethoxy, Difluormethyl, Methoxycarbonyl, Ethoxycarbonyl, Propoxycarbonyl, Cyano, Methylthio, Ethylthio, Propylthio, Isopropylthio, Difluormethylthio, Trifluormethylthio, Methylsulfinyl, Methylsulfonyl und Trifluormethylsulfonyl, und
X für Sauerstoff oder Schwefel, vorzugsweise für Sauerstoff, steht.
